# EUROPÄISCHE PATENTSCHRIFT

(11) **EP 2 906 575 B1**
(45) Veröffentlichungstag und Bekanntmachung des Hinweises auf die Patenterteilung: **17.01.2018**
(21) Anmeldenummer: 13762407.8
(22) Anmeldetag: 11.09.2013
(51) Int. Cl.: C07F 15/00, H01L 51/00, H01L 51/50

(54) **METALLKOMPLEXE**
METAL COMPLEXES
COMPLEXES MÉTALLIQUES

(30) Priorität: 09.10.2012 EP 12006990
(43) Veröffentlichungstag der Anmeldung: 19.08.2015
(73) Patentinhaber: Merck Patent GmbH, 64293 Darmstadt (DE)
(72) Erfinder: STOESSEL, Philipp, 60487 Frankfurt am Main (DE); KAISER, Joachim, 64289 Darmstadt (DE); JOOSTEN, Dominik, 60487 Frankfurt am Main (DE); KOENEN, Nils, 64285 Darmstadt (DE); BREUNING, Esther, 64372 Ober-Ramstadt (DE)
(86) Internationale Anmeldenummer: PCT/EP2013/002726
(87) Internationale Veröffentlichungsnummer: WO 2014/056564

(56) Entgegenhaltungen:
- DE-A1-102010 027 316
- DE-A1-102010 027 317
- JP-A- 2004 131 464
- JP-A- 2011 119 576
- US-A1- 2009 278 444
- RANDOLPH P. THUMMEL ET AL: "Polyaza-cavity shaped molecules. 14. Annelated 2-(2'-pyridyl)indoles, 2,2'-biindoles, and related systems", THE JOURNAL OF ORGANIC CHEMISTRY, Bd. 54, Nr. 7, 1. März 1989 (1989-03-01), Seiten 1720-1725, XP055089136, ISSN: 0022-3263, DOI: 10.1021/jo00268a040

## Beschreibung

Die vorliegende Erfindung betrifft Metallkomplexe, welche sich für den Einsatz als Emitter in organischen Elektrolumineszenzvorrichtungen eignen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Dabei werden als emittierende Materialien zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, die Triplettemission zeigen, immer noch Verbesserungsbedarf, insbesondere im Hinblick auf Effizienz, Betriebsspannung und Lebensdauer. Dies gilt insbesondere für OLEDs, welche im kürzerwelligen Bereich, also grün und insbesondere blau, emittieren.

Gemäß dem Stand der Technik werden in phosphoreszierenden OLEDs als Triplettemitter insbesondere Iridium- und Platinkomplexe eingesetzt. So sind beispielsweise Iridiumkomplexe bekannt, welche als Liganden Imidazophenanthridin-Derivate bzw. Diimidazochinazolin-Derivate enthalten (WO 2007/095118). Diese Komplexe können bei Anwendung in organischen Elektrolumineszenzvorrichtungen, je nach genauer Struktur des Liganden, zu blauer Phosphoreszenz führen. Aus WO 2010/086089 und WO 2011/157339 sind Metallkomplexe bekannt, welche als Liganden Imidazo-isochinolin-Derivate enthalten. Mit derartigen Komplexen wurden bereits gute Fortschritte in der Entwicklung blauer Triplettemitter erzielt. Es sind jedoch noch weitere Verbesserungen wünschenswert, insbesondere hinsichtlich Effizienz, Betriebsspannung und Lebensdauer.

Aufgabe der vorliegenden Erfindung ist daher die Bereitstellung neuer Metallkomplexe, welche sich als Emitter für die Verwendung in OLEDs eignen. Insbesondere ist die Aufgabe, Emitter bereitzustellen, welche sich je nach Substitution auch für blau oder grün phosphoreszierende OLEDs eignen, und welche dabei verbesserte Eigenschaften in Bezug auf Effizienz, Betriebsspannung, Lebensdauer, Farbkoordinaten und/oder Farbreinheit, d. h. Breite der Emissionsbande, zeigen. Eine weitere Aufgabe der vorliegenden Erfindung ist die Entwicklung von phosphoreszierenden Emittern, welche gleichzeitig als lochtransportierende Verbindung in der emittierenden Schicht dienen können.

Überraschend wurde gefunden, dass bestimmte, unten näher beschriebene Metallchelatkomplexe eine oder mehrere der oben genannten Aufgaben lösen und sich sehr gut für die Verwendung in einer organischen Elektrolumineszenzvorrichtung eignen. Diese Metallkomplexe und organische Elektrolumineszenzvorrichtungen, welche diese Komplexe enthalten, sind daher der Gegenstand der vorliegenden Erfindung.

Aus US 2009/0278444 sind Metallkomplexe bekannt, wobei der Ligand eine überbrückte Phenyl-imidazol-Struktur aufweist und die Substituenten am Liganden eine Carbazolstruktur bilden. Liganden, die keine Carbazolstruktur aufweisen, sind nicht offenbart.

Gegenstand der Erfindung ist somit eine Verbindung gemäß Formel (1),

M(L)n(L')m Formel (1)

welche eine Teilstruktur M(L)ₙ der Formel (2) oder Formel (3) enthält: 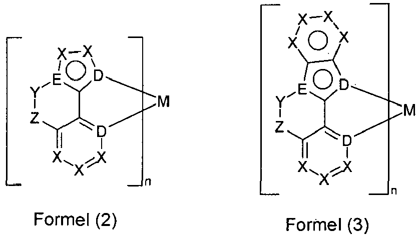 wobei für die verwendeten Symbole und Indizes gilt:
- M: ist ein Übergangsmetall;
- X: ist bei jedem Auftreten gleich oder verschieden ausgewählt aus der Gruppe bestehend aus CR und N;
- Y: ist bei jedem Auftreten gleich oder verschieden ausgewählt aus der Gruppe bestehend aus C(R¹)₂, Si(R¹)₂, PR¹, P(=O)_{R}¹ oder BR¹;
- Z: ist bei jedem Auftreten gleich oder verschieden ausgewählt aus der Gruppe bestehend aus NR¹, O oder C(R¹)₂;
- D: ist bei jedem Auftreten gleich oder verschieden C oder N, mit der Maßgabe, dass mindestens ein D für N steht;
- E: ist bei jedem Auftreten gleich oder verschieden C oder N, mit der Maßgabe, dass mindestens eine der Gruppen E oder D in dem Fünfring für N steht;
- R, R¹: ist bei jedem Auftreten gleich oder verschieden H, D, F, Cl, Br, I, N(R²)₂, CN, NO₂, OH, COOH, C(=O)N(R²)₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R² substituiert sein kann, wobei eine oder mehrere nicht benachbarte CH₂-Gruppen durch R²C=CR², C=C, Si(R²)₂, C=O, NR², O, S oder CONR² ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei benachbarte Reste R bzw. zwei benachbarte Reste R¹ auch miteinander ein mono- oder polycyclisches, aliphatisches, aromatisches oder heteroaromatisches Ringsystem bilden; weiterhin können R und R¹ auch miteinander ein mono- oder polycyclisches, aliphatisches oder heteroaromatisches Ringsystem bilden;
- R²: ist bei jedem Auftreten gleich oder verschieden H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R³ substituiert sein kann, wobei eine oder mehrere nicht benachbarte CH₂-Gruppen durch R³C=CR³, C=C, Si(R³)₂, C=O, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 40 aromatischen Ringatomen, die durch einen oder mehrere Reste R³ substituiert sein kann, oder eine Aralkyl- oder Heteroaralkylgruppe mit 5 bis 40 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann; dabei können zwei oder mehrere benachbarte Reste R² miteinander oder R² mit R bzw. mit R¹ ein mono- oder polycyclisches, aliphatisches, aromatisches oder heteroaromatisches Ringsystem bilden;
- R³: ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R³ auch miteinander ein mono- oder polycyclisches, aliphatisches Ringsystem bilden;
- L': ist gleich oder verschieden bei jedem Auftreten ein beliebiger Coligand;
- n: ist 1, 2 oder 3;
- m: ist 0, 1, 2, 3 oder 4;
dabei können auch mehrere Liganden L miteinander oder L mit L' über eine Einfachbindung oder eine bivalente oder trivalente Brücke verknüpft sein und so ein tridentates, tetradentates, pentadentates oder hexadentates Ligandensystem aufspannen, wobei in diesem Fall L' kein separater Coligand, sondern eine koordinierende Gruppe ist;

weiterhin kann auch ein Substituent R zusätzlich an das Metall koordinieren;
dabei sind die folgenden Verbindungen von der Erfindung ausgenommen:

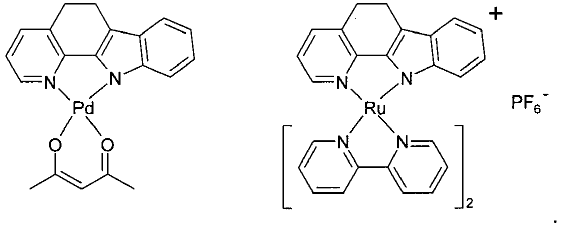

Dabei deuten die Kreise in der Struktur der Formel (2) und (3) ein aromatisches bzw. heteroaromatisches System an, wie in der organischen Chemie üblich. Auch wenn in der Struktur der Formel (3) vereinfachend zwei Kreise eingezeichnet sind, bedeutet dies dennoch, dass es sich um ein einziges heteroaromatisches System handelt.

Dabei bedeutet "benachbarte Reste" in der Definition der Reste, dass diese Reste an dasselbe oder an direkt aneinander gebundene Atome gebunden sind oder, wenn sie nicht an direkt aneinander gebundene Atome gebunden sind, dass es sich um die nächstmögliche Position handelt, in der ein Substituent gebunden sein kann. Dies wird anhand von zwei spezifischen Liganden in der folgenden schematischen Darstellung nochmals erläutert:

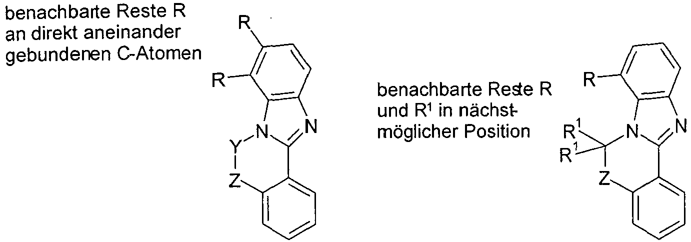

Dabei werden in den Komplexen der Formel (1) die Indizes n und m so gewählt, dass die Koordinationszahl am Metall M insgesamt, je nach Metall, der für dieses Metall üblichen Koordinationszahl entspricht. Dies ist für Übergangsmetalle je nach Metall üblicherweise die Koordinationszahl 4, 5 oder 6. Es ist generell bekannt, dass Metallkoordinationsverbindungen abhängig vom Metall und von der Oxidationsstufe des Metalls unterschiedliche Koordinationszahlen aufweisen, also eine unterschiedliche Anzahl von Liganden binden. Da die bevorzugten Koordinationszahlen von Metallen bzw. Metallionen in verschiedenen Oxidationsstufen zum allgemeinen Fachwissen des Fachmanns auf dem Gebiet der metallorganischen Chemie bzw. der Koordinationschemie gehören, ist es für den Fachmann ein Leichtes, je nach Metall und dessen Oxidationsstufe und je nach genauer Struktur des Liganden L eine geeignete Anzahl Liganden zu verwenden und somit die Indizes n und m geeignet zu wählen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl oder Terphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt sind Verbindungen gemäß Formel (1), dadurch gekennzeichnet, dass diese nicht geladen, d. h. elektrisch neutral, sind. Dies wird auf einfache Weise dadurch erreicht, dass die Ladungen der Liganden L und L' so gewählt werden, dass sie die Ladung des komplexierten Metallatoms M kompensieren. Wenn die Verbindung der Formel (1) nicht neutral ist, enthält sie noch ein oder mehrere Gegenionen, also Anionen, wenn sie kationisch ist, bzw. Kationen, wenn sie anionisch ist.

Bevorzugt sind weiterhin Verbindungen gemäß Formel (1), dadurch gekennzeichnet, dass die Summe der Valenzelektronen um das Metallatom in vierfach koordinierten Komplexen 16 und in fünffach koordinierten Komplexen 16 oder 18 und in sechsfach koordinierten Komplexen 18 beträgt. Diese Bevorzugung ist durch die besondere Stabilität dieser Metallkomplexe begründet.

In einer bevorzugten Ausführungsform der Erfindung steht M für ein Übergangsmetall, wobei Lanthanide und Actinide ausgenommen sind, insbesondere für ein tetrakoordiniertes, ein pentakoordiniertes oder ein hexakoordiniertes Übergangsmetall, besonders bevorzugt ausgewählt aus der Gruppe bestehend aus Chrom, Molybdän, Wolfram, Rhenium, Ruthenium, Osmium, Rhodium, Iridium, Nickel, Palladium, Platin, Kupfer, Silber und Gold, insbesondere Molybdän, Wolfram, Rhenium, Ruthenium, Osmium, Iridium, Kupfer, Platin und Gold. Ganz besonders bevorzugt sind Iridium, Platin und Kupfer. Die Metalle können dabei in verschiedenen Oxidationsstufen vorliegen. Bevorzugt sind dabei die oben genannten Metalle in den Oxidationsstufen Cr(0), Cr(II), Cr(III), Cr(IV), Cr(VI), Mo(0), Mo(II), Mo(III), Mo(IV), Mo(VI), W(0), W(II), W(III), W(IV), W(VI), Re(I), Re(II), Re(III), Re(IV), Ru(II), Ru(III), Os(II), Os(III), Os(IV), Rh(I), Rh(III), Ir(I), Ir(III), Ir(IV), Ni(0), Ni(II), Ni(IV), Pd(II), Pt(II), Pt(IV), Cu(I), Cu(II), Cu(III), Ag(I), Ag(II), Au(I), Au(III) und Au(V). Besonders bevorzugt sind Mo(0), W(0), Re(I), Ru(II), Os(II), Rh(III), Cu(I), Ir(III) und Pt(II). Ganz besonders bevorzugt sind Ir(III), Pt(II) und Cu(I), insbesondere Ir(III).

In einer bevorzugten Ausführungsform der Erfindung ist M ein tetrakoordiniertes Metall, und der Index n steht für 1 oder 2. Wenn der Index n = 1 ist, sind noch ein bidentater oder zwei monodentate Liganden L', bevorzugt ein bidentater Ligand L', an das Metall M koordiniert. Wenn der Index n = 2 ist, ist der Index m = 0. Bevorzugte tetrakoordinierte Metalle sind Pt(II) und Cu(I). Wenn M für Cu(I) steht, stehen bevorzugt beide Gruppen D für N. Wenn M für Pt(II) steht, stehen entweder beide Gruppen D für N oder eine Gruppe D steht für N und die andere Gruppe D steht für C, bevorzugt steht eine Gruppe D für N und die andere Gruppe D steht für C.

In einer weiteren bevorzugten Ausführungsform der Erfindung ist M ein hexakoordiniertes Metall, und der Index n steht für 1, 2 oder 3, bevorzugt für 2 oder 3. Wenn der Index n = 1 ist, sind noch vier monodentate oder zwei bidentate oder ein bidentater und zwei monodentate oder ein tridentater und ein monodentater oder ein tetradentater Ligand L', bevorzugt zwei bidentate Liganden L', an das Metall koordiniert. Wenn der Index n = 2 ist, sind noch ein bidentater oder zwei monodentate Liganden L', bevorzugt ein bidentater Ligand L', an das Metall koordiniert. Wenn der Index n = 3 ist, ist der Index m = 0. Ein bevorzugtes hexakoordiniertes Metall ist Ir(III). Wenn M für Ir(III) steht, stehen entweder beide Gruppen D für N oder eine Gruppe D steht für N und die andere Gruppe D steht für C, bevorzugt steht eine Gruppe D für N und die andere Gruppe D steht für C.

In einer bevorzugten Ausführungsform der Erfindung steht E für N und entweder das D in dem Fünfring oder das D in dem Sechsring steht N, während das andere D für C steht. In einer weiteren Ausführungsform der Erfindung steht E für C und beide Gruppen D stehen für N. Bevorzugt handelt es sich bei den Liganden L also monoanionische Liganden.

Bevorzugte Teilstrukturen der Formel (2) sind somit die Teilstrukturen der folgenden Formeln (2a), (2b) und (2c), und bevorzugte Teilstrukturen der Formel (3) sind die Teilstrukturen der folgenden Formeln (3a), (3b) und (3c), 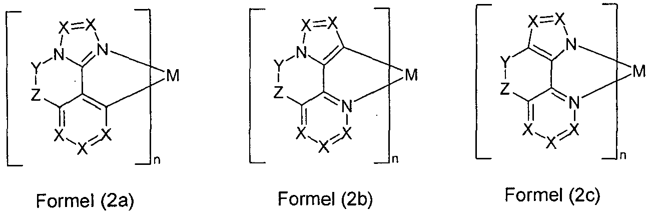 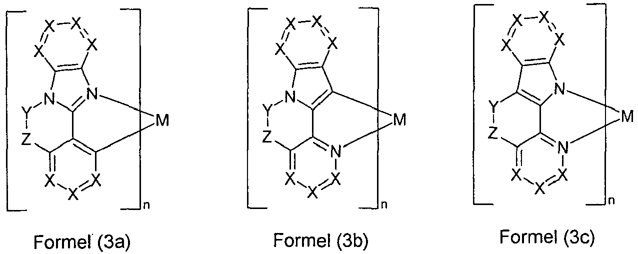 wobei die verwendeten Symbole und Indizes die oben genannten Bedeutungen aufweisen.

In einer weiteren bevorzugten Ausführungsform der Erfindung steht die Gruppe -Y-Z- gleich oder verschieden bei jedem Auftreten für -C(R¹)₂-NR¹-, -C(R¹)₂-O-, -C(R¹)₂-C(R¹)₂-, -Si(R¹)₂-NR¹-, -Si(R¹)₂-O-, -Si(R¹)₂-C(R¹)₂-, -PR¹-NR¹-, -PR¹-O-, -PR¹-C(R¹)₂-, -P(=O)R¹- NR¹-, -P(=O)R¹-O-, -P(=O)R¹-C(R¹)₂-, -BR¹-NR¹-, -BR¹-O- oder -BR¹-C(R¹)₂-Besonders bevorzugt steht die Gruppe -Y-Z- gleich oder verschieden bei jedem Auftreten für -C(R¹)₂-NR¹-, -C(R¹)₂-O-, -Si(R¹)₂-NR¹- oder -Si(R¹)₂-O-.

In einer bevorzugten Ausführungsform der Erfindung enthält die Gruppe -Y-Z- keine benzylischen Protonen. Wenn Y für C(R¹)₂ steht, ist R¹ bevorzugt gleich oder verschieden bei jedem Auftreten ausgewählt aus der Gruppe bestehend aus F, CN, eine geradkettige Alkylgruppe mit 1 bis 10 C-Atomen oder eine verzweigte oder cyclische Alkylgruppe mit 3 bis 10 C-C-Atomen, die jeweils mit einem oder mehreren Resten R² substituiert sein kann, wobei eine oder mehrere nicht benachbarte CH₂-Gruppen durch R²C=CR² ersetzt sein können und wobei ein oder mehrere H-Atome durch F ersetzt sein können, oder ein aromatisches oder heteroaromatisches Ringsystem mit 5 bis 30 aromatischen Ringatomen, das jeweils durch einen oder mehrere Reste R² substituiert sein kann, oder eine Aryloxy- oder Heteroaryloxygruppe mit 5 bis 30 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 30 aromatischen Ringatomen, welche durch einen oder mehrere Reste R² substituiert sein kann; dabei können zwei benachbarte Reste R bzw. zwei benachbarte Reste R¹ auch miteinander ein mono- oder polycyclisches, aliphatisches, aromatisches oder heteroaromatisches Ringsystem bilden; weiterhin können R und R¹ auch miteinander ein mono- oder polycyclisches, aliphatisches oder heteroaromatisches Ringsystem bilden.

Im Liganden L stehen bevorzugt keine, eine oder zwei Gruppen X, besonders bevorzugt keine oder eine Gruppe X für N.

Bevorzugte Ausführungsformen der Teilstrukturen der Formel (2) sind die Teilstrukturen der folgenden Formeln (2-A) bis (2-G), 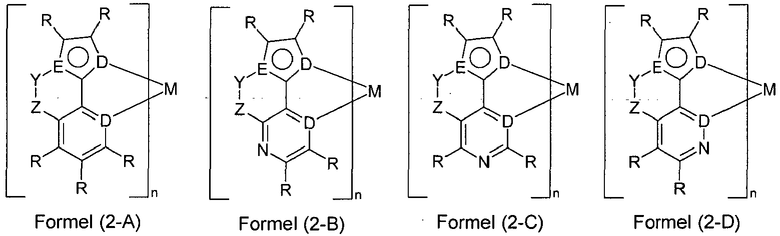 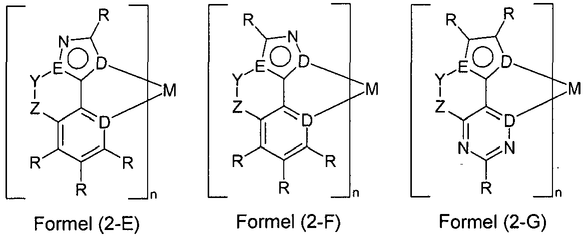 wobei die verwendeten Symbole und Indizes die oben genannten Bedeutungen aufweisen.

Bevorzugte Ausführungsformen der Teilstrukturen der Formel (3) sind die Teilstrukturen der folgenden Formeln (3-A) bis (3-H), 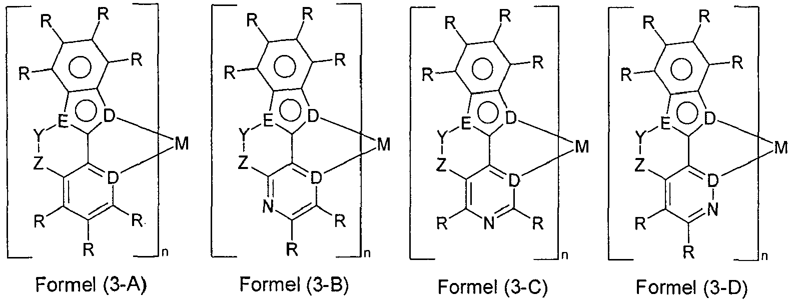 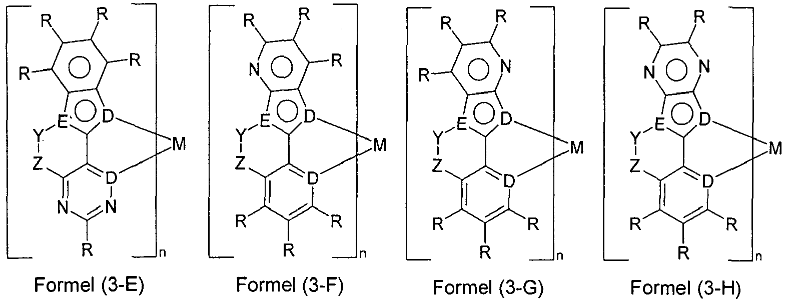 wobei die verwendeten Symbole und Indizes die oben genannten Bedeutungen aufweisen.

Besonders bevorzugt ist es, wenn in den Teilstrukturen der Formeln (2-A) bis (2-G) und (3-A) bis (3-H) die Gruppe E für N steht und entweder die Gruppe D in dem Fünfring oder die Gruppe D in dem Sechsring für N steht, während die andere Gruppe D für C steht, oder wenn die Gruppe E für C steht und beide Gruppen D für N stehen, in Analogie zu den oben aufgeführten allgemeinen Formeln (2a) bis (2c) und (3a) bis (3c).

Bevorzugt enthalten diese Verbindungen weiterhin die oben als bevorzugt genannten Gruppen -Y-Z-.

Wenn eine oder mehrere Gruppen X in den Teilstrukturen der Formel (2) oder (3) für Stickstoff stehen, ist es bevorzugt, wenn benachbart zu diesem Stickstoffatom eine Gruppe R als Substituent gebunden ist, welche ungleich Wasserstoff oder Deuterium ist. Dabei ist dieses R bevorzugt eine Gruppe, ausgewählt aus CF₃, OCF₃, Alkyl- oder Alkoxygruppen mit 1 bis 10 C-Atomen, insbesondere verzweigten oder cyclischen Alkyl- oder Alkoxygruppen mit 3 bis 10 C-Atomen, aromatischen bzw. heteroaromatischen Ringsystemen oder Aralkyl- bzw. Heteroaralkylgruppen, wobei diese Gruppen jeweils optional durch einen oder mehrere Reste R² substituiert sein können. Es handelt sich bei diesen Gruppen um sterisch anspruchsvolle Gruppen. Weiterhin bevorzugt kann dieser Rest R auch mit einem benachbarten Rest R einen ankondensierten Ring bilden.

Wenn der Rest R, der einem Stickstoffatom benachbart ist, für eine Alkylgruppe steht, dann weist diese Alkylgruppe bevorzugt 3 bis 10 C-Atome auf. Bevorzugt handelt es sich weiterhin um eine sekundäre oder tertiäre Alkylgruppe, bei der das sekundäre oder tertiäre C-Atom entweder direkt an den Liganden gebunden ist oder über eine CH₂-Gruppe an den Liganden gebunden ist. Besonders bevorzugt ist diese Alkylgruppe ausgewählt aus den Strukturen der folgenden Formeln (R-1) bis (R-33), wobei jeweils auch die Anknüpfung dieser Gruppen an den Liganden mit eingezeichnet ist: 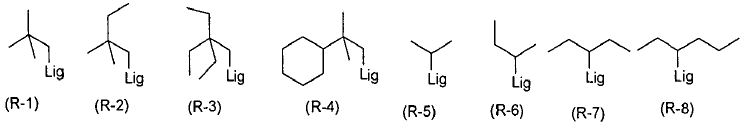 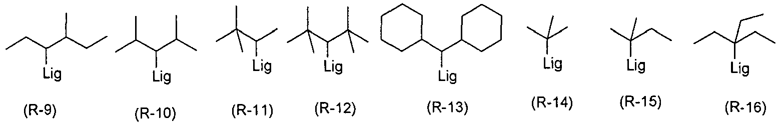 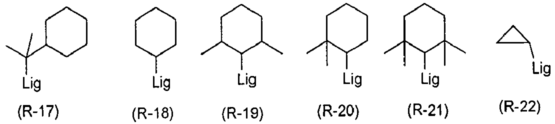 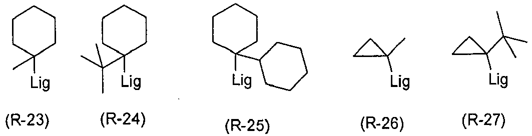 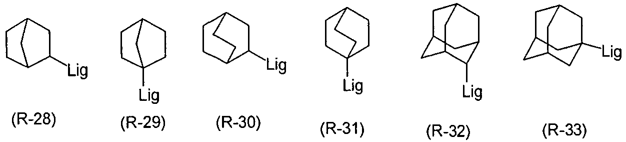 wobei Lig die Anknüpfung der Alkylgruppe an den Liganden kennzeichnet.

Wenn der Rest R, der einem Stickstoffatom benachbart ist, für eine Alkoxygruppe steht, dann weist diese Alkoxygruppe bevorzugt 3 bis 10 C-Atome auf. Bevorzugt ist diese Alkoxygruppe ausgewählt aus den Strukturen der folgenden Formeln (R-34) bis (R-47), wobei jeweils auch die Anknüpfung dieser Gruppen an den Liganden mit eingezeichnet ist: 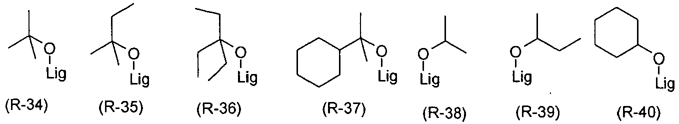 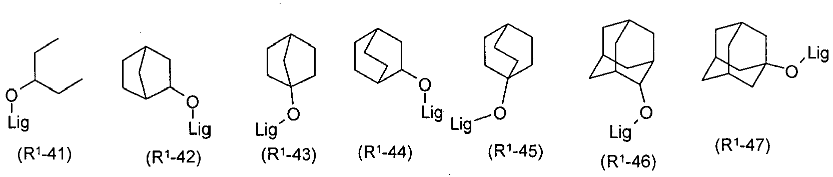 wobei Lig die Anknüpfung der Alkylgruppe an den Liganden kennzeichnet.

Wenn der Rest R, der einem Stickstoffatom benachbart ist, für eine Dialkylaminogruppe steht, dann weist jede dieser Alkylgruppen bevorzugt 1 bis 8 C-Atome auf, besonders bevorzugt 1 bis 6 C-Atome. Beispiele für geeignete Alkylgruppen sind Methyl, Ethyl oder die oben als Gruppen (R-1) bis (R-33) aufgeführten Strukturen. Besonders bevorzugt ist die Dialkylaminogruppe ausgewählt aus den Strukturen der folgenden Formeln (R-48) bis (R-55), wobei jeweils auch die Anknüpfung dieser Gruppen an den Liganden mit eingezeichnet ist: 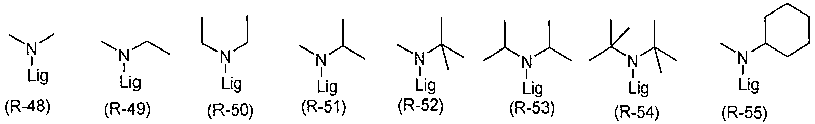 wobei Lig die Anknüpfung der Alkylgruppe an den Liganden kennzeichnet.

Wenn der Rest R, der einem Stickstoffatom benachbart ist, für eine Aralkylgruppe steht, dann ist diese Aralkylgruppe bevorzugt ausgewählt aus den Strukturen der folgenden Formeln (R-56) bis (R-69), wobei jeweils auch die Anknüpfung dieser Gruppen an den Liganden eingezeichnet ist: 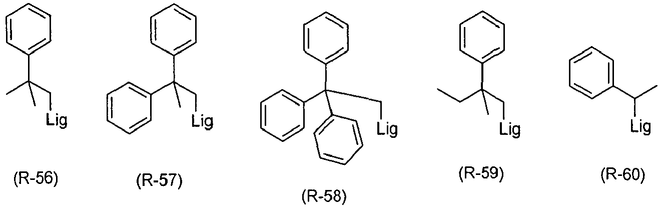 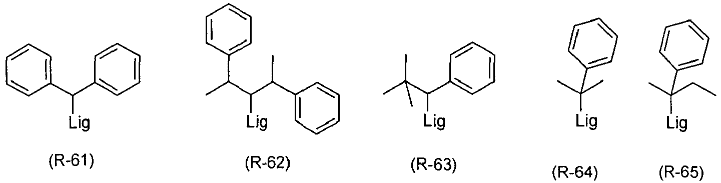 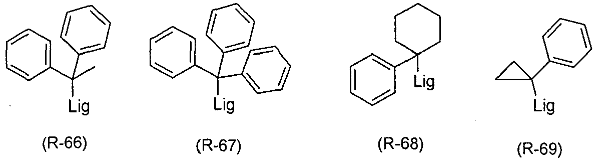 wobei Lig die Anknüpfung der Aralkylgruppe an den Liganden kennzeichnet und die Phenylgruppen jeweils durch einen oder mehrere Reste R² substituiert sein können.

Wenn der Rest R, der einem Stickstoffatom benachbart ist, für ein aromatisches bzw. heteroaromatisches Ringsystem steht, dann weist dieses aromatische bzw. heteroaromatische Ringsystem bevorzugt 5 bis 30 aromatische Ringatome auf, besonders bevorzugt 5 bis 24 aromatische Ringatome. Weiterhin enthält dieses aromatische bzw. heteroaromatische Ringsystem bevorzugt keine Aryl- bzw. Heteroarylgruppen, in denen mehr als zwei aromatische Sechsringe direkt aneinander kondensiert sind. Besonders bevorzugt enthält das aromatische bzw. heteroaromatische Ringsystem überhaupt keine kondensierten Aryl- bzw. Heteroarylgruppen, und ganz besonders bevorzugt enthält es nur Phenylgruppen. Dabei ist das aromatische Ringsystem bevorzugt ausgewählt aus den Strukturen der folgenden Formeln (R-70) bis (R-86), wobei jeweils auch die Anknüpfung dieser Gruppen an den Liganden eingezeichnet ist: 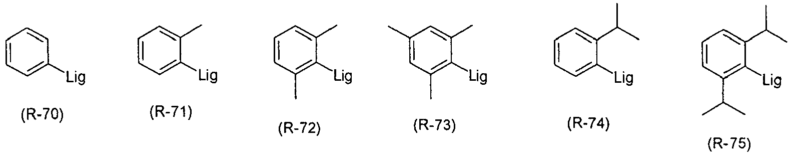 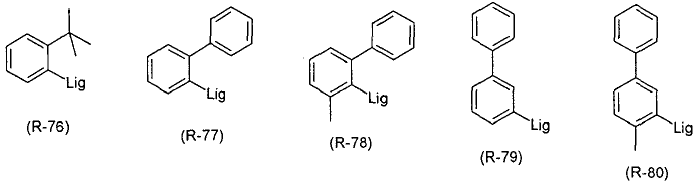 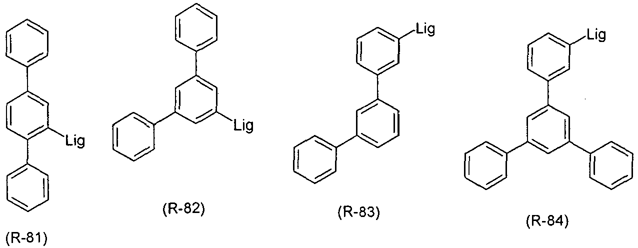 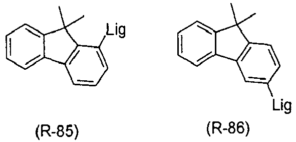 wobei Lig die Anknüpfung des aromatischen oder heteroaromatischen Ringsystems an den Liganden kennzeichnet und die Phenylgruppen jeweils durch einen oder mehrere Reste R² substituiert sein können.

Weiterhin ist das heteroaromatische Ringsystem bevorzugt ausgewählt aus den Strukturen der folgenden Formeln (R-87) bis (R-112), wobei jeweils auch die Anknüpfung dieser Gruppen an den Liganden eingezeichnet ist: 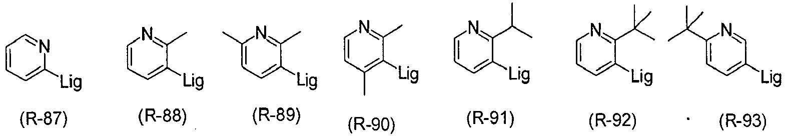 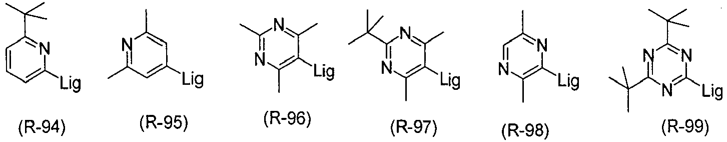 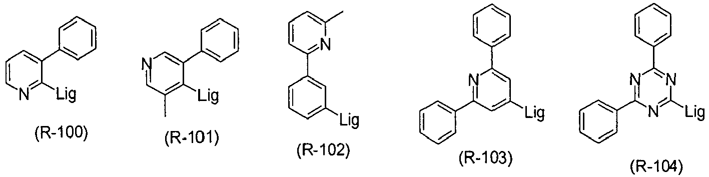 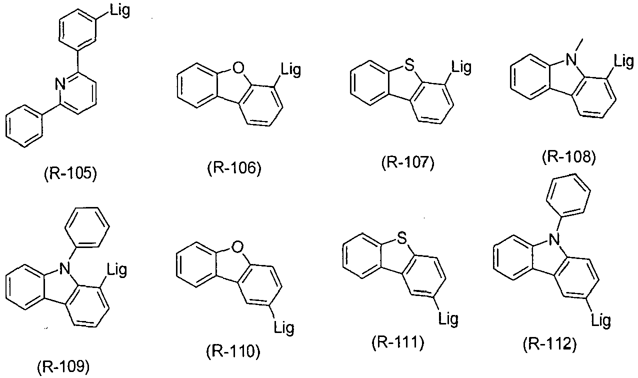 wobei Lig die Anknüpfung des aromatischen oder heteroaromatischen Ringsystems an den Liganden kennzeichnet und die aromatischen und heteroaromatischen Gruppen jeweils durch einen oder mehrere Reste R² substituiert sein können.

Weiterhin kann es bevorzugt sein, wenn zwei benachbarte Gruppen X für CR stehen und die jeweiligen Reste R zusammen mit den C-Atomen einen Ring der folgenden Formel (4) oder Formel (5) aufspannen, 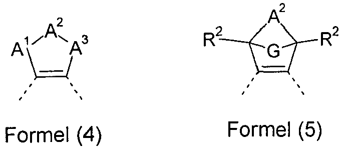 wobei R² und R³ die oben genannten Bedeutungen aufweisen, die gestrichelten Bindungen die Verknüpfung der beiden Kohlenstoffatome im Liganden andeuten und weiterhin gilt:
- A¹, A³: ist gleich oder verschieden bei jedem Auftreten C(R⁴)₂, O, S, NR⁴ oder C(=O);
- A²: ist C(R²)₂, O, S, NR⁴ oder C(=O);
- G: ist eine Alkylengruppe mit 1, 2 oder 3 C-Atomen, welche mit einem oder mehreren Resten R³ substituiert sein kann, -CR³=CR³- oder eine ortho-verknüpfte Arylen- oder Heteroarylengruppe mit 5 bis 14 aromatischen Ringatomen, welche durch einen oder mehrere Reste R³ substituiert sein kann;
- R⁴: ist gleich oder verschieden bei jedem Auftreten F, eine geradkettige Alkyl- oder Alkoxygruppe mit 1 bis 20 C-Atomen, eine verzweigte oder cyclische Alkyl- oder Alkoxygruppe mit 3 bis 20 C-Atomen, die jeweils mit einem oder mehreren Resten R³ substituiert sein kann, wobei eine oder mehrere nicht benachbarte CH₂-Gruppen durch R³C=CR³, C≡C, Si(R³)₂, C=O, NR³, O, S oder CONR³ ersetzt sein können und wobei ein oder mehrere H-Atome durch D oder F ersetzt sein können, oder ein aromatisches oder heteroaromatisches Ringsystem mit 5 bis 24 aromatischen Ringatomen, das jeweils durch einen oder mehrere Reste R³ substituiert sein kann, oder eine Aryloxy- oder Heteroaryloxygruppe mit 5 bis 24 aromatischen Ringatomen, die durch einen oder mehrere Reste R³ substituiert sein kann, oder eine Aralkyl- oder Heteroaralkylgruppe mit 5 bis 24 aromatischen Ringatomen, die durch einen oder mehrere Reste R³ substituiert sein kann; dabei können zwei Reste R⁴, welche an dasselbe Kohlenstoffatom gebunden sind, miteinander ein aliphatisches oder aromatisches Ringsystem bilden und so ein Spirosystem aufspannen; weiterhin kann R⁴ mit einem benachbarten Rest R, R¹ oder R² ein aliphatisches Ringsystem bilden;
mit der Maßgabe, dass in A¹-A²-A³ nicht zwei Heteroatome direkt aneinander gebunden sind.

Wesentlich bei den Gruppen der Formeln (4) und (5) ist, dass diese keine aziden benzylischen Protonen aufweisen. Unter benzylischen Protonen werden Protonen verstanden, die an ein Kohlenstoffatom binden, welches direkt an den heteroaromatischen Liganden gebunden sind. Die Abwesenheit von aziden benzylischen Protonen wird in Formel (4) dadurch erreicht, dass A¹ und A³, wenn diese für C(R⁴)₂ stehen, so definiert sind, dass R⁴ ungleich Wasserstoff ist. Die Abwesenheit von aziden benzylischen Protonen ist in Formel (5) automatisch dadurch erreicht, dass es sich dabei um eine bicyclische Struktur handelt. Aufgrund der starren räumlichen Anordnung ist R², wenn es für H steht, deutlich weniger azide als benzylische Protonen, da das korrespondierende Anion der bicyclischen Struktur nicht mesomeriestabilisiert ist. Auch wenn R² in Formel (5) für H steht, handelt es sich dabei um ein nicht-azides Proton im Sinne der vorliegenden Anmeldung.

Weiterhin bevorzugt ist ein ankondesierter aliphatischer Sechsring oder Siebenring, der bevorzugt keine benzylischen Protonen aufweist.

In einer bevorzugten Ausführungsform der Struktur gemäß Formel (4) steht maximal eine der Gruppen A¹, A² und A³ für ein Heteroatom, insbesondere für O oder NR⁴, und die anderen beiden Gruppen stehen für C(R⁴)₂ bzw. C(R²)₂ oder A¹ und A³ stehen gleich oder verschieden bei jedem Auftreten für O oder NR⁴ und A² steht für C(R²)₂. In einer besonders bevorzugten Ausführungsform der Erfindung stehen A¹ und A³ gleich oder verschieden bei jedem Auftreten für C(R⁴)₂ und A² steht für C(R²)₂ und besonders bevorzugt für C(R⁴)₂.

In einer bevorzugten Ausführungsform der Struktur gemäß Formel (5) stehen die Reste R², die an den Brückenkopf gebunden sind, für H, D, F oder CH₃. Weiterhin bevorzugt steht A² für C(R²)₂ oder O, und besonders bevorzugt für C(R⁴)₂. Weiterhin bevorzugt steht die Gruppe G in Formel (5) für eine Ethylengruppe, welche mit einem oder mehreren Resten R³ substituiert sein kann, wobei R³ bevorzugt gleich oder verschieden bei jedem Auftreten für H oder eine Alkylgruppe mit 1 bis 4 C-Atomen steht, oder eine ortho-Arylengruppe mit 6 bis 10 C-Atomen, welche mit einem oder mehreren Resten R³ substituiert sein kann, bevorzugt aber unsubstituiert ist, insbesondere eine ortho-Phenylengruppe, welche mit einem oder mehreren Resten R³ substituiert sein kann, bevorzugt aber unsubstituiert ist.

In einer weiteren bevorzugten Ausführungsform der Erfindung steht R⁴ in den Gruppen der Formel (4) und (5) und in den bevorzugten Ausführungsformen gleich oder verschieden bei jedem Auftreten für F, eine geradkettige Alkylgruppe mit 1 bis 10 C-Atomen oder eine verzweigte oder cyclische Alkylgruppe mit 3 bis 20 C-Atomen, wobei jeweils eine oder mehrere nicht benachbarte CH₂-Gruppen durch R³C=CR³ ersetzt sein können und ein oder mehrere H-Atome durch D oder F ersetzt sein können, oder ein aromatisches oder heteroaromatisches Ringsystem mit 5 bis 14 aromatischen Ringatomen, das jeweils durch einen oder mehrere Reste R³ substituiert sein kann; dabei können zwei Reste R⁴, welche an dasselbe Kohlenstoffatom gebunden sind, miteinander ein aliphatisches oder aromatisches Ringsystem bilden und so ein Spirosystem aufspannen.

In einer besonders bevorzugten Ausführungsform der Erfindung steht R⁴ in den Gruppen der Formeln (4) und (5) und in den bevorzugten Ausführungsformen gleich oder verschieden bei jedem Auftreten für F, eine geradkettige Alkylgruppe mit 1 bis 3 C-Atomen, insbesondere Methyl, oder ein aromatisches oder heteroaromatisches Ringsystem mit 5 bis 12 aromatischen Ringatomen, das jeweils durch einen oder mehrere Reste R³ substituiert sein kann, bevorzugt aber unsubstituiert ist; dabei können zwei Reste R⁴, welche an dasselbe Kohlenstoffatom gebunden sind, miteinander ein aliphatisches oder aromatisches Ringsystem bilden und so ein Spirosystem aufspannen.

Beispiele für besonders geeignete Gruppen der Formel (4) sind die im Folgenden aufgeführten Gruppen (4-1) bis (4-69):

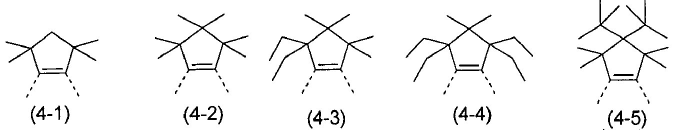

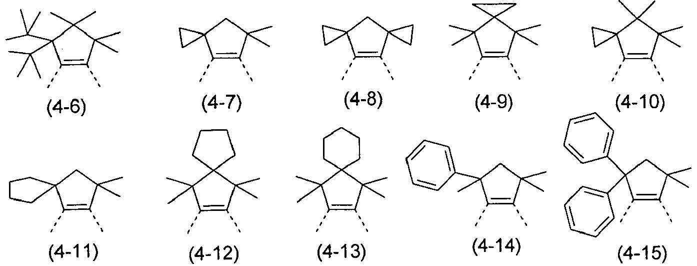

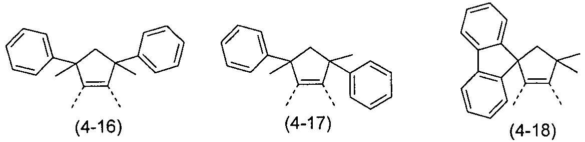

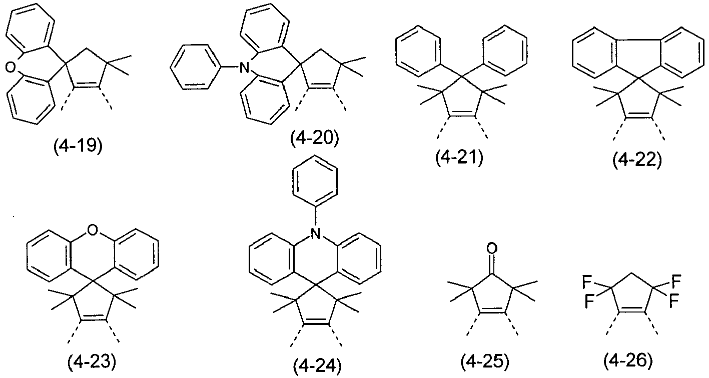

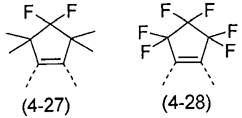

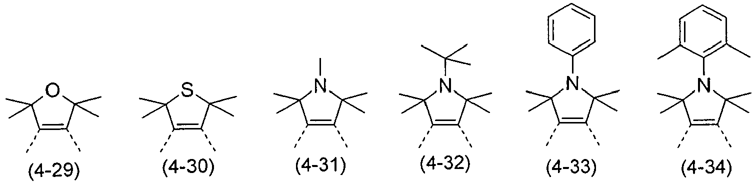

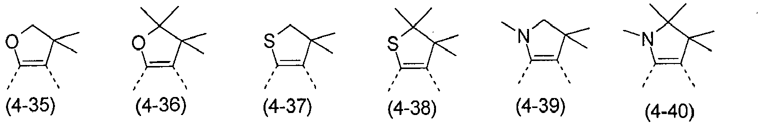

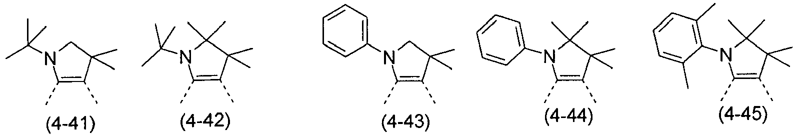

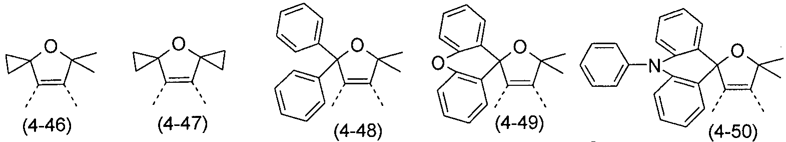

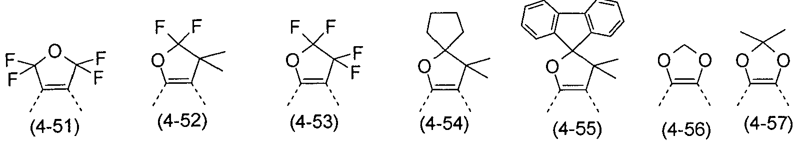

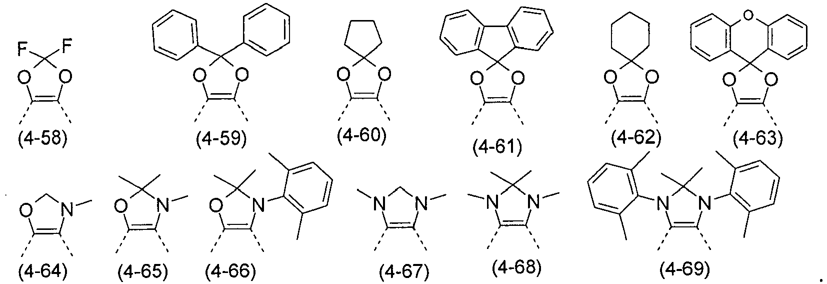

Beispiele für besonders geeignete Gruppen der Formel (5) sind die im Folgenden aufgeführten Gruppen (5-1) bis (5-21):

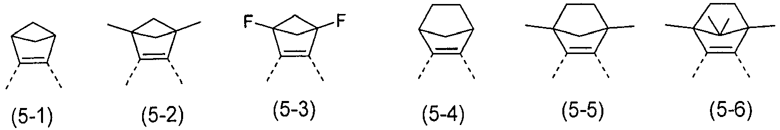

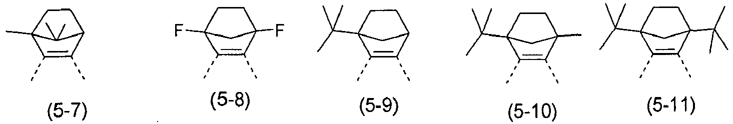

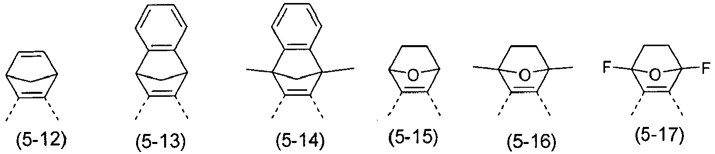

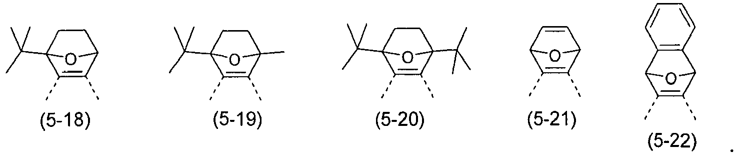

Wenn in der Teilstruktur der Formel (2) oder (3) noch weitere bzw. andere Reste R gebunden sind, so sind diese Reste R bei jedem Auftreten gleich oder verschieden bevorzugt ausgewählt aus der Gruppe bestehend aus H, D, F, Br, I, N(R²)₂, CN, Si(R²)₃, B(OR²)₂, C(=O)R², einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, die jeweils mit einem oder mehreren Resten R² substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils durch einen oder mehrere Reste R² substituiert sein kann; dabei können zwei benachbarte Rest R oder R mit R² auch miteinander ein mono- oder polycyclisches, aliphatisches oder aromatisches Ringsystem bilden. Besonders bevorzugt sind diese Reste R bei jedem Auftreten gleich oder verschieden ausgewählt aus der Gruppe bestehend aus H, D, F, CN, einer geradkettigen Alkylgruppe mit 1 bis 6 C-Atomen oder einer verzweigten oder cyclischen Alkylgruppe mit 3 bis 10 C-Atomen, wobei ein oder mehrere H-Atome durch D oder F ersetzt sein können, oder einem aromatischen oder heteroaromatischen, insbesondere einem aromatischen Ringsystem mit 5 bis 24 aromatischen Ringatomen, das jeweils durch einen oder mehrere Reste R² substituiert sein kann; dabei können zwei benachbarte Reste R oder R mit R² auch miteinander ein mono- oder polycyclisches, aliphatisches oder aromatisches Ringsystem bilden.

Weiterhin ist es möglich, dass der Substituent R, der in der ortho-Position zur Metallkoordination gebunden ist, eine Gruppe darstellt, die ebenfalls an das Metall M koordiniert bzw. bindet. Bevorzugte koordinierende Gruppen R sind Aryl- bzw. Heteroarylgruppen, beispielsweise Phenyl oder Pyridyl, Aryl- oder Alkylcyanide, Aryl- oder Alkylisocyanide, Amine oder Amide, Alkohole oder Alkoholate, Thioalkohole oder Thioalkoholate, Phosphine, Phosphite, Carbonylfunktionen, Carboxylate, Carbamide oder Aryl- oder Alkylacetylide. Beispiele für Teilstrukturen ML der Formel (2) sind die Strukturen der folgenden Formeln (6) bis (11): 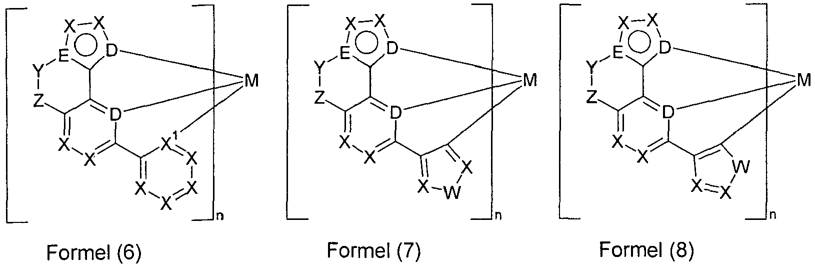 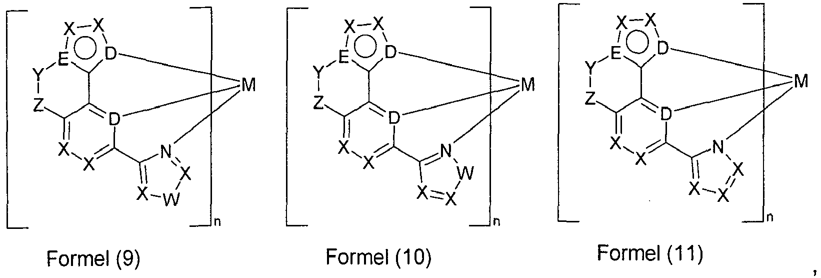 wobei die verwendeten Symbole und Indizes die gleichen Bedeutungen aufweisen, wie oben beschrieben, X¹ gleich oder verschieden bei jedem Auftreten für C oder N steht und W gleich oder verschieden bei jedem Auftreten für S, O oder NR² steht.

Die Formeln (6) bis (11) zeigen nur exemplarisch, wie der Substituent R zusätzlich an das Metall koordinieren kann. Ganz analog sind ohne weiteres erfinderisches Zutun auch andere an das Metall koordinierende Gruppen R zugänglich, beispielsweise auch Carbene. Ebenso sind ganz analog entsprechende Teilstrukturen ML möglich, die auf Formel (3) basieren.

Wie oben beschrieben, kann auch statt einem der Reste R eine verbrückende Einheit vorhanden sein, die diesen Liganden L mit einem oder mehreren weiteren Liganden L bzw. L' verknüpft. In diesem Fall steht L bzw. L' nicht für einen separaten Liganden, sondern für eine koordinierende Gruppe. In einer bevorzugten Ausführungsform der Erfindung ist statt einem der Reste R, insbesondere statt der Reste R, die in ortho- oder meta-Position zum koordinierenden Atom stehen, eine verbrückende Einheit vorhanden, so dass die Liganden dreizähnigen, mehrzähnigen oder polypodalen Charakter aufweisen. Es können auch zwei solcher verbrückenden Einheiten vorhanden sein. Dies führt zur Bildung makrocyclischer Liganden bzw. zur Bildung von Kryptanden.

Bevorzugte Strukturen mit mehrzähnigen Liganden bzw. mit polydentaten Liganden sind die Metallkomplexe der folgenden Formeln (12) bis (23), 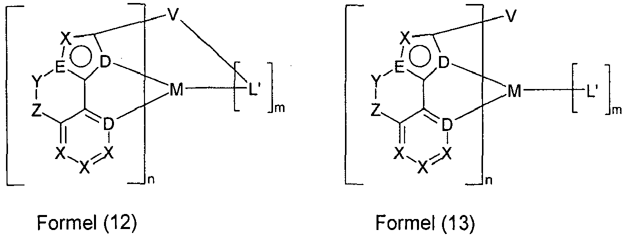 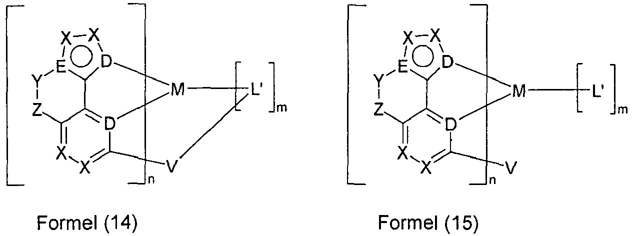 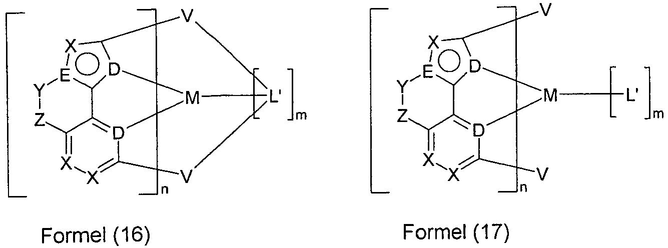 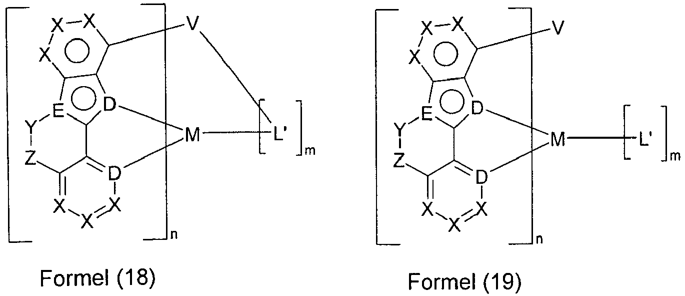 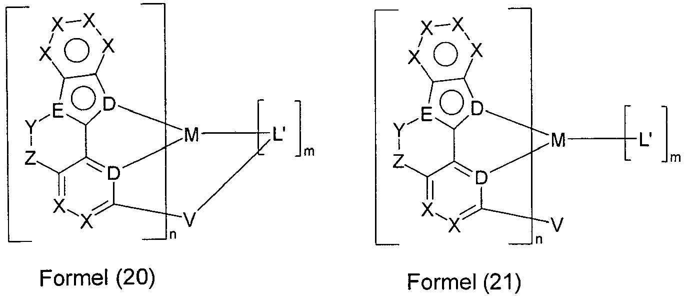 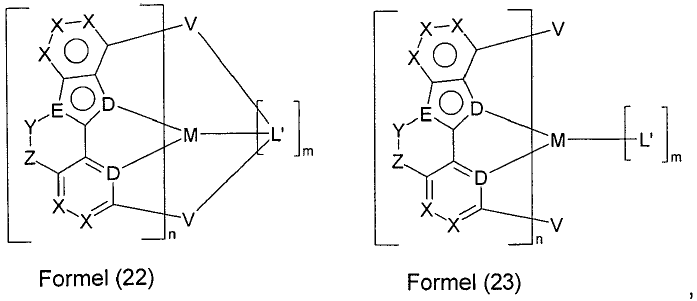 wobei die verwendeten Symbole und Indizes die oben genannten Bedeutungen aufweisen.

Dabei stellt V bevorzugt eine Einfachbindung oder eine verbrückende Einheit dar, enthaltend 1 bis 80 Atome aus der dritten, vierten, fünften und/oder sechsten Hauptgruppe (Gruppe 13, 14, 15 oder 16 gemäß IUPAC) oder einen 3- bis 6-gliedrigen Homo- oder Heterocyclus, die die Teilliganden L miteinander oder L mit L' miteinander kovalent verbindet. Dabei kann die verbrückende Einheit V auch unsymmetrisch aufgebaut sein, d. h. die Verknüpfung von V zu L bzw. L' muss nicht identisch sein. Die verbrückende Einheit V kann neutral, einfach, zweifach oder dreifach negativ oder einfach, zweifach oder dreifach positiv geladen sein. Bevorzugt ist V neutral oder einfach negativ oder einfach positiv geladen, besonders bevorzugt neutral. Dabei wird die Ladung von V bevorzugt so gewählt, dass insgesamt ein neutraler Komplex entsteht. Dabei gelten für die Liganden die oben für die Teilstruktur MLₙ genannten Bevorzugungen und n ist bevorzugt mindestens 2.

Die genaue Struktur und chemische Zusammensetzung der Gruppe V hat keinen wesentlichen Einfluss auf die elektronischen Eigenschaften des Komplexes, da die Aufgabe dieser Gruppe im Wesentlichen darin liegt, durch die Verbrückung von L miteinander bzw. mit L' die chemische und thermische Stabilität der Komplexe zu erhöhen.

Wenn V eine trivalente Gruppe ist, also drei Liganden L miteinander bzw. zwei Liganden L mit L' oder einen Liganden L mit zwei Liganden L' verbrückt, ist V bevorzugt gleich oder verschieden bei jedem Auftreten gewählt aus der Gruppe bestehend aus B, B(R²)-, B(C(R²)₂)₃, (R²)B(C(R²)₂)₃⁻, B(O)₃, (R²)B(O)₃⁻, B(C(R²)₂C(R²)₂)₃, (R²)B(C(R²)₂C(R²)₂)₃⁻, B(C(R²)₂O)₃, (R²)B(C(R²)₂O)₃⁻, B(OC(R²)₂)₃, (R²)B(OC(R²)₂)₃⁻, C(R²), CO⁻, CN(R²)₂, (R²)C(C(R²)₂)₃, (R²)C(O)₃, (R²)C(C(R₂)₂C(R²)₂)₃, (R²)C(C(R²)₂O)₃, (R²)C(OC(R²)₂)₃, (R²)C(Si(R²)₂)₃, (R²)C(Si(R²)₂C(R²)₂)₃, (R²)C(C(R²)₂Si(R²)₂)₃, (R²)C(Si(R²)₂Si(R²)₂)₃, Si(R²), (R²)Si(C(R²)₂)₃, (R²)Si(O)₃, (R²)Si(C(R²)₂C(R²)₂)₃, (R²)Si(OC(R²)₂)₃, (R²)Si(C(R²)₂O)₃, (R²)Si(Si(R²)₂)₃, (R²)Si(Si(R²)₂C(R²)₂)₃, (R²)Si(C(R²)₂Si(R²)₂)₃, (R²)Si(Si(R²)₂Si(R²)₂)₃, N, NO, N(R²)⁺, N(C(R²)₂)₃, (R²)N(C(R²)₂)₃⁺, N(C=O)₃, N(C(R²)₂C(R²)₂)₃, (R²)N(C(R²)₂C(R²)₂)⁺, P, P(R²)⁺, PO, PS, P(O)₃, PO(O)₃, P(OC(R²)₂)₃, PO(OC(R²)₂)₃, P(C(R²)₂)₃, P(R²)(C(R²)₂)₃⁺, PO(C(R²)₂)₃, P(C(R²)₂C(R²)₂)₃, P(R²)(C(R²)₂C(R²)₂)₃⁺, PO(C(R²)₂C(R²)₂)₃, S⁺, S(C(R²)₂)₃⁺, S(C(R²)₂C(R²)₂)₃⁺, oder eine Einheit gemäß einer der Formel (24) bis (28), 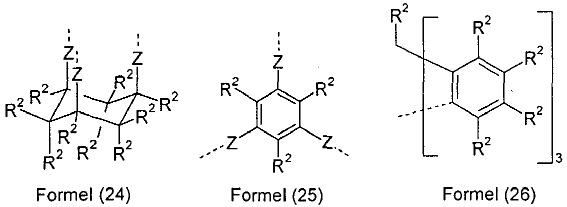 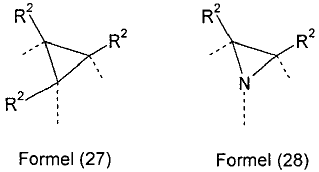 wobei die gestrichelten Bindungen jeweils die Bindung zu den Teilliganden L bzw. L' andeuten und Z gleich oder verschieden bei jedem Auftreten ausgewählt ist aus der Gruppe bestehend aus einer Einfachbindung, O, S, S(=O), S(=O)₂, NR², PR², P(=O)R², C(R²)₂, C(=O), C(=NR²), C(=C(R²)₂), Si(R²)₂ oder BR². Die weiteren verwendeten Symbole haben die oben genannten Bedeutungen.

Wenn V eine bivalente Gruppe ist, also zwei Liganden L miteinander bzw. einen Liganden L mit L' verbrückt, ist V bevorzugt gleich oder verschieden bei jedem Auftreten gewählt aus der Gruppe bestehend aus BR², B(R²)₂⁻, C(R²)₂, C(=O), Si(R²)₂, NR², PR², P(R²)₂⁺, P(=O)(R²), P(=S)(R²), O, S, Se, oder eine Einheit gemäß Formel (29) bis (38), 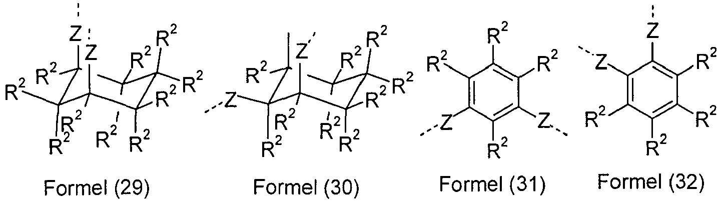 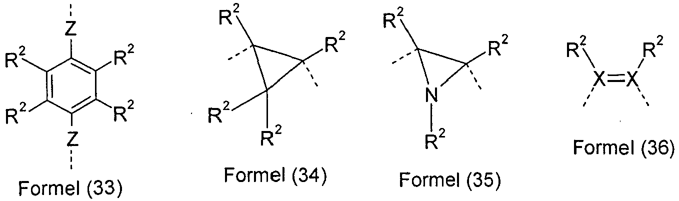 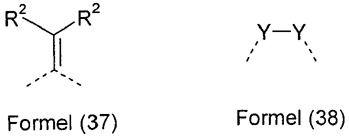 wobei die gestrichelten Bindungen jeweils die Bindung zu den Teilliganden L bzw. L' andeuten, Y bei jedem Auftreten gleich oder verschieden für C(R²)₂, N(R²), O oder S steht und die weiteren verwendeten Symbole jeweils die oben aufgeführten Bedeutungen haben.

Wenn V für eine Gruppe CR₂ steht, so können die beiden Reste R auch miteinander verknüpft sein, so dass auch Strukturen wie zum Beispiele 9,9-Fluoren geeignete Gruppen V sind.

Im Folgenden werden bevorzugte Liganden L' beschrieben, wie sie in Formel (1) vorkommen. Entsprechend können auch die Ligandengruppen L' gewählt sein, wenn diese über eine verbrückende Einheit V an L gebunden sind, wie in Formeln (12), (14), (16), (18), (20) und (22) angedeutet, wenn diese Gruppen eine freie Valenz zur Bindung an V aufweisen.

Die Liganden L' sind bevorzugt neutrale, monoanionische, dianionische oder trianionische Liganden, besonders bevorzugt neutrale oder monoanionische Liganden. Sie können monodentat, bidentat, tridentat oder tetradentat sein und sind bevorzugt bidentat, weisen also bevorzugt zwei Koordinationsstellen auf. Wie oben beschrieben, können die Liganden L' auch über eine verbrückende Gruppe V an L gebunden sein.

Bevorzugte neutrale, monodentate Liganden L' sind ausgewählt aus der Gruppe bestehend aus Kohlenmonoxid, Stickstoffmonoxid, Alkylcyaniden, wie z. B. Acetonitril, Arylcyaniden, wie z. B. Benzonitril, Alkylisocyaniden, wie z. B. Methylisonitril, Arylisocyaniden, wie z. B. Benzoisonitril, Aminen, wie z. B. Trimethylamin, Triethylamin, Morpholin, Phosphinen, insbesondere Halogenphosphine, Trialkylphosphine, Triarylphosphine oder Alkylarylphosphine, wie z. B. Trifluorphosphin, Trimethylphosphin, Tricyclohexylphosphin, Tri-*tert*-butylphosphin, Triphenylphosphin, Tris(pentafluorphenyl)phosphin, Dimethylphenylphosphin, Methyldiphenylphosphin, Bis(tert-butyl)phenylphosphin, Phosphiten, wie z. B. Trimethylphosphit, Triethylphosphit, Arsinen, wie z. B. Trifluorarsin, Trimethylarsin, Tricyclohexylarsin, Tri-*tert*-butylarsin, Triphenylarsin, Tris(pentafluorphenyl)arsin, Stibinen, wie z. B. Trifluorstibin, Trimethylstibin, Tricyclohexylstibin, Tri-tert-butylstibin, Triphenylstibin, Tris(pentafluorphenyl)stibin, stickstoffhaltigen Heterocyclen, wie z. B. Pyridin, Pyridazin, Pyrazin, Pyrimidin, Triazin, und Carbenen, insbesondere Arduengo-Carbenen.

Bevorzugte monoanionische, monodentate Liganden L' sind ausgewählt aus Hydrid, Deuterid, den Halogeniden F⁻, Cl⁻, Br⁻ und I⁻, Alkylacetyliden, wie z. B. Methyl-C≡C⁻, tert-Butyl-C≡C⁻, Arylacetyliden, wie z. B. Phenyl-C≡C⁻, Cyanid, Cyanat, Isocyanat, Thiocyanat, Isothiocyanat, aliphatischen oder aromatischen Alkoholaten, wie z. B. Methanolat, Ethanolat, Propanolat, iso-Propanolat, *tert*-Butylat, Phenolat, aliphatischen oder aromatischen Thioalkoholaten, wie z. B. Methanthiolat, Ethanthiolat, Propanthiolat, *iso*-Propanthiolat, *tert*-Thiobutylat, Thiophenolat, Amiden, wie z. B. Dimethylamid, Diethylamid, Di-*iso*-propylamid, Morpholid, Carboxylaten, wie z. B. Acetat, Trifluoracetat, Propionat, Benzoat, Arylgruppen, wie z. B. Phenyl, Naphthyl, und anionischen, stickstoffhaltigen Heterocyclen, wie Pyrrolid, Imidazolid, Pyrazolid. Dabei sind die Alkylgruppen in diesen Gruppen bevorzugt C₁-C₂₀-Alkylgruppen, besonders bevorzugt C₁-C₁₀-Alkylgruppen, ganz besonders bevorzugt C₁-C₄-Alkylgruppen. Unter einer Arylgruppe werden auch Heteroarylgruppen verstanden. Diese Gruppen sind wie oben definiert.

Bevorzugte di- bzw. trianionische Liganden sind O²⁻, S²⁻, Carbide, welche zu einer Koordination der Form R-C≡M führen, und Nitrene, welche zu einer Koordination der Form R-N=M führen, wobei R allgemein für einen Substituenten steht, oder N³⁻.

Bevorzugte neutrale oder mono- oder dianionische, bidentate oder höherdentate Liganden L'sind ausgewählt aus Diaminen, wie z. B. Ethylendiamin, N,N,N',N'-Tetramethylethylendiamin, Propylendiamin, N,N,N',N'-Tetramethylpropylendiamin, cis- oder trans-Diaminocyclohexan, cis- oder trans-N,N,N',N'-Tetramethyldiaminocyclohexan, Iminen, wie z. B. 2-[1-(Phenylimino)ethyl]pyridin, 2-[1-(2-Methylphenylimino)ethyl]pyridin, 2-[1-(2,6-Di-iso-propylphenylimino)ethyl]pyridin, 2-[1-(Methylimino)ethyl]pyridin, 2-[1-(ethylimino)ethyl]pyridin, 2-[1-(*Iso*-Propylimino)ethyl]pyridin, 2-[1-(*Tert-*Butylimino)ethyl]pyridin, Diiminen, wie z. B. 1,2-Bis(methylimino)ethan, 1,2-Bis(ethylimino)ethan, 1,2-Bis(*iso*-propylimino)ethan, 1,2-Bis(*tert*-butyl-imino)ethan, 2,3-Bis(methylimino)butan, 2,3-Bis(ethylimino)butan, 2,3-Bis-(iso-propylimino)butan, 2,3-Bis(*tert*-butylimino)butan, 1,2-Bis(phenylimino)-ethan, 1,2-Bis(2-methylphenylimino)ethan, 1,2-Bis(2,6-di-iso-propylphenyl-imino)ethan, 1,2-Bis(2,6-di-*tert*-butylphenylimino)ethan, 2,3-Bis(phenyl-imino)butan, 2,3-Bis(2-methylphenylimino)butan, 2,3-Bis(2,6-di-iso-propyl-phenylimino)butan, 2,3-Bis(2,6-di-*tert*-butylphenylimino)butan, Heterocyclen enthaltend zwei Stickstoffatome, wie z. B. 2,2'-Bipyridin, o-Phenanthrolin, Diphosphinen, wie z. B. Bis(diphenylphosphino)methan, Bis(diphenylphosphino)ethan, Bis(diphenylphosphino)propan, Bis(diphenylphosphino)butan, Bis(dimethylphosphino)methan, Bis(dimethylphosphino)ethan, Bis(dimethylphosphino)propan, Bis(diethylphosphino)-methan, Bis(diethylphosphino)ethan, Bis(diethylphosphino)propan, Bis(di*tert*-butylphosphino)methan, Bis(di-*tert*-butylphosphino)ethan, Bis(*tert-*butylphosphino)propan, 1,3-Diketonaten abgeleitet von 1,3-Diketonen, wie z. B. Acetylaceton, Benzoylaceton, 1,5-Diphenylacetylaceton, Dibenzoylmethan, Bis(1,1,1-trifluoracetyl)methan, 3-Ketonaten abgeleitet von 3-Ketoestern, wie z. B. Acetessigsäureethylester, Carboxylate, abgeleitet von Aminocarbonsäuren, wie z. B. Pyridin-2-carbonsäure, Chinolin-2-carbonsäure, Glycin, N,N-Dimethylglycin, Alanin, N,N-Dimethylamino-alanin, Salicyliminaten abgeleitet von Salicyliminen, wie z. B. Methylsalicylimin, Ethylsalicylimin, Phenylsalicylimin, Dialkoholaten abgeleitet von Dialkoholen, wie z. B. Ethylenglykol, 1,3-Propylenglykol, Dithiolaten abgeleitet von Dithiolen, wie z. B. 1,2-Ethylendithiol, 1,3-Propylendithiol, Bis(pyrazolylboraten), Bis(imidazolyl)boraten, 3-(2-Pyridyl)-diazolen oder 3-(2-Pyridyl)-triazolen.

Bevorzugte tridentate Liganden sind Borate stickstoffhaltiger Heterocyclen, wie z. B. Tetrakis(1-imidazolyl)borat und Tetrakis(1-pyrazolyl)borat.

Bevorzugt sind weiterhin bidentate monoanionische, neutrale oder dianionische Liganden L', insbesondere monoanionische Liganden, welche mit dem Metall einen cyclometallierten Fünfring oder Sechsring mit mindestens einer Metall-Kohlenstoff-Bindung aufweisen, insbesondere einen cyclometallierten Fünfring. Dies sind insbesondere Liganden, wie sie allgemein im Gebiet der phosphoreszierenden Metallkomplexe für organische Elektrolumineszenzvorrichtungen verwendet werden, also Liganden vom Typ Phenylpyridin, Naphthylpyridin, Phenylchinolin, Phenylisochinolin, etc., welche jeweils durch einen oder mehrere Reste R substituiert sein können. Dem Fachmann auf dem Gebiet der phosphoreszierenden Elektrolumineszenzvorrichtungen ist eine Vielzahl derartiger Liganden bekannt, und er kann ohne erfinderisches Zutun weitere derartige Liganden als Ligand L' für Verbindungen gemäß Formel (1) auswählen. Generell eignet sich dafür besonders die Kombination aus zwei Gruppen, wie sie durch die folgenden Formeln (39) bis (70) dargestellt sind, wobei eine Gruppe bevorzugt über ein neutrales Stickstoffatom oder ein Carbenkohlenstoffatom bindet und die andere Gruppe bevorzugt über ein negativ geladenes Kohlenstoffatom oder ein negativ geladenes Stickstoffatom bindet. Der Ligand L' kann dann aus den Gruppen der Formeln (39) bis (70) gebildet werden, indem diese Gruppen jeweils an der durch # gekennzeichneten Position aneinander binden. Die Position, an der die Gruppen an das Metall koordinieren, sind durch * gekennzeichnet. Diese Gruppen können auch über eine oder zwei verbrückende Einheiten V an den Liganden L gebunden sein.

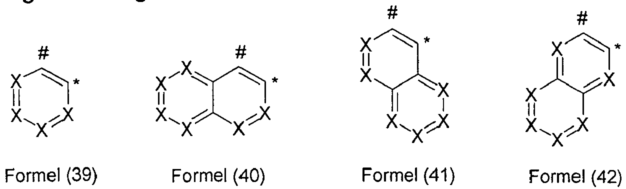

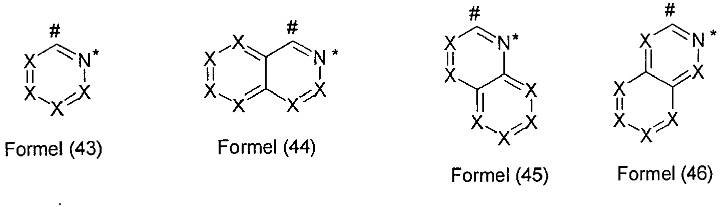

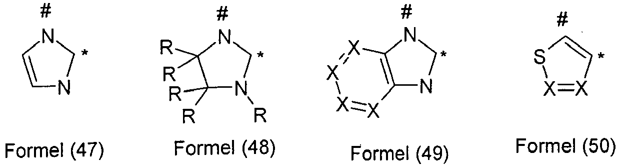

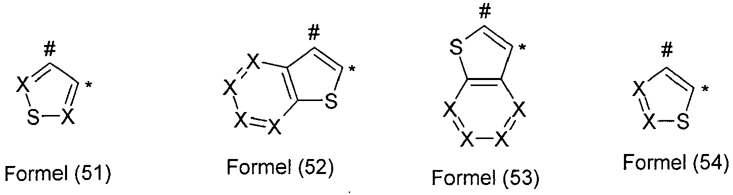

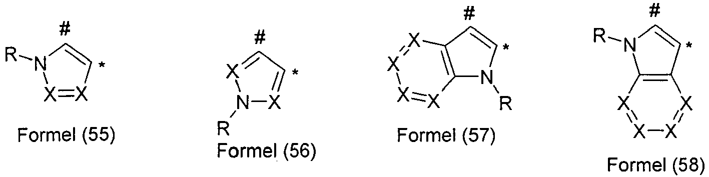

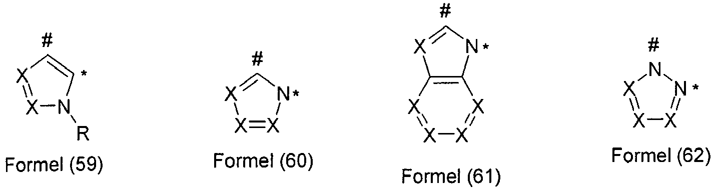

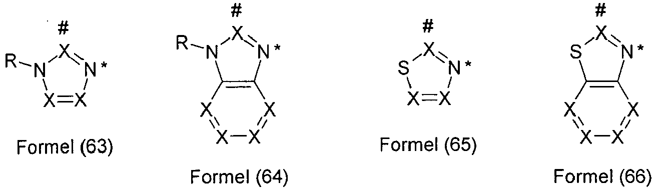

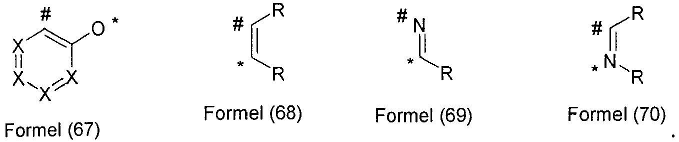

Dabei haben die verwendeten Symbole dieselbe Bedeutung, wie oben beschrieben, und es gilt die oben genannte Bevorzugung für die verwendeten Gruppen. Bevorzugt stehen maximal drei Symbole X in jeder Gruppe für N, besonders bevorzugt stehen maximal zwei Symbole X in jeder Gruppe für N, ganz besonders bevorzugt steht maximal ein Symbol X in jeder Gruppe für N. Insbesondere bevorzugt stehen alle Symbole X für CR.

Weiterhin können die Formeln (50) bis (54), (65) und (66) statt des Schwefels auch Sauerstoff enthalten.

Ebenfalls bevorzugte Liganden L' sind η⁵-Cyclopentadienyl, η⁵-Penta-methylcyclopentadienyl, η⁶-Benzol oder η⁷-Cycloheptatrienyl, welche jeweils durch einen oder mehrere Reste R substituiert sein können.

Ebenfalls bevorzugte Liganden L' sind 1,3,5-cis,cis-Cyclohexanderivate, insbesondere der Formel (71), 1,1,1-Tri(methylen)methanderivate, insbesondere der Formel (72) und 1,1,1-trisubstituierte Methane, insbesondere der Formel (73) und (74), 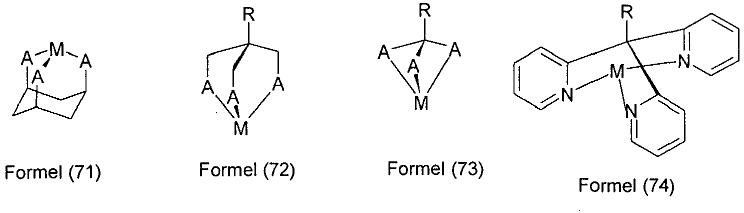 wobei in den Formeln jeweils die Koordination an das Metall M dargestellt ist, R die oben genannte Bedeutung hat und A, gleich oder verschieden bei jedem Auftreten, für O⁻, S⁻, COO⁻, PR₂ oder NR₂ steht.

Die erfindungsgemäßen Komplexe können facial bzw. pseudofacial sein, oder sie können meridional bzw. pseudomeridional sein.

Die Liganden L können je nach Struktur bzw. Substitution auch chiral sein. Dies kann beispielsweise dann der Fall sein, wenn sie eine ankondensierte bicyclische Gruppe als Substituenten enthalten oder wenn sie Substituenten enthalten, beispielsweise Alkyl-, Alkoxy, Dialkylamino- oder Aralkylgruppen, welche ein oder mehrere Stereozentren aufweisen. Da es sich bei der Grundstruktur des Komplexes auch um eine chirale Struktur handeln kann, ist die Bildung von Diastereomeren und mehreren Enantiomerenpaaren möglich. Die erfindungsgemäßen Komplexe umfassen dann sowohl die Mischungen der verschiedenen Diastereomere bzw. die entsprechenden Racemate wie auch die einzelnen isolierten Diastereomere bzw. Enantiomere.

Die oben genannten bevorzugten Ausführungsformen sind beliebig miteinander kombinierbar. In einer besonders bevorzugten Ausführungsform der Erfindung gelten mehrere oder alle der oben genannten bevorzugten Ausführungsformen gleichzeitig.

Beispiele für bevorzugte erfindungsgemäße Verbindungen sind die in der folgenden Tabelle aufgeführten Verbindungen.

| | | |
|---|---|---|
| 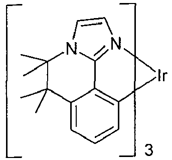 | 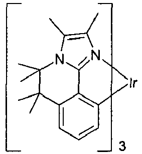 | 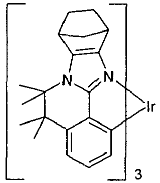 |
| 1 | 2 | 3 |
| 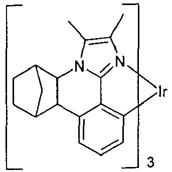 | 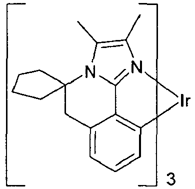 | 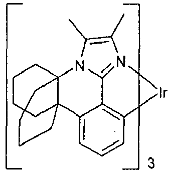 |
| 4 | 5 | 6 |
| 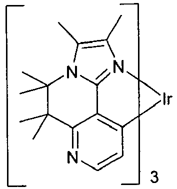 | 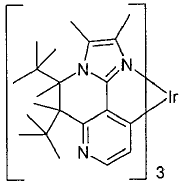 | 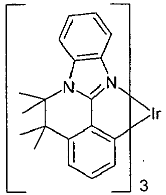 |
| 7 | 8 | 9 |
| 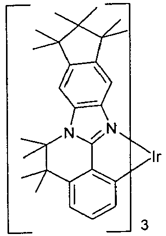 | 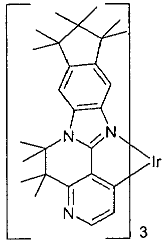 | 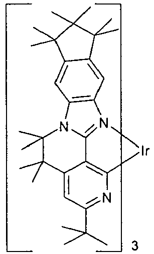 |
| 10 | 11 | 12 |
| 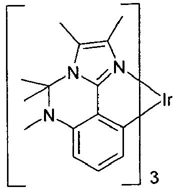 | 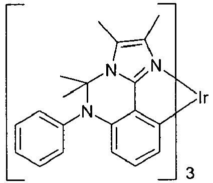 | 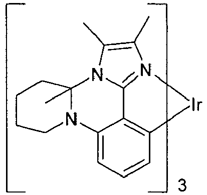 |
| 13 | 14 | 14 |
| 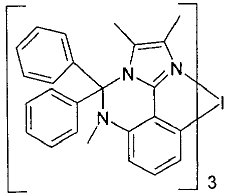 r | 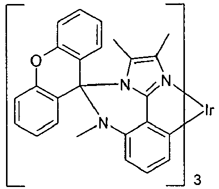 | 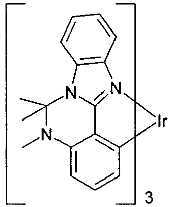 |
| 16 | 17 | 18 |
| 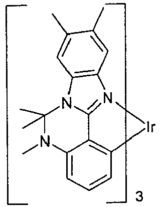 | 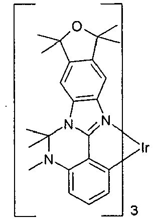 | 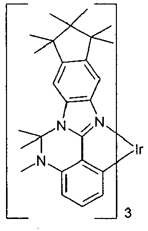 |
| 19 | 20 | 21 |
| 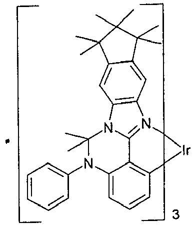 | 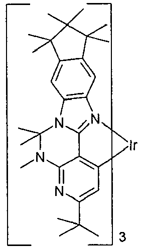 | 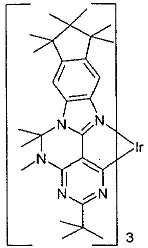 |
| 22 | 23 | 24 |
| 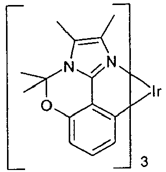 | 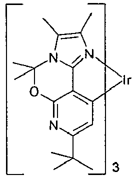 | 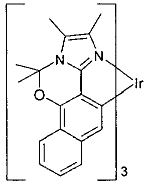 |
| 25 | 26 | 27 |
| 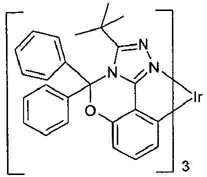 | 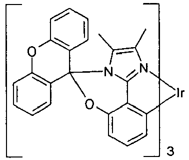 | 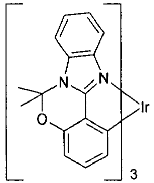 |
| 28 | 29 | 30 |
| 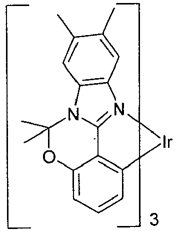 | 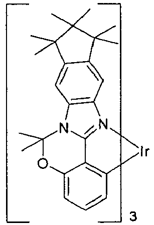 | 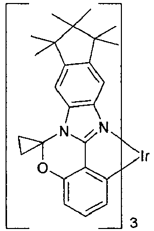 |
| 31 | 32 | 33 |
| 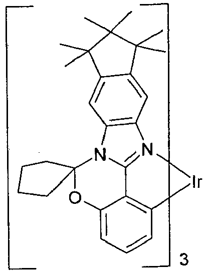 | 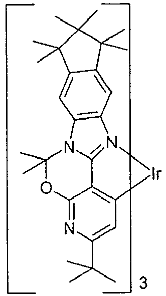 | 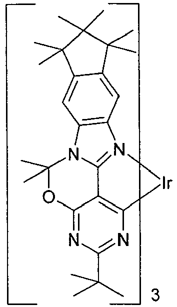 |
| 34 | 35 | 36 |
| 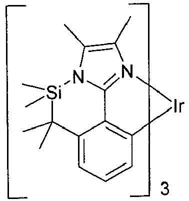 | 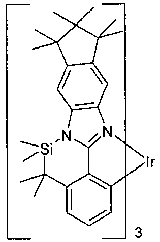 | 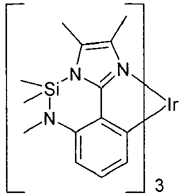 |
| 37 | 38 | 39 |
| 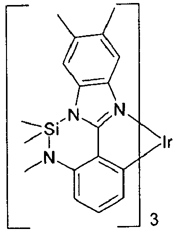 | 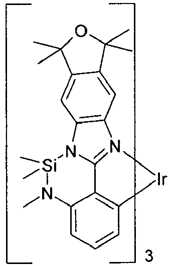 | 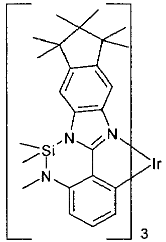 |
| 40 | 41 | 42 |
| 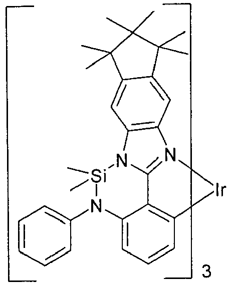 | 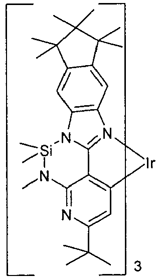 | 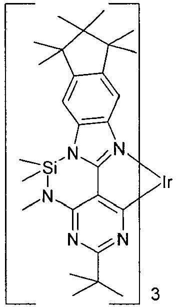 |
| 43 | 44 | 45 |
| 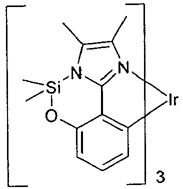 | 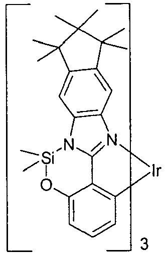 | 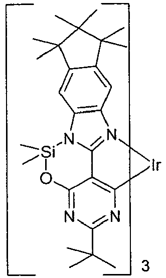 |
| 46 | 47 | 48 |
| 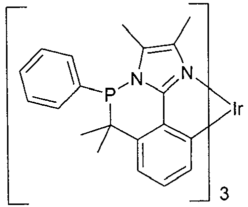 | 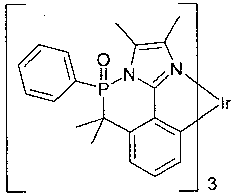 | 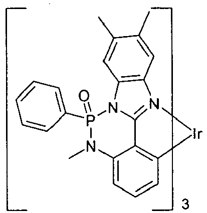 |
| 49 | 50 | 51 |
| 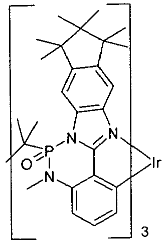 | 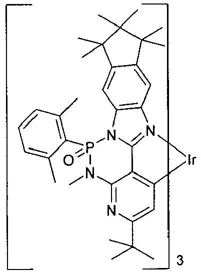 | 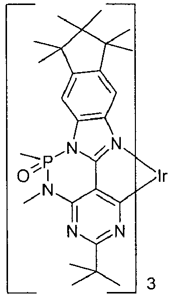 |
| 52 | 53 | 54 |
| 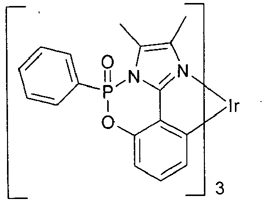 | 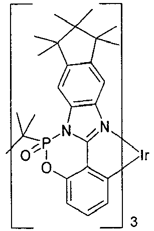 | 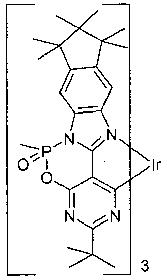 |
| 55 | 56 | 57 |
| 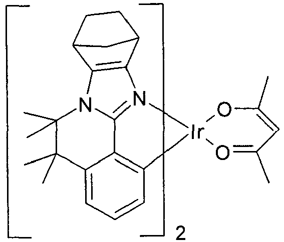 | 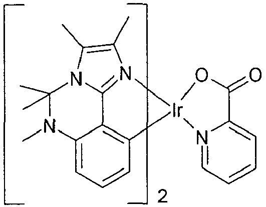 | 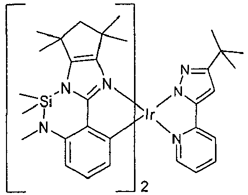 |
| 58 | 59 | 60 |
| 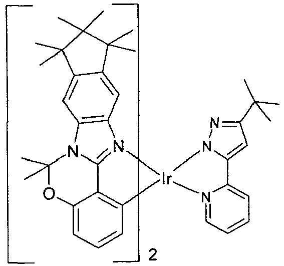 | 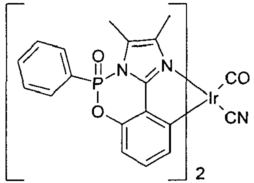 | 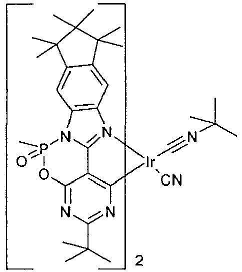 |
| 61 | 62 | 63 |
| 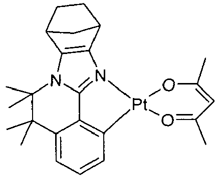 | 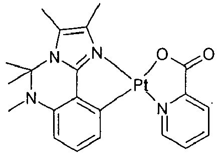 | 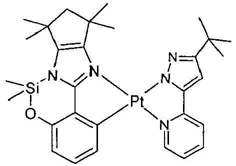 |
| 64 | 65 | 66 |
| 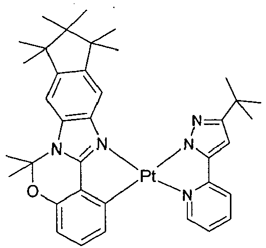 | 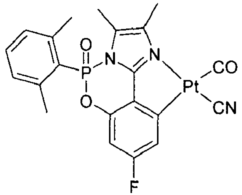 | 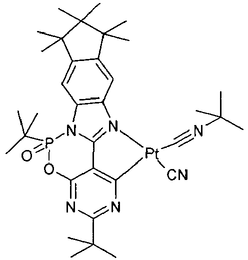 |
| 68 | 69 | 70 |

Die erfindungsgemäßen Metallkomplexe sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen gemäß Formel (1) durch Umsetzung der entsprechenden freien Liganden mit Metallalkoholaten der Formel (75), mit Metallketoketonaten der Formel (76), mit Metallhalogeniden der Formel (77), mit dimeren Metallkomplexen der Formel (78) oder mit Metallkomplexen der Formel (79), 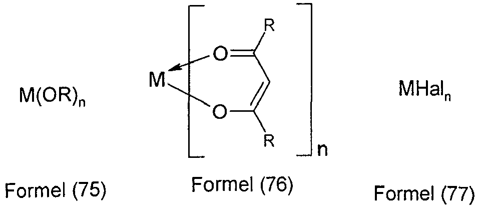 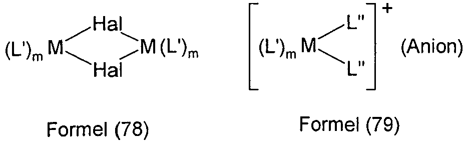 wobei die Symbole M, m, n und R die oben angegebenen Bedeutungen haben, Hal = F, Cl, Br oder I ist, L" für einen Alkohol, insbesondere für einen Alkohol mit 1 bis 4 C-Atomen oder ein Nitril, insbesondere Acetonitril oder Benzonitril, steht und (Anion) ein nicht-koordinierendes Anion ist, wie beispielsweise Triflat.

Es können ebenfalls Metallverbindungen, insbesondere Iridiumverbindungen, die sowohl Alkoholat- und/oder Halogenid- und/oder Hydroxy- wie auch Ketoketonatreste tragen, verwendet werden. Diese Verbindungen können auch geladen sein. Entsprechende Iridiumverbindungen, die als Edukte besonders geeignet sind, sind in WO 2004/085449 offenbart. Besonders geeignet sind [IrCl₂(acac)₂]⁻, beispielsweise Na[IrCl₂(acac)₂], Metallkomplexe mit Acetylacetonat-Derivaten als Ligand, beispielsweise Ir(acac)₃ oder Tris(2,2,6,6-Tetramethylheptan-3,5-dionato)iridium, und IrCl₃·xH₂O, wobei x üblicherweise für eine Zahl zwischen 2 und 4 steht.

Geeignete Platin-Edukte sind beispielsweise PtCl₂, K₂[PtCl₄], PtCl₂(DMSO)₂, Pt(Me)₂(DMSO)₂ oder PtCl₂(Benzonitril)₂.

Die Synthese der Komplexe wird bevorzugt durchgeführt wie in WO 2002/060910, WO 2004/085449 und WO 2007/065523 beschrieben. Heteroleptische Komplexe können beispielsweise auch gemäß WO 2005/042548 synthetisiert werden. Dabei kann die Synthese beispielsweise auch thermisch, photochemisch, im Autoklaven und/oder durch Mikrowellenstrahlung aktiviert werden. In einer bevorzugten Ausführungsform der Erfindung wird die Reaktion ohne die Verwendung eines zusätzlichen Lösemittels in der Schmelze durchgeführt. Dabei bedeutet "Schmelze", dass der Ligand geschmolzen vorliegt und die Metall-Vorstufe in dieser Schmelze gelöst oder suspendiert ist. Zur Aktivierung der Reaktion ist es weiterhin auch möglich, eine Lewis-Säure, beispielsweise ein Silbersalz oder AlCl₃, zuzugeben.

Durch diese Verfahren, gegebenenfalls gefolgt von Aufreinigung, wie z. B. Umkristallisation oder Sublimation, lassen sich die erfindungsgemäßen Verbindungen gemäß Formel (1) in hoher Reinheit, bevorzugt mehr als 99 % (bestimmt mittels ¹H-NMR und/oder HPLC) erhalten.

Die erfindungsgemäßen Verbindungen können auch durch geeignete Substitution, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, löslich gemacht werden. Solche Verbindungen sind dann in gängigen organischen Lösemitteln, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei ein oder mehrere Bindungen der erfindungsgemäßen Verbindung zum Polymer, Oligomer oder Dendrimer vorhanden sind. Je nach Verknüpfung der erfindungsgemäßen Verbindung bildet diese daher eine Seitenkette des Oligomers oder Polymers oder ist in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (1) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- oder trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethyleneglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung bzw. ein erfindungsgemäßes Oligomer, Polymer oder Dendrimer und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Die oben beschriebenen Komplexe gemäß Formel (1) bzw. die oben aufgeführten bevorzugten Ausführungsformen können in der elektronischen Vorrichtung als aktive Komponente verwendet werden. Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung der oben aufgeführten Formel (1) enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser), enthaltend in mindestens einer Schicht mindestens eine Verbindung gemäß der oben aufgeführten Formel (1). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäßen Verbindungen zeigen besonders gute Eigenschaften als Emissionsmaterial in organischen Elektrolumineszenzvorrichtungen. Eine bevorzugte Ausführungsform der Erfindung sind daher organische Elektrolumineszenzvorrichtungen. Weiterhin können die erfindungsgemäßen Verbindungen zur Erzeugung von Singulett-Sauerstoff oder in der Photokatalyse eingesetzt werden.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoO₃ oder WO₃ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die Verbindung gemäß Formel (1) bzw. die oben aufgeführten bevorzugten Ausführungsformen als emittierende Verbindung in einer oder mehreren emittierenden Schichten.

Wenn die Verbindung gemäß Formel (1) als emittierende Verbindung in einer emittierenden Schicht eingesetzt wird, wird sie bevorzugt in Kombination mit einem oder mehreren Matrixmaterialien eingesetzt. Die Mischung aus der Verbindung gemäß Formel (1) und dem Matrixmaterial enthält zwischen 0.1 und 99 Vol.-%, vorzugsweise zwischen 1 und 90 Vol.-%, besonders bevorzugt zwischen 3 und 40 Vol.-%, insbesondere zwischen 5 und 15 Vol.-% der Verbindung gemäß Formel (1) bezogen auf die Gesamtmischung aus Emitter und Matrixmaterial. Entsprechend enthält die Mischung zwischen 99.9 und 1 Vol.-%, vorzugsweise zwischen 99 und 10 Vol.-%, besonders bevorzugt zwischen 97 und 60 Vol.-%, insbesondere zwischen 95 und 85 Vol.-% des Matrixmaterials bezogen auf die Gesamtmischung aus Emitter und Matrixmaterial.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, oder verbrückte Carbazolderivate, z. B. gemäß US 2009/0136779, WO 2010/050778, WO 2011/042107 oder WO 2011/088877.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Eine bevorzugte Kombination ist beispielsweise die Verwendung eines aromatischen Ketons, eines Triazin-Derivats oder eines Phosphinoxid-Derivats mit einem Triarylamin-Derivat oder einem Carbazol-Derivat als gemischte Matrix für den erfindungsgemäßen Metallkomplex. Ebenso bevorzugt ist die Verwendung einer Mischung aus einem ladungstransportierenden, also ein loch- bzw. elektronentransportierendes Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum. So können beispielsweise die erfindungsgemäßen Komplexe gemäß Formel (1) als Co-Matrix für längerwellig emittierende Triplettemitter, beispielsweise für grün oder rot emittierende Triplettemitter, eingesetzt werden.

Die erfindungsgemäßen Verbindungen lassen sich auch in anderen Funktionen in der elektronischen Vorrichtung einsetzen, beispielsweise als Lochtransportmaterial in einer Lochinjektions- oder -transportschicht, als Ladungserzeugungsmaterial oder als Elektronenblockiermaterial. Ebenso lassen sich die erfindungsgemäßen Komplexe als Matrixmaterial für andere phosphoreszierende Metallkomplexe in einer emittierenden Schicht einsetzen.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoO₃ oder WO₃, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer oder noch höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die organische Elektrolumineszenzvorrichtung kann auch als Hybrid-system hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine Verbindung gemäß Formel (1) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend Verbindungen gemäß Formel (1) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Die erfindungsgemäßen Verbindungen eignen sich sehr gut für den Einsatz in elektronischen Vorrichtungen, insbesondere in organischen Elektrolumineszenzvorrichtungen und führen dort zu sehr guten Eigenschaften.
2. Organische Elektrolumineszenzvorrichtungen enthaltend Verbindungen gemäß Formel (1) als emittierende Materialien weisen eine sehr gute Lebensdauer auf.
3. Organische Elektrolumineszenzvorrichtungen enthaltend Verbindungen gemäß Formel (1) als emittierende Materialien weisen eine sehr gute Effizienz auf.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße elektronische Vorrichtungen herstellen und somit die Erfindung im gesamten beanspruchten Bereich ausführen.

### Beispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### A: Synthese der Synthone S:

### Beispiel S1: 1,1,3,3-Tetramethyl-indan-5,6-diamin, [83721-95-3], S1

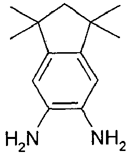

### Variante A:

### A: 5,6-Dibrom-1,1,3,3-tetramethyl-indan, S1a

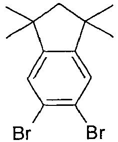

Eine Lösung von 87.2 g (500 mmol) 1,1,3,3-Tetramethyl-indan [4834-33-7] in 2000 ml Dichlormethan wird mit 1.3 g wasserfreiem Eisen(III)chlorid und dann unter Lichtausschluss tropfenweise mit einer Mischung von 64.0 ml (1.25 mol) Brom und 300 ml Dichlormethan so versetzt, dass die Temperatur 25 °C nicht übersteigt, gegebenenfalls wird mit einem Kaltwasserbad gegengekühlt. Man rührt die Reaktionsmischung 16 h bei Raumtemperatur nach, versetzt dann langsam mit 500 ml gesättigter Natriumsulfit-Lösung, trennt die wässrige Phase ab, wäscht die organische Phase dreimal mit je 1000 ml Wasser, trocknet über Natriumsulfat, filtriert über eine kurze Säule aus Kieselgel und zieht dann das Lösungsmittel ab. Abschießend wird der Feststoff einmal aus wenig (ca. 100 ml) Ethanol umkristallisiert. Ausbeute: 121.2 g (365 mmol), 73 %; Reinheit: ca. 95 %ig nach ¹H NMR.

### B: 1,1,3,3-Tetramethyl-indan-5,6-diamin, S1

Ein Gemisch aus 121.2 g (365 mmol) 5,6-Dibrom-1,1,3,3-tetramethyl-indan, 153.2 ml (913 mmol) Benzhydrylidenamin [1013-88-3], 96.1 g (1.0 mol) Natrium-tert-butylat und 1000 ml Toluol wird mit 9.34 g (15 mmol) rac-BINAP und dann mit 3.36 g (15 mmol) Palladium(II)acetat versetzt und anschließend 16 h unter Rückfluss erhitzt. Nach Erkalten fügt man 500 ml Wasser zu, trennt die organische Phase ab, wäscht diese zweimal mit je 500 ml gesättigter Kochsalz-Lösung, rotiert das Toluol ab, nimmt den Rückstand in 500 ml THF auf, fügt 200 ml 2 N Salzsäure zu und erhitzt die Reaktionsmischung 16 h unter Rückfluss. Man entfernt das Lösungsmittel im Vakuum, nimmt den Rückstand in 1000 ml Ethylacetat auf, wäscht die organische Phase mit Natriumhyrdogencarbonat-Lösung bis pH = 7 erreicht ist, tocknet die organische Phase über Magnesiumsulfat, filtriert vom Trockenmittel ab, setzt dem Filtrat 500 g Kieselgel zu und entfernt das Lösungsmittel im Vakuum. Man plaziert das beladene Kieselgel auf einer Kieselgel-Säule (1500 g, aufgeschlämmt in n-Heptan : Ethylacetat, 95:5 vv), eluiert mit n-Heptan : Ethylacetat (95:5 vv) zunächst das Benzophenon, stellt dann auf Ethylacetat um und eluiert das Produkt. Ausbeute: 56.8 g (278 mmol), 76 %; Reinheit: ca. 95 % nach ¹H-NMR.

### Variante B:

### A: 5,6-Dinitro-1,1,3,3-tetramethyl-indan, S1b

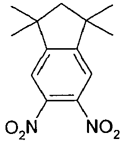

Zu einer gut gerührten, auf 0° C gekühlten Mischung aus 87.2 g (500 mmol) 1,1,3,3-Tetramethyl-indan [4834-33-7] und 350 ml 95 Gew.-%iger Schwefelsäure tropft man langsam 350 ml 100 Gew.-%ige Salpetersäure so zu, dass die Temperatur + 5° C nicht übersteigt. Anschließend lässt man während 2-3 h langsam auf Raumtemperatur erwärmen und gießt die Reaktionsmischung dann in ein gut gerührtes Gemisch aus 6 kg Eis und 2 kg Wasser. Man stellt durch Zugabe von 40 Gew.-%iger NaOH auf pH = 8-9 ein, extrahiert dreimal mit je 1000 Ethylacetat, wäscht die vereinigten organischen Phasen zweimal mit je 1000 ml Wasser, trocknet über Magnesiumsulfat, entfernt dann das Ethylacetat im Vakuum fast vollständig, bis zur beginnenden Kristallisation, und vervollständigt die Kristallisation durch Zusatz von 500 ml Heptan. Man saugt von den so erhaltenen beigefarbenen Kristallen ab und trocknet diese im Vakuum. Ausbeute: 121.6 g (460 mmol), 92 %; Reinheit: ca. 94 %ig nach ¹H-NMR, Rest ca. 4 % 4,6-Dinitro-1,1,3,3-tetramethyl-indan. Aus der Mutterlauge können ca. 3 % 4,5-Dinitro-1,1,3,3-tetramethyl-indan isoliert werden.

### B: 1,1,3,3-Tetramethyl-indan-5,6-diamin, S1

126.9 g (480 mmol) 5,6-Dinitro-1,1,3,3-tetramethyl-indan, S16b werden bei Raumtemperatur in 1200 ml Ethanol an 10 g Palladium/Kohle bei 3 bar Wasserstoffdruck während 24 h hydriert. Die Reaktionsmischung wird zweimal über ein Celite-Bett filtriert, der nach Entferen des Ethanols erhaltene braune Feststoff wird Kugelrohr-destilliert (T ca. 160 °C, p ca. 10⁻⁴ mbar). Ausbeute: 90.3 g (442 mmol), 92 %; Reinheit: ca. 95 % nach ¹H-NMR.
1,1,3,3-Tetramethyl-indan-5,6-diamin-di-hydrochlorid, S16 x 2HCl kann aus S16 durch lösen in Dichlormethan und einleiten von gasförmiger HCl bis zur Sättigung und anschließendes Entfernen des Dichlormethans erhalten werden.

Analog werden folgende Verbindungen dargestellt:

| **Bsp.** | **Edukt** | **Produkt** | **Variante Ausbeute Stufe A+B** |
|---|---|---|---|
| S2 | 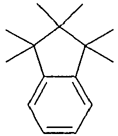 | 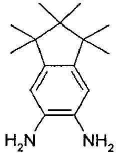 | A 63 % |
| | 91324-94-6 | S2 | B 78 % |
| S3 | 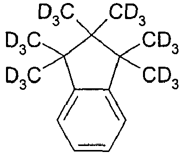 | 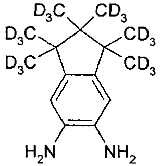 | B 80 % |
| | S1 | S3 | |
| S4 | 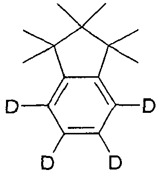 | 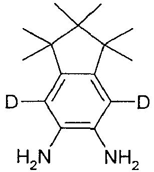 | B 76% |
| | 142076-41-3 | S4 | |
| S5 | 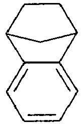 | 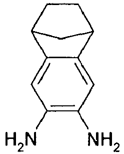 | B 71 % |
| | 4486-29-7 | 124639-03-8 S5 | |

### Beispiel S6: 2-(5,5,7,7-Tetramethyl-1,5,6,7-tetrahydro-indeno-[5,6-d]imidazol-2-yl]phenylamin, S6

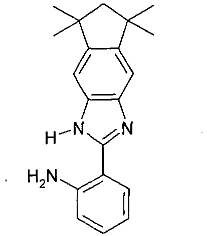

Darstellung analog Pandey, Rampal et al., Tetrahedron Letters, 53(28), 3550, 2012.
Eine Lösung von 20.4 g (100 mmol) 1,1,3,3-Tetramethyl-indan-5,6-diamin, S1 und 13.7 g (100 mmol) 2-Amino-benzoesäure [118-92-3] in 400 ml Methanol wird 30 min. bei Raumtemperatur und dann 6 h unter Rückfluss erhitzt, wobei 200 ml Methanol allmählich abdestilliert werden. Nach langsamem Erkalten rührt man 12 h bei Raumtemperatur nach, saugt von den Kristallen ab, wäscht diese mit wenig Methanol und trocknet im Vakuum. Ausbeute: 25.0 g (82 mmol) 82 %. Reinheit: 97 %ig n. ¹H-NMR.

2-(2-Hydroxyphenyl)benzimidazole werden analog M. Al Messmary et al., Int. Arch. Appl. Science and Tech. 1(1), 84, 2011 nach obiger Vorschrift erhalten, wobei Methanol durch Eisessig ersetzt wird.

Analog werden folgende Verbindungen dargestellt:

| **Bsp.** | **Diamin** | **Carbonsäure** | **Produkt** | **Ausbeute** |
|---|---|---|---|---|
| S7 | 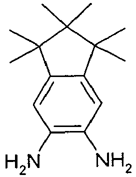 | 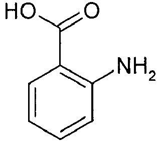 | 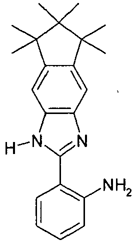 | 79 % |
| | S2 | 118-92-3 | S7 | |
| S8 | 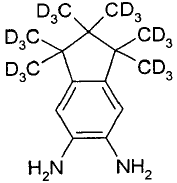 | 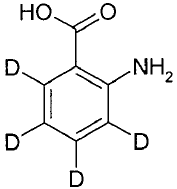 | 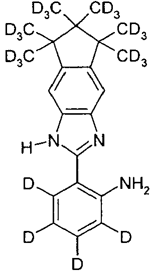 | 77 % |
| | S3 | 60124-83-6 | S8 | |
| S9 | 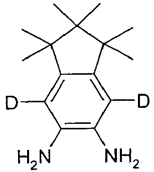 | 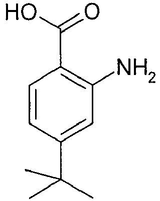 | 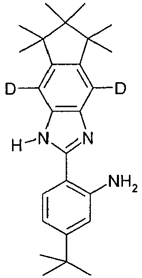 | 80 % |
| | S4 | 728945-64-0 | S9 | |
| S10 | 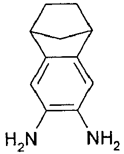 | 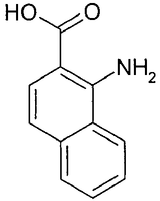 | 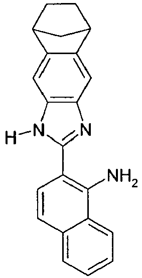 | 73 % |
| | 124639-03-8 S5 | 4919-43-1 | S10 | |
| S11 | 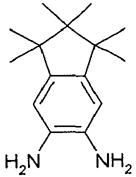 | 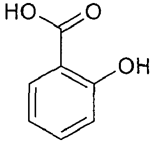 | 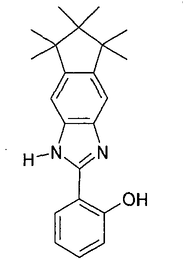 | 57 % |
| | S2 | 69-72-7 | S11 | |
| S12 | 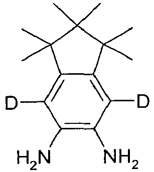 | 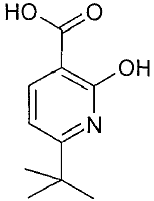 | 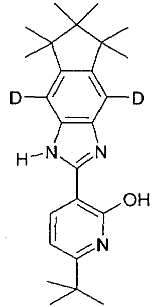 | 54 % |
| | S4 | | S12 | |

### B: Synthese der Liganden L:

### Beispiel L1: 6,6-Dimethyl-5,6-dihydro-benzo[4,5]imidazo-[1,2-c]chinazolin

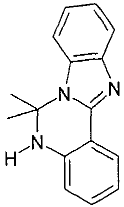

Ein Lösung von 20.9 g (100 mmol) 2-(2-Aminophenyl)benzimidazol [5805-39-0] in 100 ml Aceton wird mit 13.5 ml (110 mmol) 2,2-Dimethoxy-propan und dann mit 6.6 ml (110 mmol) Eisessig versetzt und 16 h bei Raumtemperatur gerührt. Man saugt vom ausgefallenen Feststoff ab, wäscht diesen einmal mit 20 ml Aceton und trocknet im Vakuum. Ausbeute: 19.5 g (78 mmol) 78 %. Reinheit: 99 %ig n. ¹H-NMR.
Die so erhaltenen Liganden werden durch Kugelrohrdestillation oder fraktionierte Sublimation (p ca. 10⁻⁴ - 10⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

Analog werden folgende Verbindungen dargestellt:

| **Bsp.** | **Amin Alkohol** | **Ketal Lösungsmittel** | **Produkt** | **Ausbeute** |
|---|---|---|---|---|
| L2 | 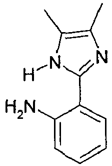 | 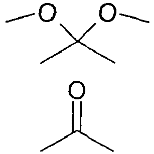 | 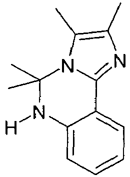 | 34 % |
| | 29528-26-5 | | | |
| L3 | 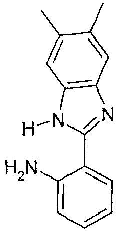 | 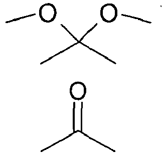 | 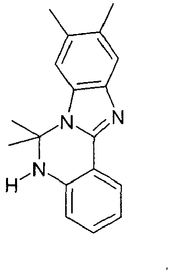 | 75 % |
| | 10173-54-3 | | | |
| L4 | 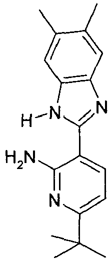 | 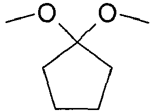 | 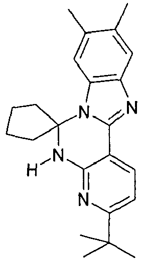 | 68 % |
| | | 931-94-2 | | |
| | 1352336-60-7 | 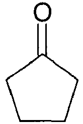 | | |
| | | 120-92-3 | | |
| L5 | 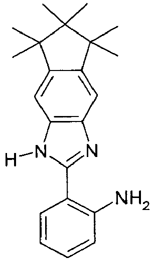 | 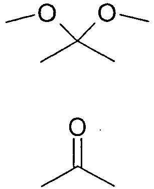 | 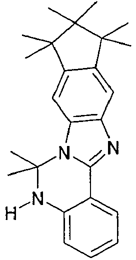 | 69 % |
| | S7 | | | |
| L6 | 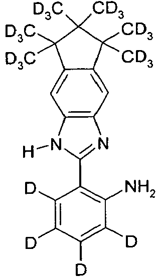 | 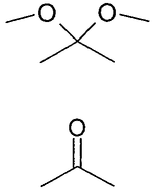 | 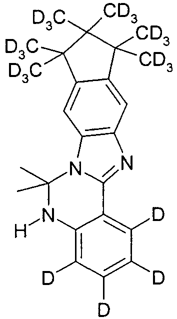 | 73 % |
| | S8 | | | |
| L7 | 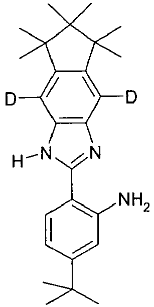 | 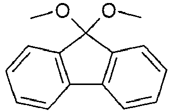 | 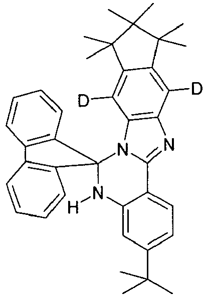 | 46 % |
| | S9 | 116143-54-5 | | |
| | | Lösungsmittel THF | | |
| L8 | 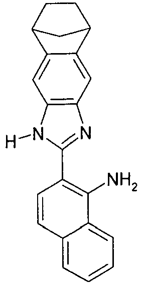 | 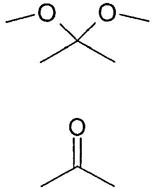 | 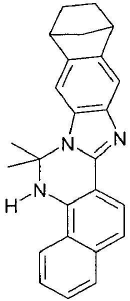 | 64 % |
| | S10 | | | |
| L9 | 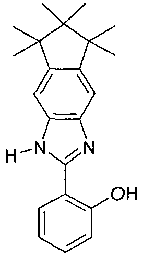 | 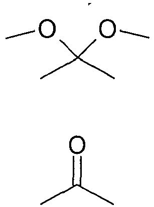 | 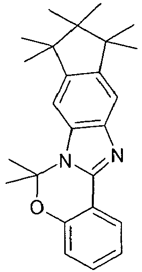 | 55 % |
| | S11 | | | |
| L10 | 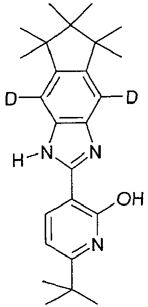 | 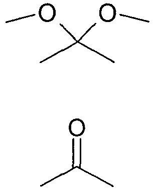 | 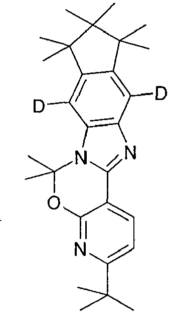 | 49 % |
| | S12 | | | |

### Beispiel L11: 5,6,6-Trimethyl-5,6-dihydro-benzo[4,5]imidazo-[1,2-c]chinazolin

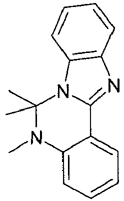

Ein Gemisch aus 24.9 g (100 mmol) 6,6-Dimethyl-5,6-dihydro-benzo[4,5]-imidazo-[1,2-c]chinazolin, L1 und 11.5 g (120 mmol) Natrium-tert-butylat in 300 ml THF wird 30 min. bei 60 °C gerührt. Nach Erkalten auf Raumtemperatur tropft man 7.5 ml (120 mmol) Methyliodid in 50 ml THF zu, rührt dann 4 h bei 60 °C nach, entfernt das THF im Vakuum, nimmt den Rückstand in 500 ml Ethylacetat auf, wäscht die organische Phase zweimal mit je 300 ml Wasser, einmal mit gesättigter Kochsalzlösung, trocknet über Magnesiumsulfat und entfernt dann das Lösungsmittel im Vakuum. Ausbeute: 17.9 g (68 mmol) 68 %. Reinheit: 99 %ig n. ¹H-NMR.

Die so erhaltenen Liganden werden durch Kugelrohrdestillation oder fraktionierte Sublimation (p ca. 10⁻⁴ - 10⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

Analog werden folgende Verbindungen dargestellt:

| **Bsp.** | **Amin** | **Alkylierungsmittel** | **Produkt** | **Ausbeute** |
|---|---|---|---|---|
| L12 | 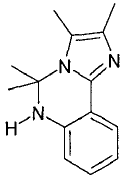 | H₃C-I | 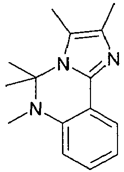 | 73 % |
| | L2 | | | |
| L13 | 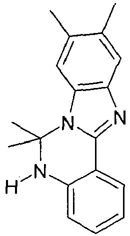 | H₃C-I | 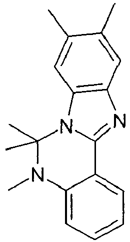 | 70 % |
| | L3 | | | |
| L14 | 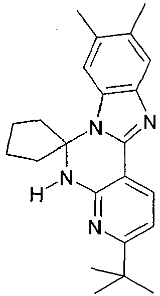 | n-Bu-I | 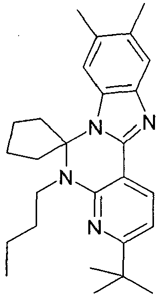 | 48 % |
| | L4 | | | |
| L15 | 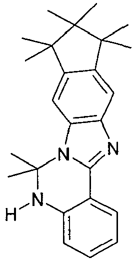 | H₃C-I | 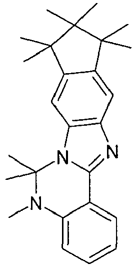 | 67 % |
| | L5 | | | |
| L16 | 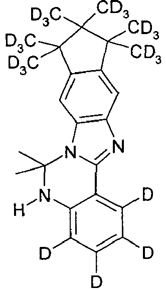 | H₃C-I | 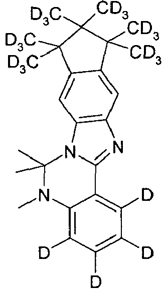 | 70 % |
| | L6 | | | |
| L17 | 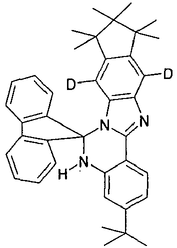 | H₃C-I | 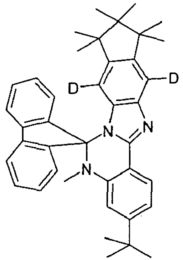 | 36 % |
| | LL7 | | | |

### Beispiel L18: 6,6-Dimethyl-5-phenyl-5,6-dihydro-benzo[4,5]imidazo-[1,2-c]chinazolin

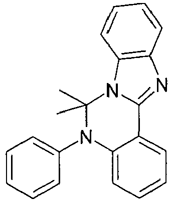

Ein Gemisch aus 24.9 g (100 mmol) 6,6-Dimethyl-5,6-dihydro-benzo[4,5]-imidazo-[1,2-c]chinazolin, L1, 11.2 ml (120 mmol) Fluorbenzol [462-06-6] und 11.5 g (120 mmol) Natrium-tert-butylat in Dimethylacetamid wird 30 h bei 160 °C gerührt. Nach Erkalten auf Raumtemperatur gibt man 500 ml Ethylacetat zu, wäscht die organische Phase fünfmal mit je 300 ml Wasser, einmal mit gesättigter Kochsalzlösung, trocknet über Magnesiumsulfat und entfernt dann das Lösungsmittel im Vakuum. Der ölige Rückstand wird zweimal Kugelrohr-destilliert (p ca. 10⁻⁴ - 10⁻⁵ mbar, T ca. 200 - 220 °C). Ausbeute: 15.3 g (47 mmol) 47 %. Reinheit: 99.5 %ig n. ¹H-NMR.

Analog können folgende Verbindungen dargestellt werden.

| **Bsp.** | **Amin** | **Fluoraromat** | **Produkt** | **Aus-beute** |
|---|---|---|---|---|
| L19 | 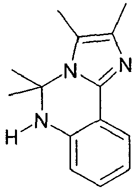 | 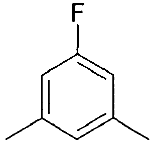 | 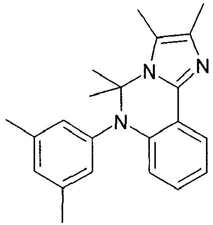 | 43 % |
| | L2 | 461-97-2 | | |
| L20 | 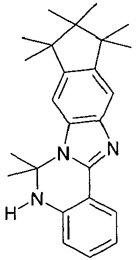 | 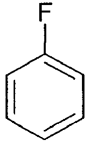 | 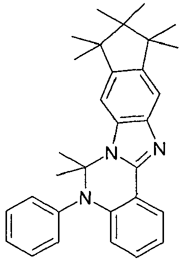 | 45 % |
| | L5 | 462-06-6 | | |

### Beispiel L21: 6,6-Dimethyl-5-oxa-6a,11-diaza-6-sila-benzo[a]fluoren

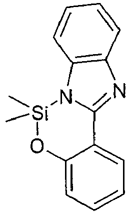

Ein Lösung von 21.0 g (100 mmol) 2-(2-Hydroxyphenyl)benzimidazol [2963-66-8] in einem Gemisch aus 300 ml THF und 30.5 ml (220 mmol) Triethylamin wird tropfenweise mit einem Gemisch aus 12.8 ml (105 mmol) Dichlordimethylsilan [75-78-5] und 50 ml THF versetzt und 16 h bei Raumtemperatur gerührt. Man entfernt das THF im Vakuum, nimmt den Rückstand in 200 ml Cyclohexan auf, saugt vom Triethylammoniumchlorid ab und entfernt das Cyclohexan im Vakuum. Der ölige Rückstand wird zweimal Kugelrohr-destilliert (p ca. 10⁻⁴ - 10⁻⁵ mbar, T ca. 200 - 220 °C). Ausbeute: 13.6 g (51 mmol) 51 %. Reinheit: 99.5 %ig n. ¹H-NMR.

Analog werden folgende Verbindungen dargestellt:

| **Bsp.** | **Imidazol** | **Elektrophil** | **Produkt** | **Ausbeute** |
|---|---|---|---|---|
| L22 | 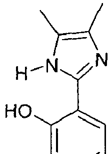 | 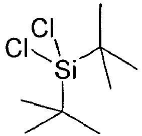 | 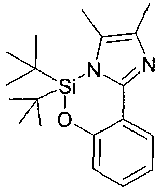 | 54 % |
| | 611-51-8 | 18395-90-9 | | |
| L23 | 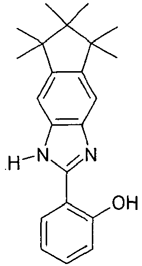 | 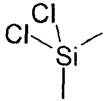 | 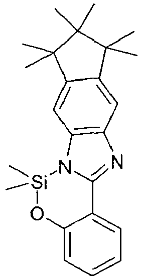 | 47 % |
| | S11 | | | |
| L24 | 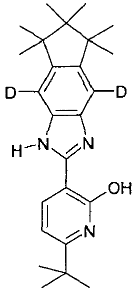 | 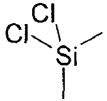 | 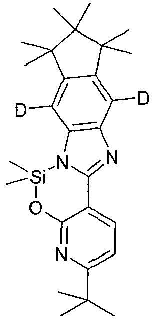 | 50 % |
| | S12 | | | |
| L25 | 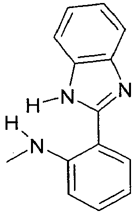 | 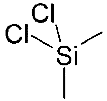 | 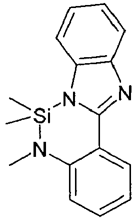 | 39 % |
| | 16367-94-5 | | | |
| L26 | 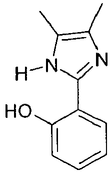 | 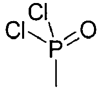 | 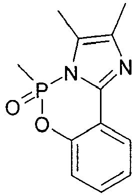 | 44 % |
| | 611-51-8 | 676-97-1 24 h bei 60 °C | | |
| L27 | 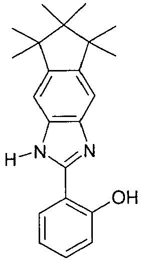 | 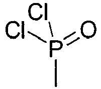 | 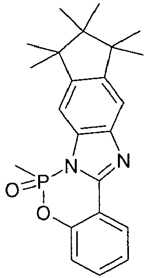 | 40 % |
| | | 676-97-1 24 h bei 60 °C | | |
| | S11 | | | |
| L28 | 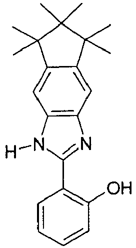 | 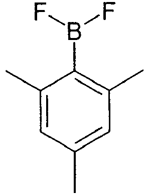 | 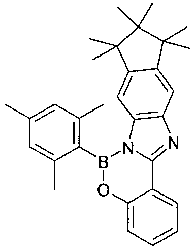 | 30 % |
| | S11 | 119615-26-8 | | |
| L29 | 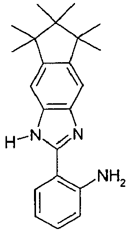 | 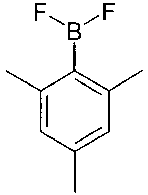 | 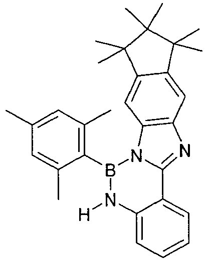 | 27 % |
| | S7 | 119615-26-8 | | |

### Beispiel L30: 5,5,6,6-Tetramethyl-5,6-dihydro-benzo[4,5]-imidazo[2,1a]isochinolin

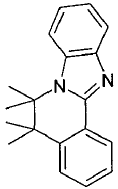

Ein Gemisch aus 19.4 g (100 mmol) 2-Phenylbenzimidazol[716-79-0], 11.0 g (110 mmol) 2,2,3,3-Tetramethyloxiran [5076-20-0], 0.5 ml Bortrifluorid-Etherat und 50 ml Triethylenglykoldimethylether wird im Autoklaven 12 h auf 180 °C erhitzt. Nach Erkalten versetzt man die Reaktionsmischung mit 300 ml Ethylacetat, wäscht fünfmal mit je 200 ml Wasser, einmal mit 200 ml ges. Kochsalzlösung und trocknet die org. Phase über Magnesiumsulfat. Die org. Phase wird vom Lösungsmittel befreit, mit 300 ml Eisessig, 30 ml Essigsäureanhydrid und 10 ml konz. Schwefelsäure versetzt und dann 4 h bei 80 °C gerührt. Man entfernt die Essigsäure im Vakuum, nimmt den Rückstand in 500 ml Dichlormethan auf, alkalisiert unter Eiskühlung vorsichtig mit 10 Gew.-%iger NaOH-Lösung, trennt die org. Phase ab, wäscht diese einmal mit 200 ml Wasser, einmal mit 200 ml ges. Kochsalzlösung und trocknet über Magnesiumsulfat. Der nach Entfernen des Lösungsmittels verbleibende Rückstand wird an Kieselgel (Ethylacetat: MeOH 9:1) chromatographiert und dann zweimal Kugelrohr-destilliert (p ca. 10⁻⁴ - 10⁻⁵ mbar, T ca. 200 - 210 °C). Ausbeute: 8.6 g (31 mmol) 31 %. Reinheit: 99.5 %ig n. ¹H-NMR.

### C: Synthese der Metallkomplexe

### 1) Homoleptische tris-faciale Iridium-Komplexe:

### Variante A: Tris-acetylacetonato-iridium(III) als Iridium-Edukt

Ein Gemisch aus 10 mmol Tris-acetylacetonato-iridium(III) [15635-87-7] und 60 mmol des Liganden L und ein glasummantelter Magnetrührkern werden unter Vakuum (10⁻⁵ mbar) in eine dickwandige 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Um eine Sublimation der Liganden an kältere Stellen der Ampulle zu vermeiden, muss die gesamte Ampulle die angegebene Temperatur besitzen. Alternativ kann die Synthese in einem Rührautoklaven mit Glaseinsatz erfolgen. Nach Erkalten (ACHTUNG: die Ampullen stehen meist unter Druck!) wird die Ampulle geöffnet, der Sinterkuchen wird mit 100 g Glaskugeln (3 mm Durchmessser) in 100 ml eines Suspensionsmittels (das Suspensionsmittel wird so gewählt, dass der Ligand gut, der Metallkomplex jedoch schlecht darin löslich ist, typische Suspensionsmittel sind Methanol, Ethanol, Dichlormethan, Aceton, THF, Ethylacetat, Toluol, etc.) 3 h gerührt und dabei mechanisch aufgeschlossen. Man dekantiert die feine Suspension von den Glaskugeln ab, saugt den Feststoff ab, wäscht mit 50 ml des Suspensionsmittels nach und trocknet diesen im Vakuum. Der trockene Feststoff wird in einem kontinuierlichen Heißextraktor auf einem 3-5 cm hohen Alox-Bett (Alox, basisch Aktivitätsstufe 1) platziert und dann mit einem Extraktionsmittel (Vorlagemenge ca. 500 ml, das Extraktionsmittel wird so gewählt, dass der Komplex darin in der Hitze gut und in der Kälte schlecht löslich ist, besonders geeignete Extraktionsmittel sind Kohlenwasserstoffe wie Toluol, Xylole, Mesitylen, Naphthalin, o-Dichlorbenzol, halogenierte aliphatische Kohlenwasserstoffe sind in der Regel ungeeignet, da sie die Komplexe gegebenenfalls halogenieren oder zersetzen) extrahiert. Nach beendeter Extraktion wird das Extraktionsmittel im Vakuum auf ca. 100 ml eingeengt. Metallkomplexe, die im Extraktionsmittel eine zu gute Löslichkeit aufweisen, werden durch Zutropfen von 200 ml Methanol zur Kristallisation gebracht. Der Feststoff der so erhaltenen Suspensionen wird abgesaugt, einmal mit ca. 50 ml Methanol gewaschen und getrocknet. Nach Trocknen wird die Reinheit des Metall-Komplexes mittels NMR und / oder HPLC bestimmt. Liegt die Reinheit unter 99.5 % wird der Heißextraktionsschritt wiederholt, wobei ab der 2ten Extraktion das Alox-Bett weggelassen wird, ist eine Reinheit von 99.5 - 99.9 % erreicht, wird der Metallkomplex getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 300 - 400 °C, wobei die Sublimation bevorzugt in Form einer fraktionierten Sublimation durchgeführt wird. Werden Liganden der Punktgruppe C1 racemisch eingesetzt, fallen die abgeleiteten fac-Metallkomplexe als Diastereomerenmischung an. Das Enantiomerenpaar Λ,Δ der Punktgruppe C3 weist in der Regel eine deutlich geringere Löslichkeit im Extraktionsmittel auf als das der Punktgruppe C1, das sich folglich in der Mutterlauge anreichert. Eine Trennung der Diasteromeren auf diesem Wege ist häufig möglich. Daneben können die Diastereomeren auch chromatographisch getrennt werden. Werden Liganden der Punktgruppe C1 enantiomerenrein eingesetzt, entsteht das Enantiomerenpaar Λ,Δ der Punktgruppe C3.

### Variante B: Tris-(2,2,6,6-tetramethyl-3,5-heptandionato)iridium(III) als Iridium-Edukt

Durchführung analog zu Variante A, wobei anstelle von 10 mmol Tris-acetylacetonato-iridium(III) [15635-87-7] 10 mmol Tris-(2,2,6,6-tetramethyl-3,5-heptandionato)iridium [99581-86-9] eingesetzt werden. Die Verwendung dieses Edukts ist vorteilhaft, da die Reinheit der erhaltenen Rohprodukte häufig besser ist als bei Variante A. Außerdem ist der Druckaufbau in der Ampulle häufig nicht so ausgeprägt.

| **Bsp.** | **Ligand L** | **Ir-Komplex Diastereomer** | **Variante Reaktionstemp./ Reaktionszeit Suspensionsmittel Extraktionsmittel** | **Ausbeute** |
|---|---|---|---|---|
| Ir(L2)₃ | L2 | 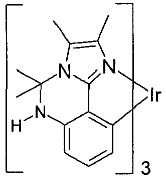 | A 230 °C / 60 h DCM Mesitylen | 24 % |
| | | Ir(L2)₃ | | |
| Ir(L9)₃ | L9 | 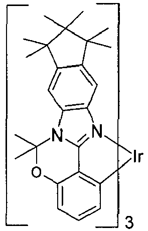 | A 230 °C / 60 h DCM Mesitylen | 21 % |
| | | Ir(L9)₃ | | |
| Ir(L12)₃ | L12 | 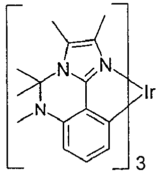 | B 240 °C / 80 h DCM Mesitylen | 25 % |
| | | Ir(L12)₃ | | |
| Ir(L15)₃ | L15 | 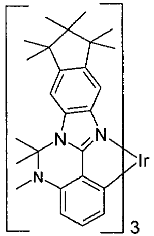 | B 240 °C / 80 h Aceton Mesitylen | 23 % |
| | | Ir(L15)₃ | | |
| Ir(L16)₃ | L16 | 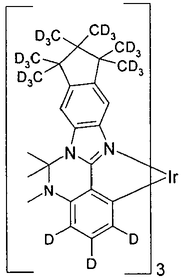 | B 250 °C / 80 h Aceton Mesitylen | 21 % |
| | | Ir(L16)₃ | | |
| Ir(L20)₃ | L20 | 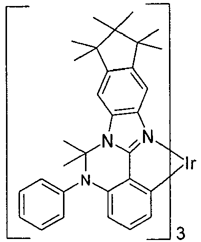 | B 250 °C / 80 h Aceton Mesitylen | 22 % |
| | | Ir(L20)₃ | | |
| Ir(L24)₃ | L24 | 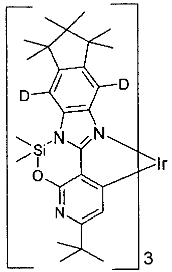 | B 250 °C / 100 h Aceton Mesitylen | 18 % |
| | | Ir(L24)₃ | | |
| Ir(L27)₃ | L27 | 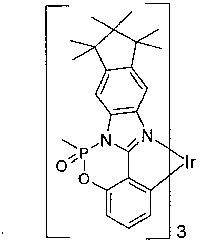 | B 230 °C / 100 h THF Mesitylen | 21 % |
| | | Ir(L27)₃ | | |
| Ir(L28)₃ | L28 | 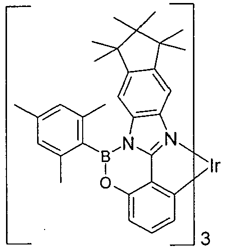 | A 260 °C / 30 h THF Toluol | 15 % |
| Ir(L29)₃ | L29 | 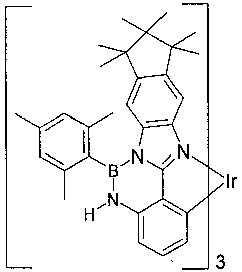 | A 260 °C / 30 h THF Toluol | 13 % |
| Ir(L30)₃ | L30 | 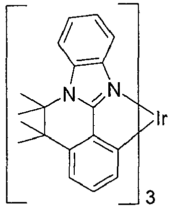 | A 255 °C / 60 h EtOH Toluol | 24 % |

### 2) Heteroleptische Iridium-Komplexe:

### Variante A:

### Schritt 1:

Ein Gemisch aus 10 mmol Natrium-bis-acetylacetonato-dichloro-iridat(III) [770720-50-8] und 24 mmol des Liganden L und ein glasummantelter Magnetrührkern werden unter Vakuum (10⁻⁵ mbar) in eine dickwandige 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Nach Erkalten - ACHTUNG: die Ampullen stehen meist unter Druck! - wird die Ampulle geöffnet, der Sinterkuchen wird mit 100 g Glaskugeln (3 mm Durchmessser) in 100 ml des angegebenen Suspensionsmittels (das Suspensionsmittel wird so gewählt, dass der Ligand gut, das Chloro-Dimer der Formel [Ir(L)₂Cl]₂ jedoch schlecht darin löslich ist, typische Suspensionsmittel sind Dichlormethan, Aceton, Ethylacetat, Toluol, etc.) 3 h gerührt und dabei mechanisch aufgeschlossen. Man dekantiert die feine Suspension von den Glaskugeln ab, saugt den Feststoff (Ir(L)₂Cl]₂, das noch ca. 2 eq NaCl enthält, nachfolgend das rohe Chloro-Dimer genannt, ab und trocknet diesen im Vakuum.

### Schritt 2:

Das so erhaltene rohe Chloro-Dimer der Formel [Ir(L)₂Cl]₂ wird in einem Gemisch aus 75 ml 2-Ethoxyethanol und 25 ml Wasser suspendiert, mit 13 mmol des Co-Liganden CL bzw. der Co-Liganden-Verbindung CL und 15 mmol Natriumcarbonat versetzt. Nach 20 h unter Rückfluss gibt man weitere 75 ml Wasser tropfenweise zu, saugt nach Erkalten vom Feststoff ab, wäscht diesen dreimal mit je 50 ml Wasser und dreimal mit je 50 ml Methanol und trocknet diesen im Vakuum. Der trockene Feststoff wird in einem kontinuierlichen Heißextraktor auf einem 3-5 cm hohen Alox-Bett (Alox, basisch Aktivitätsstufe 1) platziert und dann mit dem angegebenen Extraktionsmittel (Vorlagemenge ca. 500 ml, das Extraktionsmittel wird so gewählt, dass der Komplex darin in der Hitze gut und in der Kälte schlecht löslich ist, besonders geeignete Extraktionsmittel sind Kohlenwasserstoffe wie Toluol, Xylole, Mesitylen, Naphthalin, o-Dichlorbenzol; halogenierte aliphatische Kohlenwasserstoffe sind in der Regel ungeeignet, da sie die Komplexe gegebenenfalls halogenieren oder zersetzen) extrahiert. Nach beendeter Extraktion wird das Extraktionsmittel im Vakuum auf ca. 100 ml eingeengt. Metallkomplexe, die im Extraktionsmittel eine zu gute Löslichkeit aufweisen, werden durch Zutropfen von 200 ml Methanol zur Kristallisation gebracht. Der Feststoff der so erhaltenen Suspensionen wird abgesaugt, einmal mit ca. 50 ml Methanol gewaschen und getrocknet. Nach Trocknen wird die Reinheit des Metall-Komplexes mittels NMR und / oder HPLC bestimmt. Liegt die Reinheit unter 99.5 % wird der Heißextraktionsschritt wiederholt, ist eine Reinheit von 99.5 - 99.9 % erreicht, wird der Metallkomplex getempert oder sublimiert. Neben dem Heißextraktionsverfahren zur Reinigung kann die Reinigung auch chromatographisch an Kieselgel oder Alox erfolgen. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 300 - 400 °C, wobei die Sublimation bevorzugt in Form einer fraktionierten Sublimation durchgeführt wird.

| **Bsp.** | **Ligand L** | **Co-Ligand CL** | **Ir-Komplex Schritt 1: Reaktionstemp. / Reaktionszeit / Suspensionsmittel Schritt 2: Extraktionsmittel** | **Ausbeute** |
|---|---|---|---|---|
| Ir(L10)₂(CL1) | L10 | 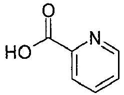 | 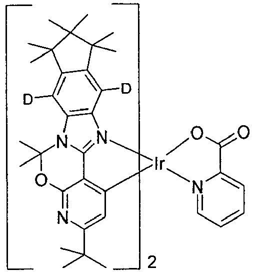 | 27 % |
| | | 98-98-6 CL1 | 240 °C / 80 h Aceton / Mesitylen | |
| Ir(L11)₂(CL2) | L11 | 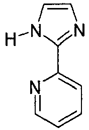 | 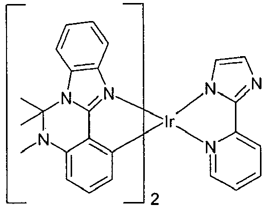 | 24 % |
| | | 18653-75-3 CL2 | 240 °C / 80 h / Aceton / Mesitylen | |
| Ir(L17)₂(CL3) | L17 | 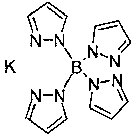 | 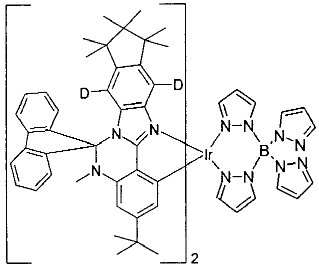 | 20 % |
| | | 14782-58-2 CL3 | 240 °C / 80 h Aceton / Mesitylen | |
| Ir(L18)₂(CL4) | L18 | 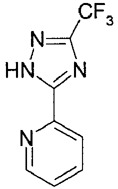 | 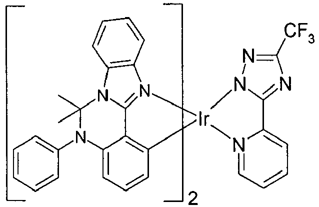 | 23 % |
| | | 219508-27-7 CL4 | 240 °C / 80 h / Aceton / Mesitylen | |

### Variante B:

### Schritt 1:

### Siehe Variante A, Schritt 1.

### Schritt 2:

Das so erhaltene rohe Chloro-Dimer der Formel [Ir(L)₂Cl]₂ wird in 1000 ml Dichlormethan und 150 ml Ethanol suspendiert, die Suspension wird mit 20 mmol Silber(I)trifluormethansulfonat versetzt und 24 h bei Raumtemperatur gerührt. Man saugt vom ausgefallen Feststoff (AgCl) über eine kurzes Celite-Bett ab und engt das Filtrat im Vakuum zur Trockene ein. Der so erhaltene Feststoff wird in 100 ml Ethylenglykol aufgenommen, mit 20 mmol des Co-Liganden CL versetzt und dann 30 h bei 130 °C gerührt. Nach Erkalten saugt man vom Feststoff ab, wäscht diesen zweimal mit je 50 ml Ethanol und trocknet im Vakuum. Heißextraktion und Sublimation wie in Variante A.

| **Bsp.** | **Ligand L** | **Co-Ligand CL** | **Ir-Komplex Schritt 1: Reaktionstemp. / Reaktionszeit / Suspensionsmittel Schritt 2: Extraktionsmittel** | **Ausbeute** |
|---|---|---|---|---|
| Ir(L13)₂(CL5) | L13 | 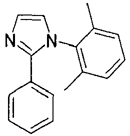 | 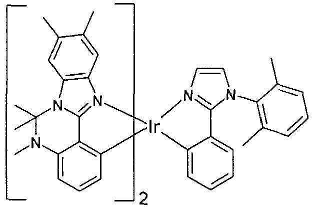 | 26 % |
| | | 914306-48-2 CL5 | 230 °C / 80 h / Aceton / Xylol | |
| Ir(L14)₂(CL5) | L14 | CL5 | 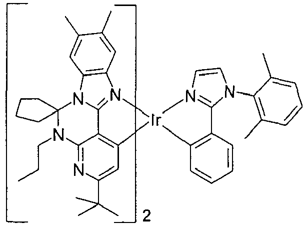 | 29 % |
| | | | 230 °C / 80 h / Aceton / Xylol | |
| Ir(L19)₂(CL6) | L19 | 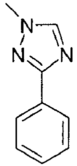 | 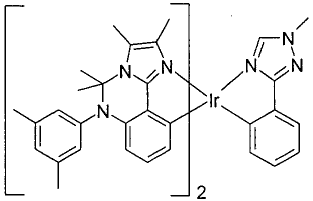 | 24 % |
| | | 39696-58-7 CL6 | 230 °C / 80 h / Aceton / Xylol | |
| Ir(L21)₂(CL6) | L21 | CL6 | 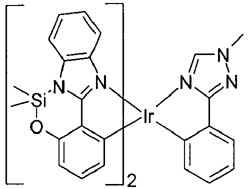 | 24 % |
| | | | 230 °C / 80 h / Aceton / Xylol | |
| Ir(L23)₂(CL6) | L23 | CL6 | 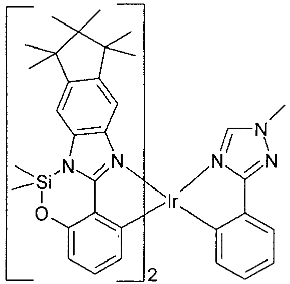 | 22 % |
| | | | 230 °C / 80 h / Aceton / Xylol | |
| Ir(L26)₂(CL6) | L26 | CL6 | 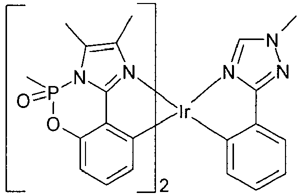 | 17 % |
| | | | 220 °C / 80 h / Aceton / Xylol | |

### Heteroleptische Platin-Komplexe:

Ein Gemisch aus 10 mmol Platin(II)chlorid und 12 mmol des Liganden L und ein glasummantelter Magnetrührkern werden unter Vakuum (10⁻⁵ mbar) in eine dickwandige 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Nach Erkalten - ACHTUNG: die Ampullen stehen meist unter Druck! - wird die Ampulle geöffnet, der Sinterkuchen wird mit 100 g Glaskugeln (3 mm Durchmessser) in 100 ml des angegebenen Suspensionsmittels (das Suspensionsmittel wird so gewählt, dass der Ligand gut, das Chloro-Dimer der Formel [Pt(L)Cl]₂ jedoch schlecht darin löslich ist, typische Suspensionsmittel sind Dichlormethan, Aceton, THF, Ethylacetat, Toluol, etc.) 3 h gerührt und dabei mechanisch aufgeschlossen. Man dekantiert die feine Suspension von den Glaskugeln ab, saugt den Feststoff ab und trocknet diesen im Vakuum. Das so erhaltene rohe Chloro-Dimer der Formel [Pt(L)Cl]₂ wird in einem Gemisch aus 60 ml 2-Ethoxyethanol und 20 ml Wasser suspendiert und mit 20 mmol des Co-Liganden CL bzw. der Co-Liganden-Verbindung CL und 20 mmol Natriumcarbonat versetzt. Nach 20 h unter Rückfluss gibt man weitere 100 ml Wasser tropfenweise zu, saugt nach Erkalten vom Feststoff ab, wäscht diesen dreimal mit je 50 ml Wasser und dreimal mit je 50 ml Methanol und trocknet diesen im Vakuum. Der so erhaltene Feststoff wird in einem Heißextraktor auf einem 3-5 cm hohen Celite-Bett platziert und dann mit dem angegebenen Extraktionsmittel (Vorlagemenge ca. 500 ml) extrahiert. Nach beendeter Extraktion wird das Extraktionsmittel im Vakuum auf ca. 100 ml eingeengt. Metallkomplexe, die im Extraktionmittel eine zu gute Löslichkeit aufweisen, werden durch Zutropfen von 200 ml Methanol zur Kristallisation gebracht. Der Feststoff der so erhaltenen Suspensionen wird abgesaugt, einmal mit ca. 50 ml Methanol gewaschen und getrocknet. Nach Trocknen wird die Reinheit des Metall-Komplexes mittels NMR und / oder HPLC bestimmt. Liegt die Reinheit unter 99.5 % wird der Heißextraktionsschritt wiederholt, ist eine Reinheit von 99.5 - 99.9 % erreicht, wird der Metallkomplex getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 250 - 350 °C, wobei die Sublimation bevorzugt in Form einer fraktionierten Sublimation durchgeführt wird.

| **Bsp.** | **Ligand L** | **Co-Ligand CL** | **Pt-Komplex** | **Ausbeute** |
|---|---|---|---|---|
| Pt(L22)(CL7) | L22 | CL7 | 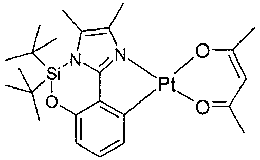 | 21 % |
| | | 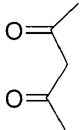 | | |
| | | 123-54-6 | 230 °C / 60 h / Aceton / Xylol | |
| Pt(L25)(CL2) | L25 | CL8 | 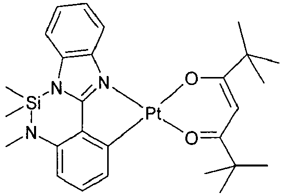 | 23 % |
| | | 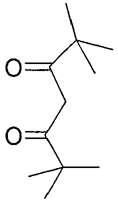 | | |
| | | 1118-71-4 | 220 °C / 60 h / THF / Xylol | |

### Herstellung der OLEDs

### 1) Vakuum-prozessierte Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Glasplättchen, mit strukturiertem ITO (Indium Zinn Oxid) bilden die Substrate, auf welche die OLEDs aufgebracht werden. Die OLEDs haben prinzipiell folgenden Schichtaufbau: Substrat / Lochtransportschicht 1 (HTL1) bestehend aus HTM dotiert mit 3 % NDP-9 (kommerziell erhältlich von der Fa. Novaled), 20 nm / Lochtransportschicht 2 (HTL2) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet.

Zunächst werden vakuumprozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie M3:M2:Ir(L1)₃ (55%:35%:10%) bedeutet hierbei, dass das Material M3 in einem Volumenanteil von 55%, M2 in einem Anteil von 35% und Ir(L1)₃ in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 6 gezeigt.

Die OLEDs werden standardmäßig charakterisiert. Hierfür werden die Elektrolumineszenzspektren, die Stromeffizienz (gemessen in cd/A) und die Spannung (gemessen bei 1000 cd/m² in V) bestimmt aus Strom-Spannungs-Helligkeits-Kennlinien (IUL-Kennlinien). Für ausgewählte Versuche wird die Lebensdauer bestimmt. Als Lebensdauer wird die Zeit definiert, nach der die Leuchtdichte von einer bestimmten Startleuchtdichte aus auf einen gewissen Anteil abgesunken ist. Die Angabe LD50 bedeutet, dass es sich bei der genannten Lebensdauer um die Zeit handelt, bei der die Leuchtdichte auf 50% der Startleuchtdichte abgefallen ist, also von z.B. 1000 cd/m² auf 500 cd/m². Je nach Emissionsfarbe wurden unterschiedliche Starthelligkeiten gewählt. Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von 1000 cd/m² eine übliche Angabe.

### Verwendung von erfindungsgemäßen Verbindungen als Emittermaterialien in phosphoreszierenden OLEDs

Die erfindungsgemäßen Verbindungen lassen sich unter anderem als phosphoreszierende Emittermaterialien in der Emissionsschicht in OLEDs einsetzen. Die Ergebnisse der OLEDs sind in Tabelle 2 zusammengefasst.

**Tabelle 1: Aufbau der OLED**

| **Bsp.** | **HTL2 Dicke** | **EBL Dicke** | **EML Dicke** | **HBL Dicke** | **ETL Dicke** |
|---|---|---|---|---|---|
| D-Ir(L2)₃ | HTM 180 nm | EBM 20 nm | M1:M4:Ir(L2)₃ (65%:30%:5%) 25 nm | HBM 10 nm | ETM1:ETM2 (50%:50%) 20 nm |
| D-Ir(L9)₃ | HTM 180 nm | EBM 20 nm | M1:M3:Ir(L9)₃ (65%:30%:5%) 25 nm | HBM 10 nm | ETM1:ETM2 (50%:50%) 20 nm |
| D-Ir(L12)₃ | HTM 180 nm | EBM 20 nm | M1:M4:Ir(L12)₃ (65%:30%:5%) 25 nm | HBM 10 nm | ETM1:ETM2 (50%:50%) 20 nm |
| D-Ir(L15)₃ | HTM 180 nm | EBM 20 nm | M1:M4:Ir(L15)₃ (65%:30%:5%) 25 nm | HBM 10 nm | ETM1:ETM2 (50%:50%) 20 nm |
| D-Ir(L16)₃ | HTM 180 nm | EBM 20 nm | M1:M3:Ir(L16)₃ (55%:40%:5%) 25 nm | HBM 10 nm | ETM1:ETM2 (50%:50%) 20 nm |
| D-Ir(L24)₃ | HTM 180 nm | EBM 20 nm | M1:M4:Ir(L24)₃ (65%:30%:5%) 25 nm | HBM 10 nm | ETM1:ETM2 (50%:50%) 20 nm |
| D-Ir(L27)₃ | HTM 180 nm | EBM 20 nm | M1:M4:Ir(L27)₃ (65%:30%:5%) 25 nm | HBM 10 nm | ETM1:ETM2 (50%:50%) 20 nm |
| D-Ir(L10)₂(CL1) | HTM 180 nm | EBM 20 nm | M1:M3:Ir(L10)₂(CL1) (65%:30%:5%) 25 nm | HBM 10 nm | ETM1:ETM2 (50%:50%) 20 nm |
| D-Ir(L11)₂(CL2) | HTM 180 nm | EBM 20 nm | M1:M4:Ir(L11)₂(CL2) (65%:30%:5%) 25 nm | HBM 10 nm | ETM1:ETM2 (50%:50%) 20 nm |
| D-Ir(L18)₂(CL4) | HTM 180 nm | EBM 20 nm | M1:M4:Ir(L18)₂(CL4) (75%:20%:5%) 25 nm | HBM 10 nm | ETM1:ETM2 (50%:50%) 20 nm |
| D-Ir(L13)₂(CL5) | HTM 180 nm | EBM 20 nm | M1:M4:Ir(L230)₃ (65%:30%:5%) 25 nm | HBM 10 nm | ETM 1: ETM2 (50%:50%) 20 nm |
| D-Ir(L19)₂(CL6) | HTM 180 nm | EBM 20 nm | M1:M4:Ir(L19)₂(CL6) (65%:30%:5%) 25 nm | HBM 10 nm | ETM1:ETM2 (50%:50%) 20 nm |
| D-Ir(L21)₂(CL6) | HTM 180 nm | EBM 20 nm | M1:M4:Ir(L21)₂(CL6) (65%:30%:5%) 25 nm | HBM 10 nm | ETM1:ETM2 (50%:50%) 20 nm |
| D-Ir(L23)₂(CL6) | HTM 180 nm | EBM 20 nm | M1:M4:Ir(L23)₂(CL6) (65%:30%:5%) 25 nm | HBM 10 nm | ETM 1: ETM2 (50%:50%) 20 nm |
| D-Ir(L26)₂(CL6) | HTM 180 nm | EBM 20 nm | M1:M4:Ir(L26)₂(CL6) (75%:20%:5%) 25 nm | HBM 10 nm | ETM1:ETM2 (50%:50%) 20 nm |
| D-Pt(L22)(CL7) | HTM 180 nm | EBM 20 nm | M1:M4:Pt(L22)(CL7) (75%:20%:5%) 25 nm | HBM 10 nm | ETM1:ETM2 (50%:50%) 20 nm |
| D-Pt(L25)(CL2) | HTM 180 nm | EBM 20 nm | M1:M4:Pt(L25)(CL2) (75%:20%:5%) 25 nm | HBM 10 nm | ETM1:ETM2 (50%:50%) 20 nm |

**Tabelle 2: Ergebnisse der Vakuum-prozessierten OLEDs**

| **Bsp.** | **EQE (%) 1000 cd/m²** | **Spannung(V) 1000 cd/m²** | **CIE x/y 1000 cd/m²** | **LD50 (h) 1000 cd/m²** |
|---|---|---|---|---|
| D-Ir(L2)₃ | 9.4 | 4.6 | 0.16/0.24 | --- |
| D-Ir(L9)₃ | 14.3 | 4.1 | 0.15/0.36 | 600 |
| D-Ir(L12)₃ | 9.9 | 4.5 | 0.16/0.24 | 250 |
| D-Ir(L15)₃ | 16.4 | 4.3 | 0.15/0.38 | 700 |
| D-Ir(L16)₃ | 16.0 | 4.2 | 0.15/0.38 | --- |
| D-Ir(L24)₃ | 15.5 | 4.1 | 0.15/0.30 | --- |
| D-Ir(L27)₃ | 9.7 | 4.3 | 0.15/0.35 | --- |
| D-Ir(L10)₂(CL1) | 15.5 | 4.1 | 0.15/0.27 | --- |
| D-Ir(L11)₂(CL2) | 14.1 | 4.2 | 0.15/0.34 | 500 |
| D-Ir(L18)₂(CL4) | 9.9 | 4.6 | 0.17/0.31 | --- |
| D-Ir(L13)₂(CL5) | 13.8 | 4.3 | 0.15/0.33 | --- |
| D-Ir(L19)₂(CL6) | 12.9 | 4.4 | 0.16/0.26 | --- |
| D-Ir(L21)₂(CL6) | 10.5 | 4.3 | 0.16/0.34 | --- |
| D-Ir(L23)₂(CL6) | 15.5 | 4.3 | 0.16/0.35 | 600 |
| D-Ir(L26)₂(CL6) | 4.4 | 4.9 | 0.15/0.22 | --- |
| D-Pt(L22)(CL7) | 6.7 | 4.8 | 0.15/0.25 | --- |
| D-Pt(L25)(CL2) | 8.7 | 4.4 | 0.15/0.35 | --- |

### 2) Lösungs-prozessierte Devices:

### A: Aus löslichen Funktionsmaterialien

Die erfindungsgemäßen Komplexe können auch aus Lösung verarbeitet werden und führen dort zu prozesstechnisch wesentlich einfacheren OLEDs, im Vergleich zu den vakuumprozessierten OLEDs, mit dennoch guten Eigenschaften. Die Herstellung solcher Bauteile lehnt sich an die Herstellung polymerer Leuchtdioden (PLEDs) an, die in der Literatur bereits vielfach beschrieben ist (z.B. in der WO 2004/037887). Der Aufbau setzt sich aus Substrat / ITO / PEDOT (80 nm) / Interlayer (80 nm) / Emissionsschicht (80 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum als Pufferschicht eine 80 nm Schicht PEDOT (PEDOT ist ein Polythiophen-Derivat (Baytron P VAI 4083sp.) von H. C. Starck, Goslar, das als wässrige Dispersion geliefert wird) durch Spin-Coating aufgebracht. Die benötigte Spinrate hängt vom Verdünnungsgrad und der spezifischen Spin-Coater-Geometrie ab (typisch für 80 nm: 4500 rpm). Um Restwasser aus der Schicht zu entfernen, werden die Substrate für 10 Minuten bei 180 °C auf einer Heizplatte ausgeheizt. Die verwendete Interlayer dient der Lochinjektion, in diesem Fall wird HIL-012 von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Emitter zusammen mit den Matrixmaterialien in Toluol gelöst. Der typische Feststoffgehalt solcher Lösungen liegt zwischen 16 und 25 g/L, wenn, wie hier, die für eine Device typische Schichtdicke von 80 nm mittels Spincoating erzielt werden soll. Die lösungsprozessierten Devices enthalten eine Emissionsschicht aus (Polystyrol):M5:M6:Ir(L)₃ (25%:25%:40%:10%). Die Emissionsschicht wird in einer Inertgasatmosphäre, im vorliegenden Fall Argon, aufgeschleudert und 30 min bei 130 °C ausgeheizt. Zuletzt wird eine Kathode aus Barium (5 nm) und dann Aluminium (100 nm) (hochreine Metalle von Aldrich, besonders Barium 99.99 % (Best-Nr. 474711); Aufdampfanlagen von Lesker o.a., typischer Aufdampfdruck 5 x 10⁻⁶ mbar) aufgedampft. Optional kann zunächst eine Lockblockierschicht und dann eine Eletronentransportschicht und dann erst die Kathode (z.B. Al oder LiF/Al) im Vakuum aufgedampft werden. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen.

**Tabelle 3: Ergebnisse mit aus Lösung prozessierten Materialien**

| **Bsp.** | **Komplex** | **EQE (%) 1000 cd/m²** | **Spannung(V) 1000 cd/m²** | **CIE x/y 1000 cd/m²** |
|---|---|---|---|---|
| D-Ir(L20)₃ | Ir(L20)₃ | 12.6 | 4.7 | 0.16/0.36 |
| D-Ir(L28)₃ | Ir(L28)₃ | 9.6 | 4.5 | 0.15./0,32 |
| D-Ir(L30)₃ | Ir(L30)₃ | 13.8 | 4.8 | 0.16/0.33 |
| D-Ir(L17)₂(CL3) | Ir(L17)₂(CL3) | 8.7 | 5.0 | 0.17/0.35 |
| D-Ir(L14)₂(CL5) | Ir(L14)₂(CL5) | 9.3 | 4.9 | 0.16/0.27 |

### 3) Weiß emittierende OLEDs

Gemäß den allgemeinen Verfahren aus 1) wird eine weiß emittierende OLED mit folgendem Schichtaufbau hergestellt:

**Tabelle 4: Aufbau der weißen OLEDs**

| **Bsp.** | **HTL2** | **EML Rot** | **EML Blau** | **EML Grün** | **HBL** | **ETL** |
|---|---|---|---|---|---|---|
| | **Dicke** | **Dicke** | **Dicke** | **Dicke** | **Dicke** | **Dicke** |
| D-W1 | HTM | EBM:Ir-R (97%:3%) | M1:M3:Ir(L9)₃ (45%:50%:5%) | M3:Ir-G (90%:10%) | M3 | ETM1:ETM2 (50%:50%) |
| | 230 nm | 9 nm | 8 nm | 7 nm | 10 nm | 30 nm |

**Tabelle 5: Deviceergebnisse**

| **Bsp.** | **EQE (%)** | **Spannung (V) 1000 cd/m²** | **CIE x/y 1000 cd/m²** | **LD50 (h)** |
|---|---|---|---|---|
| | **1000 cd/m²** | | **CRI** | **1000 cd/m²** |
| D-W1 | 13.0 | 6.6 | 0.45/0.43 | 1500 |
| | | | 80 | |

**Tabelle 6: Strukturformeln der verwendeten Materialien**

| | |
|---|---|
| 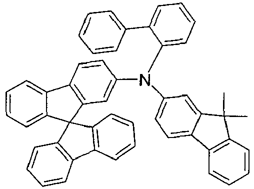 | 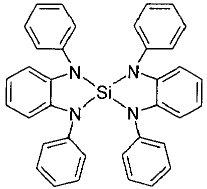 |
| HTM | EBM |
| 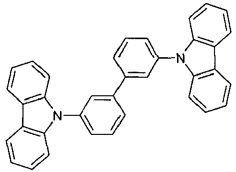 | 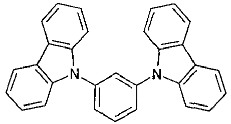 |
| M1 | M2 |
| 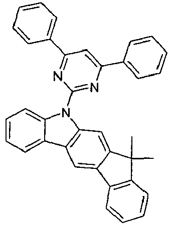 | 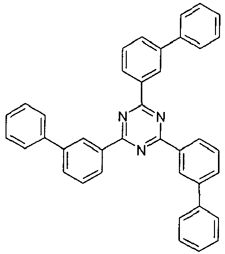 |
| M3 | M4 = HBM |
| 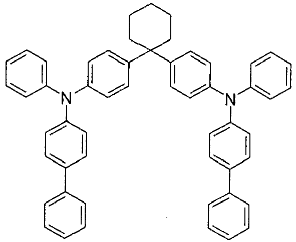 | 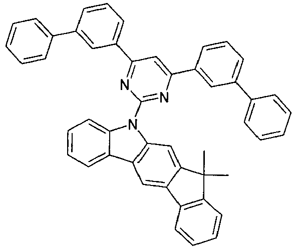 |
| M5 | M6 |
| 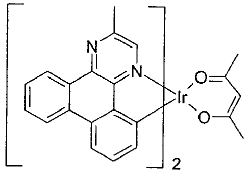 | 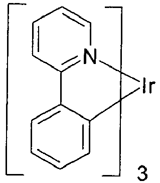 |
| Ir-R | Ir-G |
| 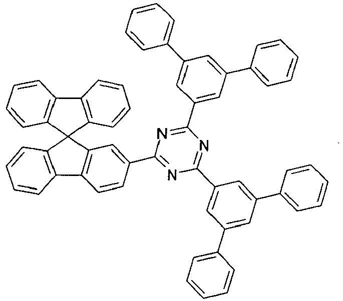 | 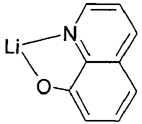 |
| ETM1 | ETM2 |

## Patentansprüche

1. Verbindung gemäß Formel (1),
M(L)ₙ(L')ₘ Formel (1)
welche eine Teilstruktur M(L)n der Formel (2) oder Formel (3) enthält: 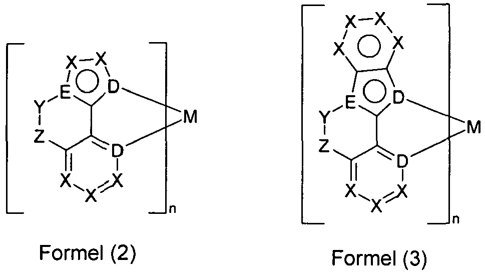 wobei für die verwendeten Symbole und Indizes gilt:
M ist ein Übergangsmetall;
X ist bei jedem Auftreten gleich oder verschieden ausgewählt aus der Gruppe bestehend aus CR und N;
Y ist bei jedem Auftreten gleich oder verschieden ausgewählt aus der Gruppe bestehend aus C(R¹)₂, Si(R¹)₂, PR¹, P(=O)R¹ oder BR¹;
Z ist bei jedem Auftreten gleich oder verschieden ausgewählt aus der Gruppe bestehend aus NR¹, O oder C(R¹)₂;
D ist bei jedem Auftreten gleich oder verschieden C oder N, mit der Maßgabe, dass mindestens ein D für N steht;
E ist bei jedem Auftreten gleich oder verschieden C oder N, mit der Maßgabe, dass mindestens eine der Gruppen E oder D in dem Fünfring für N steht;
R, R¹ ist bei jedem Auftreten gleich oder verschieden H, D, F, Cl, Br, I, N(R²)₂, CN, NO₂, OH, COOH, C(=O)N(R²)₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R² substituiert sein kann, wobei eine oder mehrere nicht benachbarte CH₂-Gruppen durch R²C=CR², C=C, Si(R²)₂, C=O, NR², O, S oder CONR² ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei benachbarte Reste R bzw. zwei benachbarte Reste R¹ auch miteinander ein mono- oder polycyclisches, aliphatisches, aromatisches oder heteroaromatisches Ringsystem bilden; weiterhin können R und R¹ auch miteinander ein mono- oder polycyclisches, aliphatisches oder heteroaromatisches Ringsystem bilden;
R² ist bei jedem Auftreten gleich oder verschieden H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R³ substituiert sein kann, wobei eine oder mehrere nicht benachbarte CH₂-Gruppen durch R³C=CR³, C≡C, Si(R³)₂, C=O, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 40 aromatischen Ringatomen, die durch einen oder mehrere Reste R³ substituiert sein kann, oder eine Aralkyl- oder Heteroaralkylgruppe mit 5 bis 40 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann; dabei können zwei oder mehrere benachbarte Reste R² miteinander oder R² mit R bzw. mit R¹ ein mono- oder polycyclisches, aliphatisches, aromatisches oder heteroaromatisches Ringsystem bilden;
R³ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R³ auch miteinander ein mono- oder polycyclisches, aliphatisches Ringsystem bilden;
L' ist gleich oder verschieden bei jedem Auftreten ein beliebiger Coligand;
n ist 1, 2 oder 3;
m ist 0, 1, 2, 3 oder 4;
dabei können auch mehrere Liganden L miteinander oder L mit L' über eine Einfachbindung oder eine bivalente oder trivalente Brücke verknüpft sein und so ein tridentates, tetradentates, pentadentates oder hexadentates Ligandensystem aufspannen, wobei in diesem Fall L' kein separater Coligand, sondern eine koordinierende Gruppe ist;
weiterhin kann auch ein Substituent R zusätzlich an das Metall koordinieren;
dabei sind die folgenden Verbindungen von der Erfindung ausgenommen:

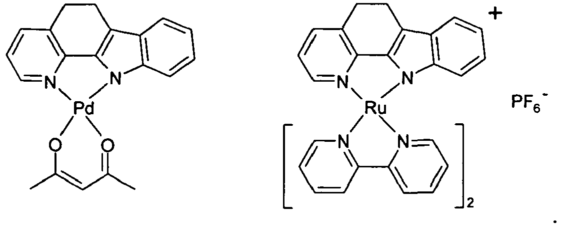

2. Verbindung nach Anspruch 1, **dadurch gekennzeichnet, dass** M ausgewählt ist aus der Gruppe bestehend aus Chrom, Molybdän, Wolfram, Rhenium, Ruthenium, Osmium, Rhodium, Iridium, Nickel, Palladium, Platin, Kupfer, Silber und Gold, insbesondere Molybdän, Wolfram, Rhenium, Ruthenium, Osmium, Iridium, Kupfer, Platin und Gold.

3. Verbindung nach Anspruch 1 oder 2, **dadurch gekennzeichnet, dass** die Teilstrukturen der Formel (2) ausgewählt sind aus den Teilstrukturen der Formeln (2a), (2b) und (2c) und die Teilstrukturen der Formel (3) ausgewählt sind aus den Teilstrukturen der Formeln (3a), (3b) und (3c), 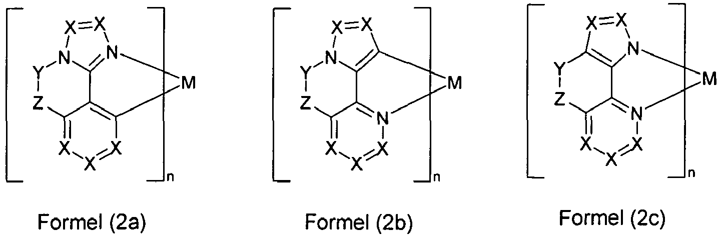 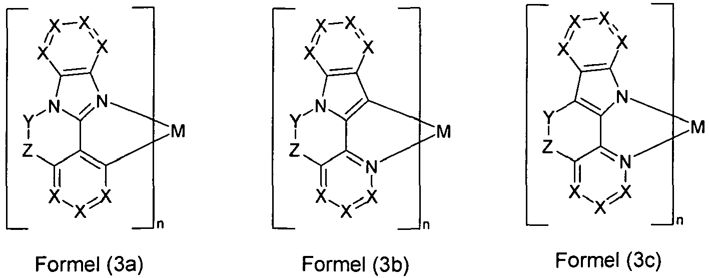 wobei die verwendeten Symbole und Indizes die in Anspruch 1 genannten Bedeutungen aufweisen.

4. Verbindung nach einem oder mehreren der Ansprüche 1 bis 3, **dadurch gekennzeichnet, dass** die Gruppe -Y-Z- gleich oder verschieden bei jedem Auftreten für -C(R¹)₂-NR¹-, -C(R¹)₂-O-, -Si(R¹)₂-NR¹-, -Si(R¹)₂-O- oder -C(R¹)₂-C(R¹)₂- steht.

5. Verbindung nach einem oder mehreren der Ansprüche 1 bis 4, **dadurch gekennzeichnet, dass** R¹ gleich oder verschieden bei jedem Auftreten ausgewählt ist aus der Gruppe bestehend aus F, CN, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R² substituiert sein kann, wobei eine oder mehrere nicht benachbarte CH₂-Gruppen durch R²C=CR² ersetzt sein können und wobei ein oder mehrere H-Atome durch F ersetzt sein können, oder einem aromatischen oder heteroaromatischen Ringsystem mit 5 bis 30 aromatischen Ringatomen, das jeweils durch einen oder mehrere Reste R² substituiert sein kann, oder einer Aryloxy- oder Heteroaryloxygruppe mit 5 bis 30 aromatischen Ringatomen, die durch einen oder mehrere Reste R² substituiert sein kann, oder einer Aralkyl- oder Heteroaralkylgruppe mit 5 bis 40 aromatischen Ringatomen, die durch einen oder mehrere Reste R² substituiert sein kann, oder einer Diarylaminogruppe, Diheteroarylaminogruppe oder Arylheteroarylaminogruppe mit 10 bis 30 aromatischen Ringatomen, welche durch einen oder mehrere Reste R² substituiert sein kann; dabei können zwei benachbarte Reste R¹ auch miteinander ein mono- oder polycyclisches, aliphatisches, aromatisches oder heteroaromatisches Ringsystem bilden; weiterhin können R und R¹ auch miteinander ein mono- oder polycyclisches, aliphatisches oder heteroaromatisches Ringsystem bilden.

6. Verbindung nach einem oder mehreren der Ansprüche 1 bis 5, **dadurch gekennzeichnet, dass** im Liganden L keine, eine oder zwei Gruppen X für N stehen.

7. Verbindung nach einem oder mehreren der Ansprüche 1 bis 6, **dadurch gekennzeichnet, dass** die Teilstrukturen der Formel (2) ausgewählt sind aus den Teilstrukturen der Formeln (2-A) bis (2-G) und dass die Teilstrukturen der Formel (3) ausgewählt sind aus den Teilstrukturen der Formeln (3-A) bis (3-H), 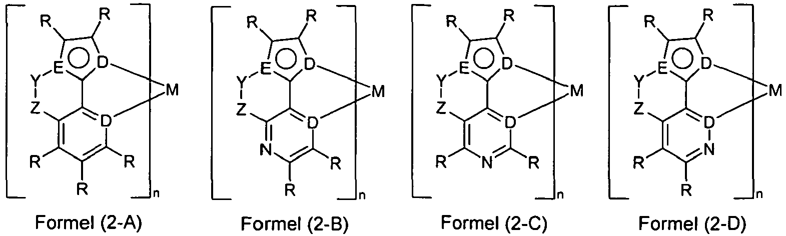 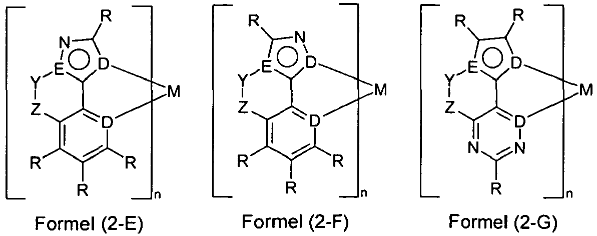 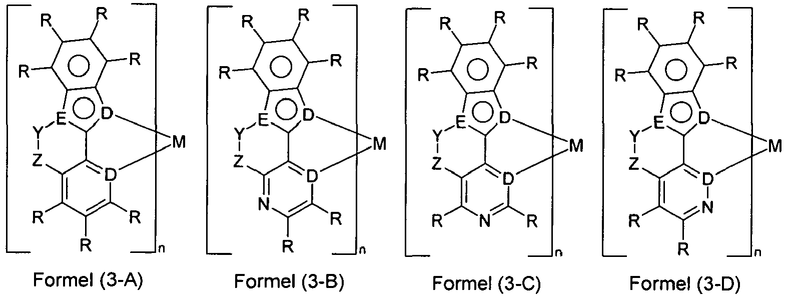 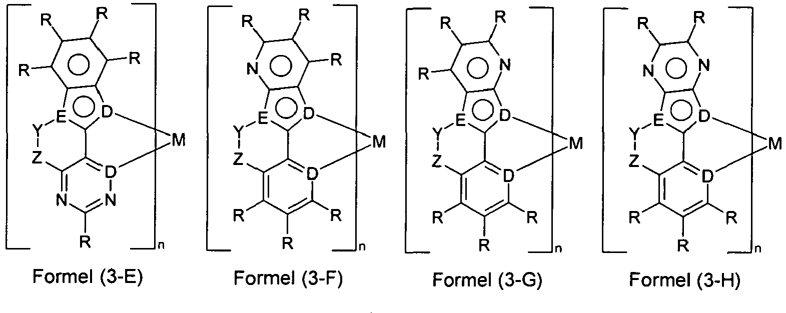 wobei die verwendeten Symbole und Indizes die in Anspruch 1 genannten Bedeutungen aufweisen.

8. Verbindung nach einem oder mehreren der Ansprüche 1 bis 7, **dadurch gekennzeichnet, dass** die Gruppe E für N steht und entweder die Gruppe D in dem Fünfring oder die Gruppe D in dem Sechsring für N steht, während die andere Gruppe D für C steht, oder dass die Gruppe E für C steht und beide Gruppen D für N stehen.

9. Verbindung nach einem oder mehreren der Ansprüche 1 bis 8, **dadurch gekennzeichnet, dass**, wenn eine oder mehrere Gruppen X für Stickstoff stehen, benachbart zu diesem Stickstoffatom eine Gruppe R als Substituent gebunden ist, welche ungleich Wasserstoff oder Deuterium ist, bevorzugt eine Gruppe, ausgewählt aus CF₃, OCF₃, Alkyl- oder Alkoxygruppen mit 1 bis 10 C-Atomen, insbesondere verzweigten oder cyclischen Alkyl- oder Alkoxygruppen mit 3 bis 10 C-Atomen, aromatischen bzw. heteroaromatischen Ringsystemen oder Aralkyl- bzw. Heteroaralkylgruppen, wobei diese Gruppen jeweils optional durch einen oder mehrere Reste R² substituiert sein können.

10. Verbindung nach einem oder mehreren der Ansprüche 1 bis 9, **dadurch gekennzeichnet, dass** zwei benachbarte Gruppen X für CR stehen und die jeweiligen Reste R zusammen mit den C-Atomen einen Ring der Formel (4) oder Formel (5) aufspannen, 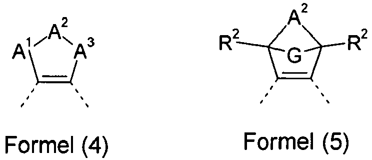 wobei R² und R³ die in Anspruch 1 genannten Bedeutungen aufweisen, die gestrichelten Bindungen die Verknüpfung der beiden Kohlenstoffatome im Liganden andeuten und weiterhin gilt:
A¹, A³ ist gleich oder verschieden bei jedem Auftreten C(R⁴)₂, O, S, NR⁴ oder C(=O);
A² ist C(R²)₂, O, S, NR⁴ oder C(=O);
G ist eine Alkylengruppe mit 1, 2 oder 3 C-Atomen, welche mit einem oder mehreren Resten R³ substituiert sein kann, -CR³=CR³- oder eine ortho-verknüpfte Arylen- oder Heteroarylengruppe mit 5 bis 14 aromatischen Ringatomen, welche durch einen oder mehrere Reste R³ substituiert sein kann;
R⁴ ist gleich oder verschieden bei jedem Auftreten F, eine geradkettige Alkyl- oder Alkoxygruppe mit 1 bis 20 C-Atomen, eine verzweigte oder cyclische Alkyl- oder Alkoxygruppe mit 3 bis 20 C-Atomen, die jeweils mit einem oder mehreren Resten R³ substituiert sein kann, wobei eine oder mehrere nicht benachbarte CH₂-Gruppen durch R³C=CR³, C≡C, Si(R³)₂, C=O, NR³, O, S oder CONR³ ersetzt sein können und wobei ein oder mehrere H-Atome durch D oder F ersetzt sein können, oder ein aromatisches oder heteroaromatisches Ringsystem mit 5 bis 24 aromatischen Ringatomen, das jeweils durch einen oder mehrere Reste R³ substituiert sein kann, oder eine Aryloxy- oder Heteroaryloxygruppe mit 5 bis 24 aromatischen Ringatomen, die durch einen oder mehrere Reste R³ substituiert sein kann, oder eine Aralkyl- oder Heteroaralkylgruppe mit 5 bis 24 aromatischen Ringatomen, die durch einen oder mehrere Reste R³ substituiert sein kann; dabei können zwei Reste R⁴, welche an dasselbe Kohlenstoffatom gebunden sind, miteinander ein aliphatisches oder aromatisches Ringsystem bilden und so ein Spirosystem aufspannen; weiterhin kann R⁴ mit einem benachbarten Rest R, R¹ oder R² ein aliphatisches Ringsystem bilden;
mit der Maßgabe, dass in A¹-A²-A³ nicht zwei Heteroatome direkt aneinander gebunden sind.

11. Verbindung nach einem oder mehreren der Ansprüche 1 bis 10, ausgewählt aus den Strukturen der Formeln (12) bis (23), 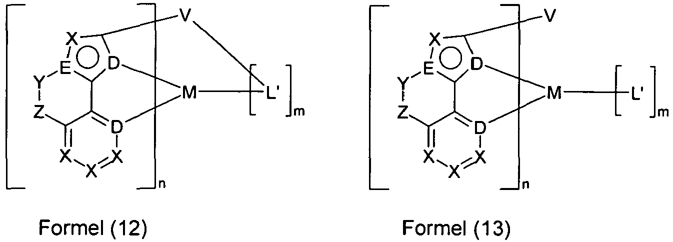 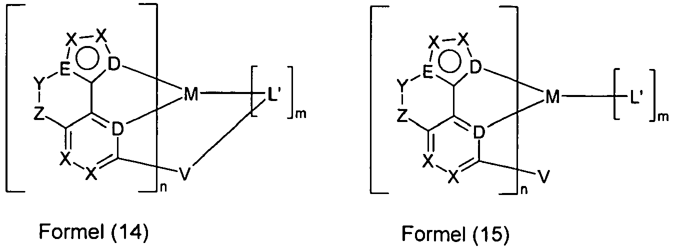 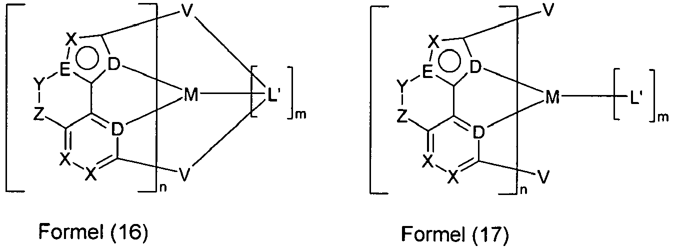 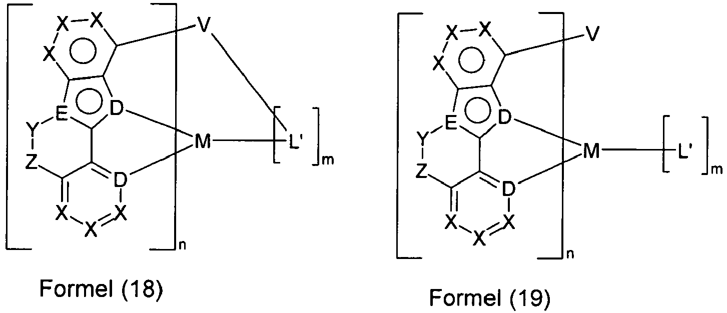 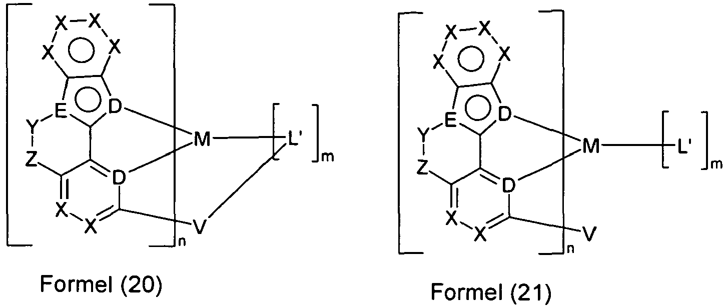 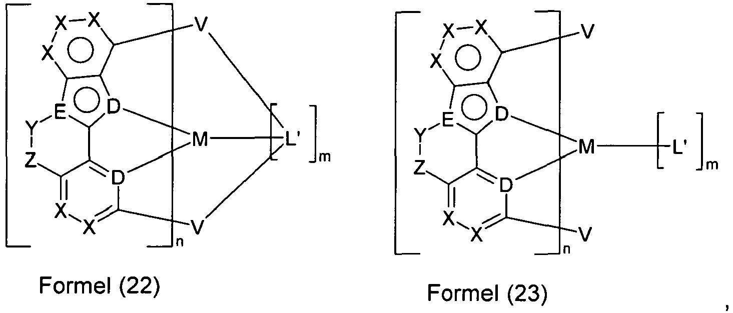 wobei die verwendeten Symbole und Indizes die in Anspruch 1 genannten Bedeutungen aufweisen und V eine Einfachbindung oder eine verbrückende Einheit darstellt, enthaltend 1 bis 80 Atome aus der dritten, vierten, fünften und/oder sechsten Hauptgruppe (Gruppe 13, 14, 15 oder 16 gemäß IUPAC) oder einen 3- bis 6-gliedrigen Homo- oder Heterocyclus, die die Teilliganden L miteinander oder L mit L' miteinander kovalent verbindet

12. Verbindung nach einem oder mehreren der Ansprüche 1 bis 11, **dadurch gekennzeichnet, dass** die Liganden L' ausgewählt sind aus der Gruppe bestehend aus Kohlenmonoxid, Stickstoffmonoxid, Alkylcyaniden, Arylcyaniden, Alkylisocyaniden, Arylisocyaniden, Aminen, Phosphinen, Phosphiten, Arsinen, Stibinen, stickstoffhaltigen Heterocyclen, Carbenen, Hydrid, Deuterid, den Halogeniden F⁻, Cl⁻, Br⁻ und I⁻, Alkylacetyliden, Arylacetyliden, Cyanid, Cyanat, Isocyanat, Thiocyanat, Isothiocyanat, aliphatischen oder aromatischen Alkoholaten, aliphatischen oder aromatischen Thioalkoholaten, Amiden, Carboxylaten, Arylgruppen, O²⁻, S²⁻, Carbide, Nitrene, N³⁻, Diaminen, Iminen, Diiminen, Heterocyclen enthaltend zwei Stickstoffatome, Diphosphinen, 1,3-Diketonaten abgeleitet von 1,3-Diketonen, 3-Ketonaten abgeleitet von 3-Ketoestern, Carboxylaten abgeleitet von Aminocarbonsäuren, Salicyliminaten abgeleitet von Salicyliminen, Dialkoholaten abgeleitet von Dialkoholen, Dithiolaten abgeleitet von Dithiolen, Bis(pyrazolylboraten), Bis(imidazolyl)boraten, 3-(2-Pyridyl)-diazolen, 3-(2-Pyridyl)-triazolen oder bidentate monoanionische, neutrale oder dianionische Liganden, insbesondere monoanionische Liganden, welche mit dem Metall einen cyclometallierten Fünfring oder Sechsring mit mindestens einer Metall-Kohlenstoff-Bindung aufweisen.

13. Verfahren zur Herstellung einer Verbindung nach einem oder mehreren der Ansprüche 1 bis 12 durch Umsetzung der freien Liganden L und gegebenenfalls L' mit Metallalkoholaten der Formel (75), mit Metallketoketonaten der Formel (76), mit Metallhalogeniden der Formel (77), mit dimeren Metallkomplexen der Formel (78) oder mit Metallkomplexen der Formel (79), 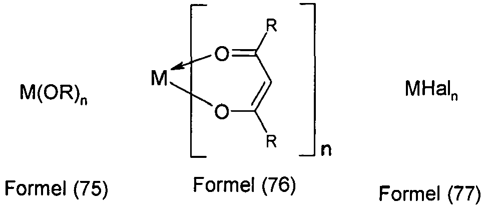 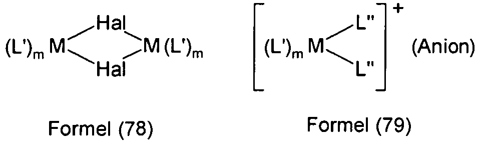 wobei die Symbole M, m, n und R die in Anspruch 1 angegebenen Bedeutungen haben, Hal = F, Cl, Br oder I ist, L" für einen Alkohol oder ein Nitril steht und (Anion) ein nicht-koordinierendes Anion ist.

14. Oligomer, Polymer oder Dendrimer enthaltend eine oder mehrere Verbindungen nach einem oder mehreren der Ansprüche 1 bis 12, wobei ein oder mehrere Bindungen der Verbindung zum Polymer, Oligomer oder Dendrimer vorhanden sind.

15. Formulierung, enthaltend eine Verbindung nach einem oder mehreren der Ansprüche 1 bis 12 bzw. ein Oligomer, Polymer oder Dendrimer nach Anspruch 14 und mindestens eine weitere Verbindung, insbesondere ein Lösemittel sein und/oder eine weitere organische oder anorganische Verbindung, die ebenfalls in der elektronischen Vorrichtung eingesetzt wird.

16. Verwendung einer Verbindung nach einem oder mehreren der Ansprüche 1 bis 12 oder eines Oligomers, Polymers oder Dendrimers nach Anspruch 14 in einer elektronischen Vorrichtung.

17. Elektronische Vorrichtung, insbesondere ausgewählt aus der Gruppe bestehend aus aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden, enthaltend in mindestens einer Schicht mindestens eine Verbindung nach einem oder mehreren der Ansprüche 1 bis 12 oder ein Oligomer, Polymer oder Dendrimer nach Anspruch 14.

## Claims

1. Compound of formula (1),
M(L)ₙ(L')ₘ formula (1)
which contains a moiety M(L)ₙ of the formula (2) or formula (3): 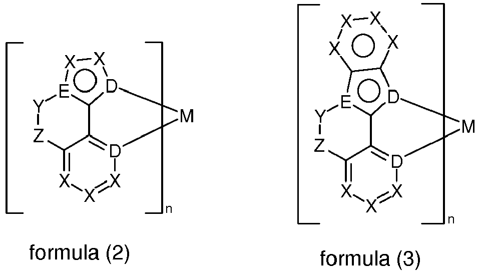 where the following applies to the symbols and indices used:
M is a transition metal;
X is selected on each occurrence, identically or differently, from the group consisting of CR and N;
Y is selected on each occurrence, identically or differently, from the group consisting of C(R¹)₂, Si(R¹)₂, PR¹, P(=O)R¹ or BR¹;
Z is selected on each occurrence, identically or differently, from the group consisting of NR¹, O or C(R¹)₂;
D is on each occurrence, identically or differently, C or N, with the proviso that at least one D stands for N;
E is on each occurrence, identically or differently, C or N, with the proviso that at least one of the groups E or D in the five-membered ring stands for N;
R, R¹ is on each occurrence, identically or differently, H, D, F, Cl, Br, I, N(R²)₂, CN, NO₂, OH, COOH, C(=O)N(R²)₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R², where one or more non-adjacent CH₂ groups may be replaced by R²C=CR², C≡C, Si(R²)₂, C=O, NR², O, S or CONR² and where one or more H atoms may be replaced by D, F, Cl, Br, I or CN, or an aromatic or heteroaromatic ring system having 5 to 60 aromatic ring atoms, which may in each case be substituted by one or more radicals R², or an aryloxy or heteroaryloxy group having 5 to 40 aromatic ring atoms, which may be substituted by one or more radicals R², or an aralkyl or heteroaralkyl group having 5 to 40 aromatic ring atoms, which may be substituted by one or more radicals R², or a diarylamino group, diheteroarylamino group or arylheteroarylamino group having 10 to 40 aromatic ring atoms, which may be substituted by one or more radicals R²; two adjacent radicals R or two adjacent radicals R¹ here may also form a mono- or polycyclic, aliphatic, aromatic or heteroaromatic ring system with one another; furthermore R and R¹ may also form a mono- or polycyclic, aliphatic or heteroaromatic ring system with one another;
R² is on each occurrence, identically or differently, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, a straight-chain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R³, where one or more non-adjacent CH₂ groups may be replaced by R³C=CR³, C≡C, Si(R³)₂, C=O, NR³, O, S or CONR³ and where one or more H atoms may be replaced by D, F, Cl, Br, I, CN or NO₂, or an aromatic or heteroaromatic ring system having 5 to 60 aromatic ring atoms, which may in each case be substituted by one or more radicals R³, or an aryloxy or heteroaryloxy group having 5 to 40 aromatic ring atoms, which may be substituted by one or more radicals R³, or an aralkyl or heteroaralkyl group having 5 to 40 aromatic ring atoms, which may be substituted by one or more radicals R³, or a diarylamino group, diheteroarylamino group or arylheteroarylamino group having 10 to 40 aromatic ring atoms, which may be substituted by one or more radicals R³; two or more adjacent radicals R² with one another or R² with R or with R¹ here may form a mono- or polycyclic, aliphatic, aromatic or heteroaromatic ring system;
R³ is on each occurrence, identically or differently, H, D, F or an aliphatic, aromatic and/or heteroaromatic hydrocarbon radical having 1 to 20 C atoms, in which, in addition, one or more H atoms may be replaced by F; two or more substituents R³ here may also form a mono- or polycyclic, aliphatic ring system with one another;
L' is, identically or differently on each occurrence, any desired co-ligand;
n is 1, 2 or 3;
m is 0, 1, 2, 3 or 4;
a plurality of ligands L with one another or L with L' may also be linked here via a single bond or a divalent or trivalent bridge and thus form a tridentate, tetradentate, pentadentate or hexadentate ligand system, where in this case L' is not a separate co-ligand, but instead a coordinating group;
furthermore a substituent R may also additionally be coordinated to the metal;
the following compounds are excluded from the invention:

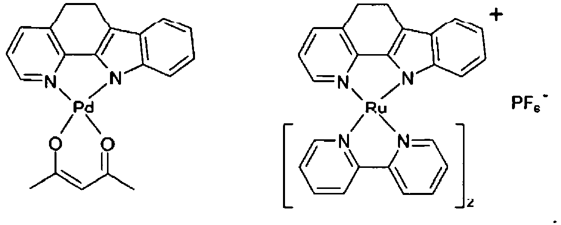

2. Compound according to Claim 1, **characterised in that** M is selected from the group consisting of chromium, molybdenum, tungsten, rhenium, ruthenium, osmium, rhodium, iridium, nickel, palladium, platinum, copper, silver and gold, in particular molybdenum, tungsten, rhenium, ruthenium, osmium, iridium, copper, platinum and gold.

3. Compound according to Claim 1 or 2, **characterised in that** the moieties of the formula (2) are selected from the moieties of the formulae (2a), (2b) and (2c) and the moieties of the formula (3) are selected from the moieties of the formulae (3a), (3b) and (3c), 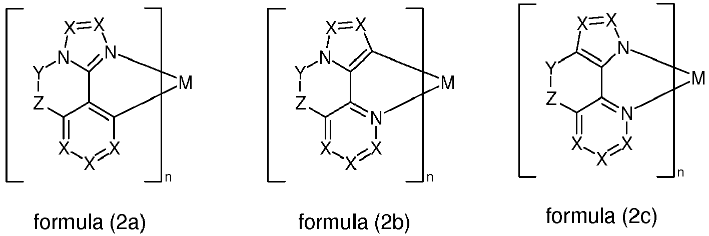 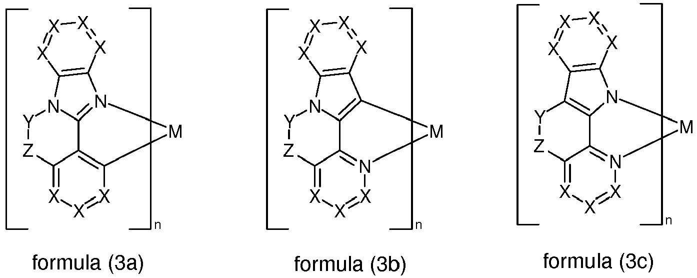 where the symbols and indices used have the meanings given in Claim 1.

4. Compound according to one or more of Claims 1 to 3, **characterised in that** the group -Y-Z- stands, identically or differently on each occurrence,
for -C(R¹)₂-NR¹-, -C(R¹)₂-O-, -Si(R¹)₂-NR¹-, -Si(R¹)₂-O- or -C(R¹)₂-C(R¹) 2-.

5. Compound according to one or more of Claims 1 to 4, **characterised in that** R¹ is selected, identically or differently on each occurrence, from the group consisting of F, CN, a straight-chain alkyl group having 1 to 10 C atoms or a branched or cyclic alkyl group having 3 to 10 C atoms, which may in each case be substituted by one or more radicals R², where one or more non-adjacent CH₂ groups may be replaced by R²C=CR² and where one or more H atoms may be replaced by F, or an aromatic or heteroaromatic ring system having 5 to 30 aromatic ring atoms, which may in each case be substituted by one or more radicals R², or an aryloxy or heteroaryloxy group having 5 to 30 aromatic ring atoms, which may be substituted by one or more radicals R², or an aralkyl or heteroaralkyl group having 5 to 40 aromatic ring atoms, which may be substituted by one or more radicals R², or a diarylamino group, diheteroarylamino group or arylheteroarylamino group having 10 to 30 aromatic ring atoms, which may be substituted by one or more radicals R²; two adjacent radicals R¹ here may also form a mono- or polycyclic, aliphatic, aromatic or heteroaromatic ring system with one another; furthermore R and R¹ may also form a mono- or polycyclic, aliphatic or heteroaromatic ring system with one another;

6. Compound according to one or more of Claims 1 to 5, **characterised in that** no, one or two groups X in the ligand L stand for N.

7. Compound according to one or more of Claims 1 to 6, **characterised in that** the moieties of the formula (2) are selected from the moieties of the formulae (2-A) to (2-G) and **in that** the moieties of the formula (3) are selected from the moieties of the formulae (3-A) to (3-H), 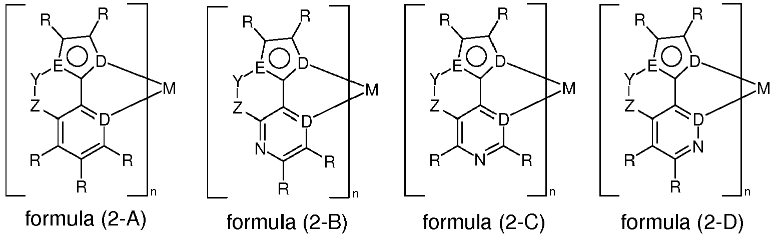 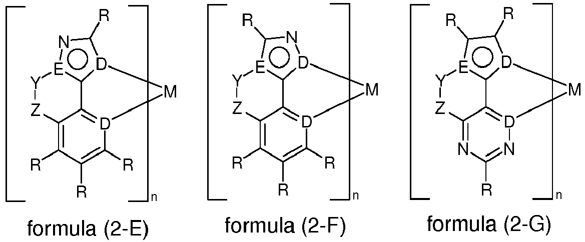 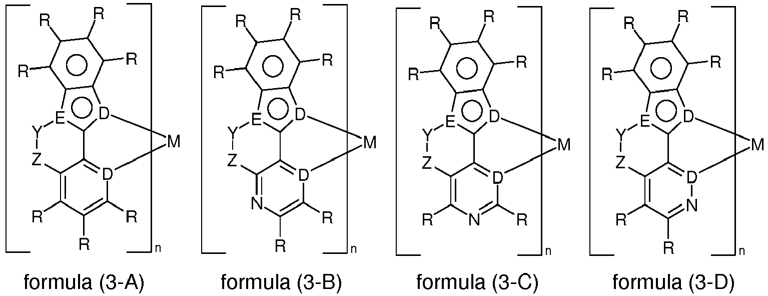 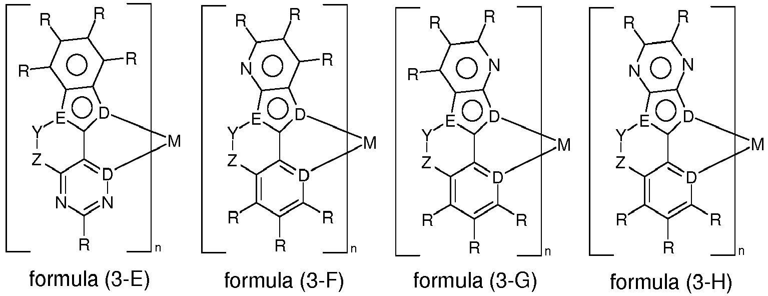 where the symbols and indices used have the meanings given in Claim 1.

8. Compound according to one or more of Claims 1 to 7, **characterised in that** the group E stands for N and either the group D in the five-membered ring or the group D in the six-membered ring stands for N, while the other group D stands for C, or **in that** the group E stands for C and the two groups D stand for N.

9. Compound according to one or more of Claims 1 to 8, **characterised in that**, if one or more groups X stand for nitrogen, a group R which is not equal to hydrogen or deuterium, preferably a group selected from CF₃, OCF₃, alkyl or alkoxy groups having 1 to 10 C atoms, in particular branched or cyclic alkyl or alkoxy groups having 3 to 10 C atoms, aromatic or heteroaromatic ring systems or aralkyl or heteroaralkyl groups, where these groups may each optionally be substituted by one or more radicals R², is bonded as substituent adjacent to this nitrogen atom.

10. Compound according to one or more of Claims 1 to 9, **characterised in that** two adjacent groups X stand for CR and the respective radicals R, together with the C atoms, form a ring of the formula (4) or formula (5), 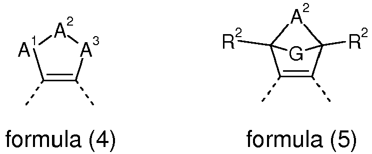 where R² and R³ have the meanings given in Claim 1, the dashed bonds indicate the linking of the two carbon atoms in the ligand and furthermore:
A¹, A³ is, identically or differently on each occurrence, C(R⁴)₂, O, S, NR⁴ or C(=O);
A² is C(R²)₂, O, S, NR⁴ or C(=O);
G is an alkylene group having 1, 2 or 3 C atoms, which may be substituted by one or more radicals R³, or is -CR³=CR³- or an ortho-linked arylene or heteroarylene group having 5 to 14 aromatic ring atoms, which may be substituted by one or more radicals R³;
R⁴ is, identically or differently on each occurrence, F, a straight-chain alkyl or alkoxy group having 1 to 20 C atoms, a branched or cyclic alkyl or alkoxy group having 3 to 20 C atoms, which may in each case be substituted by one or more radicals R³, where one or more non-adjacent CH₂ groups may be replaced by R³C=CR³, C≡C, Si(R³)₂, C=O, NR³, O, S or CONR³ and where one or more H atoms may be replaced by D or F, or an aromatic or heteroaromatic ring system having 5 to 24 aromatic ring atoms, which may in each case be substituted by one or more radicals R³, or an aryloxy or heteroaryloxy group having 5 to 24 aromatic ring atoms, which may be substituted by one or more radicals R³, or an aralkyl or heteroaralkyl group having 5 to 24 aromatic ring atoms, which may be substituted by one or more radicals R³; two radicals R⁴ which are bonded to the same carbon atom may form an aliphatic or aromatic ring system with one another here and thus form a spiro system; furthermore, R⁴ may form an aliphatic ring system with an adjacent radical R, R¹ or R²;
with the proviso that no two heteroatoms in A¹-A²-A³ are bonded directly to one another.

11. Compound according to one or more of Claims 1 to 10, selected from the structures of the formulae (12) to (23), 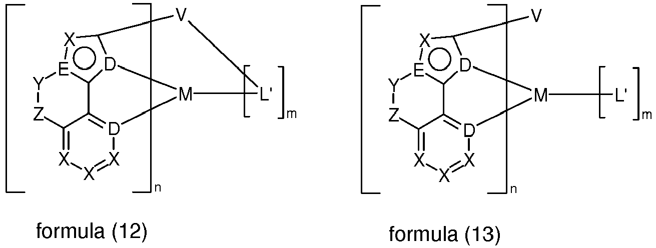 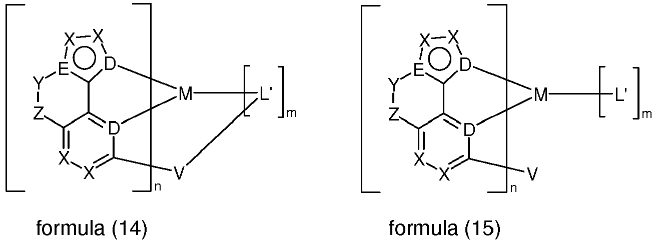 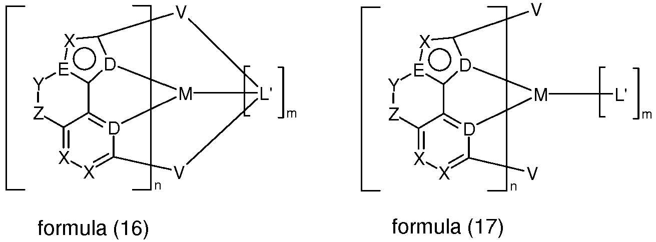 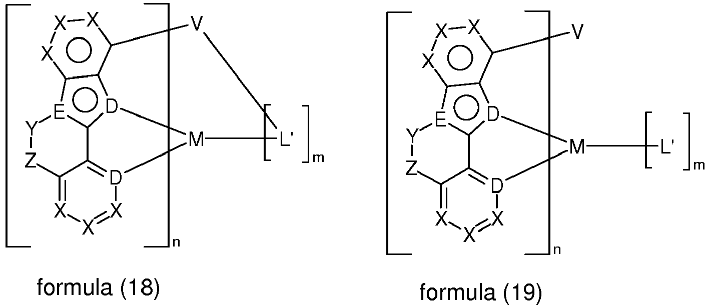 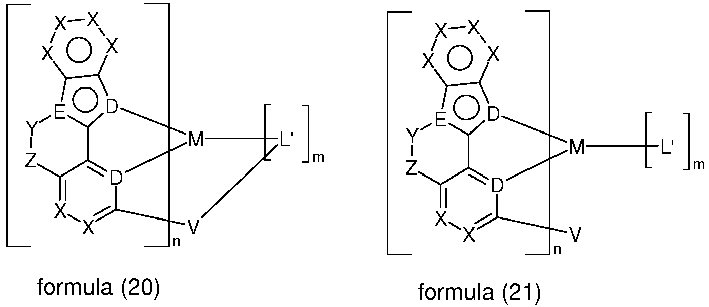 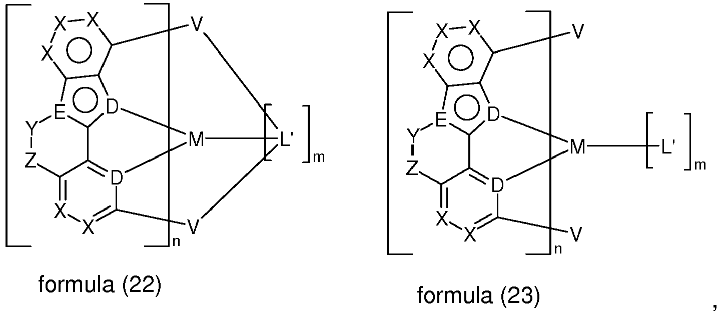 where the symbols and indices used have the meanings given in Claim 1 and V represents a single bond or a bridging unit containing 1 to 80 atoms from the third, fourth, fifth and/or sixth main group (group 13, 14, 15 or 16 in accordance with IUPAC) or a 3- to 6-membered homo- or heterocycle which covalently bonds the part-ligands L to one another or L to L'.

12. Compound according to one or more of Claims 1 to 11, **characterised in that** the ligands L' are selected from the group consisting of carbon monoxide, nitrogen monoxide, alkyl cyanides, aryl cyanides, alkyl isocyanides, aryl isocyanides, amines, phosphines, phosphites, arsines, stibines, nitrogen-containing heterocycles, carbenes, hydride, deuteride, the halides F⁻, Cl⁻, Br⁻ and I⁻, alkylacetylides, arylacetylides, cyanide, cyanate, isocyanate, thiocyanate, isothiocyanate, aliphatic or aromatic alcoholates, aliphatic or aromatic thioalcoholates, amides, carboxylates, aryl groups, O²⁻, S²⁻, carbides, nitrenes, N³⁻, diamines, imines, diimines, heterocycles containing two nitrogen atoms, diphosphines, 1,3-diketonates derived from 1,3-diketones, 3-ketonates derived from 3-ketoesters, carboxylates derived from aminocarboxylic acids, salicyliminates derived from salicylimines, dialcoholates derived from dialcohols, dithiolates derived from dithiols, bis(pyrazolylborates), bis(imidazolyl)borates, 3-(2-pyridyl)diazoles, 3-(2-pyridyl)triazoles, bi-dentate monoanionic, neutral or dianionic ligands, in particular monoanionic ligands, which, with the metal, form a cyclometallated five-membered ring or six-membered ring having at least one metal-carbon bond.

13. Process for the preparation of a compound according to one or more of Claims 1 to 12 by reaction of the free ligands L and optionally L' with metal alkoxides of the formula (75), with metal ketoketonates of the formula (76), with metal halides of the formula (77), with dimeric metal complexes of the formula (78) or with metal complexes of the formula (79), 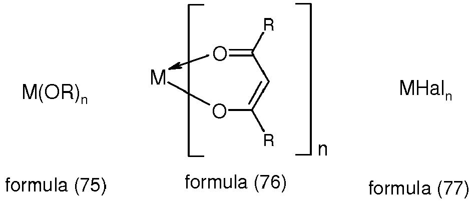 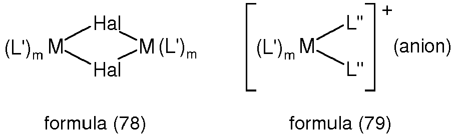 where the symbols M, m, n and R have the meanings indicated in Claim 1, Hal = F, Cl, Br or I, L" stands for an alcohol or a nitrile and (anion) is a non-coordinating anion.

14. Oligomer, polymer or dendrimer containing one or more compounds according to one or more of Claims 1 to 12, where one or more bonds are present from the compound to the polymer, oligomer or dendrimer.

15. Formulation comprising a compound according to one or more of Claims 1 to 12 or an oligomer, polymer or dendrimer according to Claim 14 and at least one further compound, in particular a solvent and/or a further organic or inorganic compound which is likewise employed in the electronic device.

16. Use of a compound according to one or more of Claims 1 to 12 or an oligomer, polymer or dendrimer according to Claim 14 in an electronic device.

17. Electronic device, in particular selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes, comprising in at least one layer at least one compound according to one or more of Claims 1 to 12 or an oligomer, polymer or dendrimer according to Claim 14.

## Revendications

1. Composé de la formule (1) :
M(L)ₙ(L')ₘ formule (1)
lequel composé contient une moitié M(L)ₙ de la formule (2) ou de la formule (3) : 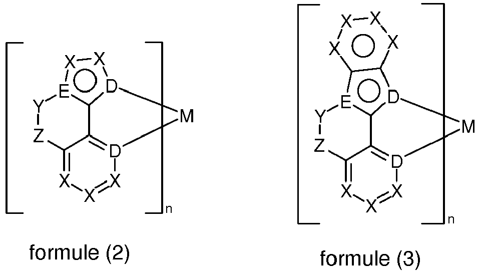 formules dans lesquelles ce qui suit s'applique aux symboles et indices qui sont utilisés :
M est un métal de transition ;
X est sélectionné pour chaque occurrence, de manière identique ou différente, parmi le groupe qui est constitué par CR et N ;
Y est sélectionné pour chaque occurrence, de manière identique ou différente, parmi le groupe qui est constitué par C(R¹)₂, Si(R¹)₂, PR¹, P(=O)R¹ et BR¹ ;
Z est sélectionné pour chaque occurrence, de manière identique ou différente, parmi le groupe qui est constitué par NR¹, O et C(R¹)₂ ;
D est pour chaque occurrence, de manière identique ou différente, C ou N, étant entendu qu'au moins un D représente N ;
E est pour chaque occurrence, de manière identique ou différente, C ou N, étant entendu qu'au moins l'un des groupes E et D dans le cycle à cinq éléments représente N ;
R, R¹ sont pour chaque occurrence, de manière identique ou différente, H, D, F, CI, Br, I, N(R²)₂, CN, NO₂, OH, COOH, C(=O)N(R²)₂, Si(R²)₃, B(OR²)₂, C(=O)R², P(=O)(R²)₂, S(=O)R², S(=O)₂R², OSO₂R², un groupe alkyle, alcoxy ou thioalcoxy en chaîne droite qui comporte 1 à 20 atome(s) de C ou un groupe alkényle ou alkynyle qui comporte 2 à 20 atomes de C ou un groupe alkyle, alcoxy ou thioalcoxy ramifié ou cyclique qui comporte 3 à 20 atomes de C, lequel groupe peut dans chaque cas être substitué par un radical ou par plusieurs radicaux R², où un ou plusieurs groupe(s) CH₂ non adjacents peut/peuvent être remplacé(s) par R²C=CR², C=C, Si(R²)₂, C=O, NR², O, S ou CONR² et où un ou plusieurs atome(s) de H peut/peuvent être remplacé(s) par D, F, CI, Br, I ou CN, ou un système de cycle aromatique ou hétéroaromatique qui comporte 5 à 60 atomes de cycle aromatique, lequel système de cycle peut dans chaque cas être substitué par un radical ou par plusieurs radicaux R², ou un groupe aryloxy ou hétéroaryloxy qui comporte 5 à 40 atomes de cycle aromatique, lequel groupe peut être substitué par un radical ou par plusieurs radicaux R², ou un groupe aralkyle ou hétéroaralkyle qui comporte 5 à 40 atomes de cycle aromatique, lequel groupe peut être substitué par un radical ou par plusieurs radicaux R², ou un groupe diarylamino, un groupe dihétéroarylamino ou un groupe arylhétéroarylamino qui comporte 10 à 40 atomes de cycle aromatique, lequel groupe peut être substitué par un radical ou par plusieurs radicaux R² ; deux radicaux R adjacents ou deux radicaux R¹ adjacents peuvent ici également former un système de cycle aliphatique, aromatique ou hétéroaromatique monocyclique ou polycyclique l'un avec l'autre ; qui plus est, R et R¹ peuvent également former un système de cycle aliphatique ou hétéroaromatique monocyclique ou polycyclique l'un avec l'autre;
R² est pour chaque occurrence, de manière identique ou différente, H, D, F, CI, Br, I, N(R³)₂, CN, NO₂, Si(R³)₃, B(OR³)₂, C(=O)R³, P(=O)(R³)₂, S(=O)R³, S(=O)₂R³, OSO₂R³, un groupe alkyle, alcoxy ou thioalcoxy en chaîne droite qui comporte 1 à 20 atome(s) de C ou un groupe alkényle ou alkynyle qui comporte 2 à 20 atomes de C ou un groupe alkyle, alcoxy ou thioalcoxy ramifié ou cyclique qui comporte 3 à 20 atomes de C, lequel groupe peut dans chaque cas être substitué par un radical ou par plusieurs radicaux R³, où un ou plusieurs groupe(s) CH₂ non adjacents peut/peuvent être remplacé(s) par R³C=CR³, C=C, Si(R³)₂, C=O, NR³, O, S ou CONR³ et où un ou plusieurs atome(s) de H peut/peuvent être remplacé(s) par D, F, CI, Br, I, CN ou NO₂, ou un système de cycle aromatique ou hétéroaromatique qui comporte 5 à 60 atomes de cycle aromatique, lequel système de cycle peut dans chaque cas être substitué par un radical ou par plusieurs radicaux R³, ou un groupe aryloxy ou hétéroaryloxy qui comporte 5 à 40 atomes de cycle aromatique, lequel groupe peut être substitué par un radical ou par plusieurs radicaux R³, ou un groupe aralkyle ou hétéroaralkyle qui comporte 5 à 40 atomes de cycle aromatique, lequel groupe peut être substitué par un radical ou par plusieurs radicaux R³, ou un groupe diarylamino, un groupe dihétéroarylamino ou un groupe arylhétéroarylamino qui comporte 10 à 40 atomes de cycle aromatique, lequel groupe peut être substitué par un radical ou par plusieurs radicaux R³ ; deux radicaux R² adjacents l'un à l'autre ou plus adjacents les uns aux autres ou R² avec R ou avec R¹ peuvent ici former un système de cycle aliphatique, aromatique ou hétéroaromatique monocyclique ou polycyclique ;
R³ est pour chaque occurrence, de manière identique ou différente, H, D, F ou un radical hydrocarbone aliphatique, aromatique et/ou hétéroaromatique qui comporte 1 à 20 atome(s) de C, où, en outre, un ou plusieurs atome(s) de H peut/peuvent être remplacé(s) par F ; deux substituants R³ ou plus peuvent ici également former un système de cycle aliphatique monocyclique ou polycyclique l'un avec l'autre ou les uns avec les autres ;
L' est, de manière identique ou différente pour chaque occurrence, n'importe quel co-ligand souhaité ;
n est 1, 2 ou 3 ;
m est 0, 1, 2, 3 ou 4 ;
une pluralité de ligands L les uns avec les autres ou le ligand L avec le ligand L' peuvent également être liés ici via une liaison simple ou un pont divalent ou trivalent et ainsi former un système de ligands tridenté, tétradenté, pentadenté ou hexadenté, où, dans ce cas, le ligand L' n'est pas un co-ligand séparé, mais en lieu et place, est un groupe de coordination ;
qui plus est, un substituant R peut également, de manière additionnelle, être coordonné sur le métal ;
les composés qui suivent sont exclus de l'invention :

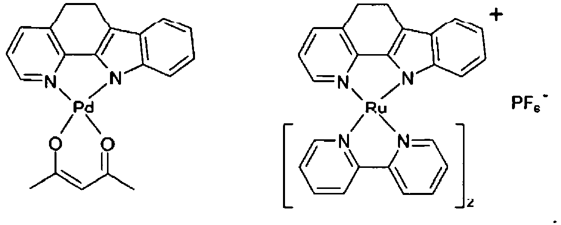

2. Composé selon la revendication 1, **caractérisé en ce que** M est sélectionné parmi le groupe qui est constitué par le chrome, le molybdène, le tungstène, le rhénium, le ruthénium, l'osmium, le rhodium, l'iridium, le nickel, le palladium, le platine, le cuivre, l'argent et l'or, en particulier le molybdène, le tungstène, le rhénium, le ruthénium, l'osmium, l'iridium, le cuivre, le platine et l'or.

3. Composé selon la revendication 1 ou 2, **caractérisé en ce que** les moitiés de la formule (2) sont sélectionnées parmi les moitiés des formules (2a), (2b) et (2c) et les moitiés de la formule (3) sont sélectionnées parmi les moitiés des formules (3a), (3b) et (3c) : 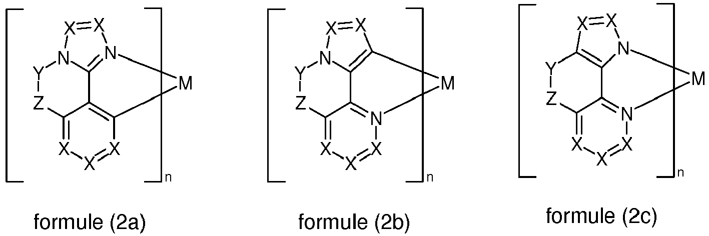 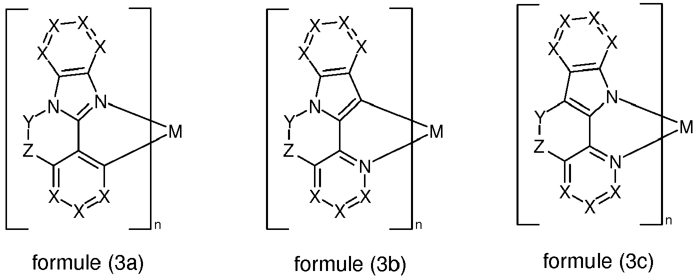 formules dans lesquelles les symboles et indices qui sont utilisés présentent les significations qui ont été données selon la revendication 1.

4. Composé selon une ou plusieurs des revendications 1 à 3, **caractérisé en ce que** le groupe -Y-Z- représente, de manière identique ou différente pour chaque occurrence, -C(R¹)₂-NR¹-, -C(R¹)₂-O-, -Si(R¹)₂-NR¹-, -Si(R¹)₂-O- ou -C(R¹)₂-C(R¹)₂-.

5. Composé selon une ou plusieurs des revendications 1 à 4, **caractérisé en ce que** R¹ est sélectionné, de manière identique ou différente pour chaque occurrence, parmi le groupe qui est constitué par F, CN, un groupe alkyle en chaîne droite qui comporte 1 à 10 atome(s) de C ou un groupe alkyle ramifié ou cyclique qui comporte 3 à 10 atomes de C, lequel groupe peut dans chaque cas être substitué par un radical ou par plusieurs radicaux R², où un ou plusieurs groupe(s) CH₂ non adjacents peut/peuvent être remplacé(s) par R²C=CR² et où un ou plusieurs atome(s) de H peut/peuvent être remplacé(s) par F, ou un système de cycle aromatique ou hétéroaromatique qui comporte 5 à 30 atomes de cycle aromatique, lequel système de cycle peut dans chaque cas être substitué par un radical ou par plusieurs radicaux R², ou un groupe aryloxy ou hétéroaryloxy qui comporte 5 à 30 atomes de cycle aromatique, lequel groupe peut être substitué par un radical ou par plusieurs radicaux R², ou un groupe aralkyle ou hétéroaralkyle qui comporte 5 à 40 atomes de cycle aromatique, lequel groupe peut être substitué par un radical ou par plusieurs radicaux R², ou un groupe diarylamino, un groupe dihétéroarylamino ou un groupe arylhétéroarylamino qui comporte 10 à 30 atomes de cycle aromatique, lequel groupe peut être substitué par un radical ou par plusieurs radicaux R² ; deux radicaux R¹ adjacents peuvent ici également former un système de cycle aliphatique, aromatique ou hétéroaromatique monocyclique ou polycyclique l'un avec l'autre ; qui plus est, R et R¹ peuvent également former un système de cycle aliphatique ou hétéroaromatique monocyclique ou polycyclique l'un avec l'autre.

6. Composé selon une ou plusieurs des revendications 1 à 5, **caractérisé en ce qu'**aucun groupe X dans le ligand L ne représente N, ou un ou deux groupe(s) X dans le ligand L représente(nt) N.

7. Composé selon une ou plusieurs des revendications 1 à 6, **caractérisé en ce que** les moitiés de la formule (2) sont sélectionnées parmi les moitiés des formules (2-A) à (2-G) et **en ce que** les moitiés de la formule (3) sont sélectionnées parmi les moitiés des formules (3-A) à (3-H) : 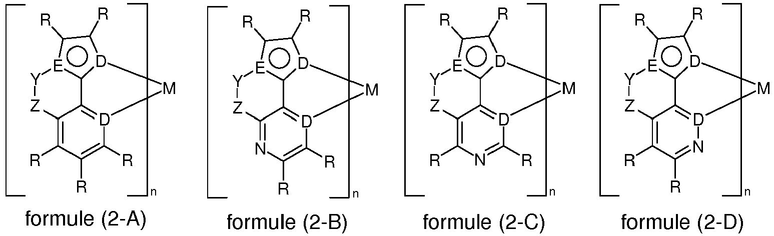 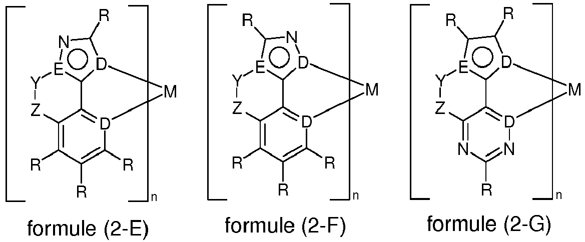 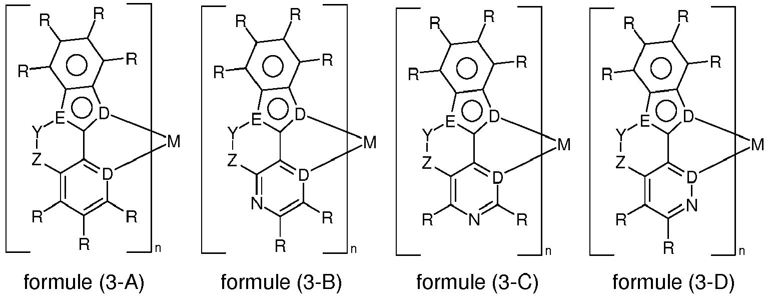 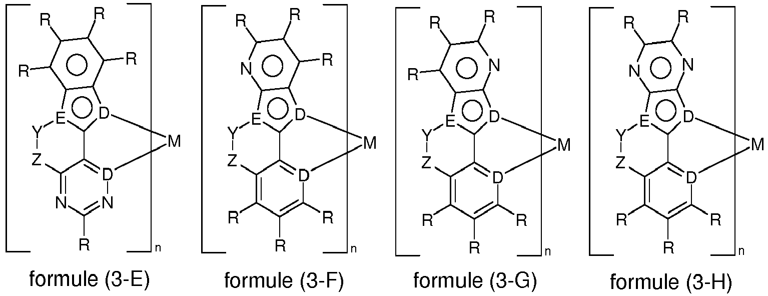 formules dans lesquelles les symboles et indices qui sont utilisés présentent les significations qui ont été données selon la revendication 1.

8. Composé selon une ou plusieurs des revendications 1 à 7, **caractérisé en ce que** le groupe E représente N et soit le groupe D dans le cycle à cinq éléments, soit le groupe D dans le cycle à six éléments représente N, tandis que l'autre groupe D représente C, ou **en ce que** le groupe E représente C et les deux groupes D représentent N.

9. Composé selon une ou plusieurs des revendications 1 à 8, **caractérisé en ce que**, si un ou plusieurs groupe(s) X représente(nt) azote, un groupe R qui n'est égal ni à hydrogène, ni à deutérium, de façon préférable un groupe qui est sélectionné parmi CF₃, OCF₃, des groupes alkyle ou alcoxy qui comportent 1 à 10 atome(s) de C, en particulier des groupes alkyle ou alcoxy ramifiés ou cycliques qui comportent 3 à 10 atomes de C, des systèmes de cycle aromatique ou hétéroaromatique ou des groupes aralkyle ou hétéroaralkyle, où ces groupes peuvent chacun, en option, être substitués par un radical ou par plusieurs radicaux R², est lié en tant que substituant adjacent à cet atome d'azote.

10. Composé selon une ou plusieurs des revendications 1 à 9, **caractérisé en ce que** deux groupes X adjacents représentent CR et les radicaux respectifs R, en association avec les atomes de C, forment un cycle de la formule (4) ou de la formule (5) : 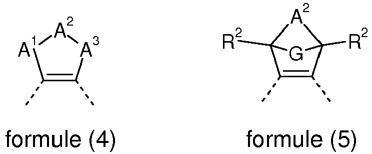 formules dans lesquelles R² et R³ présentent les significations qui ont été données selon la revendication 1, les liens en pointillés indiquent la liaison des deux atomes de carbone dans le ligand et qui plus est :
A¹, A³ sont, de manière identique ou différente pour chaque occurrence, C(R⁴)₂, O, S, NR⁴ ou C(=O) ;
A² est C(R²)₂, O, S, NR⁴ ou C(=O) ;
G est un groupe alkylène qui comporte 1, 2 ou 3 atome(s) de C, lequel groupe peut être substitué par un radical ou par plusieurs radicaux R³, ou est -CR³=CR³- ou un groupe arylène ou hétéroarylène ortho-lié qui comporte 5 à 14 atomes de cycle aromatique, lequel groupe peut être substitué par un radical ou par plusieurs radicaux R³ ;
R⁴ est, de manière identique ou différente pour chaque occurrence, F, un groupe alkyle ou alcoxy en chaîne droite qui comporte 1 à 20 atome(s) de C, un groupe alkyle ou alcoxy ramifié ou cyclique qui comporte 3 à 20 atomes de C, lequel groupe peut dans chaque cas être substitué par un radical ou par plusieurs radicaux R³, où un ou plusieurs groupe(s) CH₂ non adjacents peut/ peuvent être remplacé(s) par R³C=CR³, C=C, Si(R³)₂, C=O, NR³, O, S ou CONR³ et où un ou plusieurs atome(s) de H peut/ peuvent être remplacé(s) par D ou F, ou un système de cycle aromatique ou hétéroaromatique qui comporte 5 à 24 atomes de cycle aromatique, lequel système de cycle peut dans chaque cas être substitué par un radical ou par plusieurs radicaux R³, ou un groupe aryloxy ou hétéroaryloxy qui comporte 5 à 24 atomes de cycle aromatique, lequel groupe peut être substitué par un radical ou par plusieurs radicaux R³, ou un groupe aralkyle ou hétéroaralkyle qui comporte 5 à 24 atomes de cycle aromatique, lequel groupe peut être substitué par un radical ou par plusieurs radicaux R³ ; deux radicaux R⁴ qui sont liés au même atome de carbone peuvent former un système de cycle aliphatique ou aromatique l'un avec l'autre ici et peuvent de fait former un système spiro ; qui plus est, R⁴ peut former un système de cycle aliphatique avec un radical R, R¹ ou R² adjacent ;
étant entendu que deux hétéroatomes dans A¹-A²-A³ ne sont pas liés directement l'un à l'autre.

11. Composé selon une ou plusieurs des revendications 1 à 10, sélectionné parmi les structures des formules (12) à (23) : 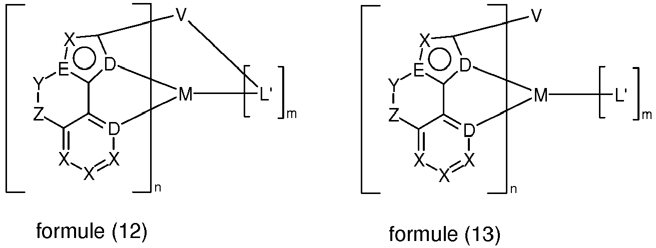 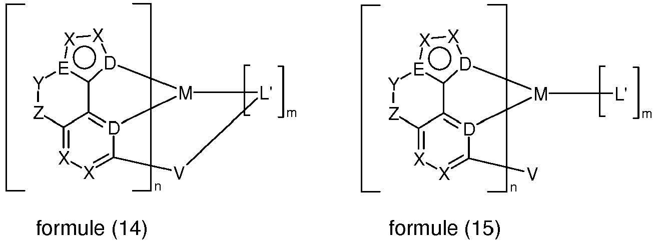 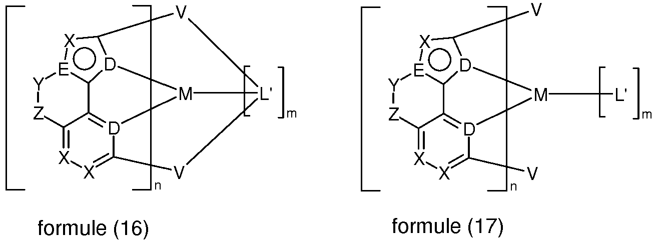 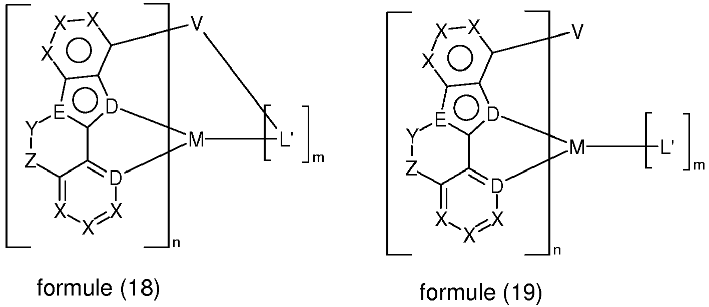 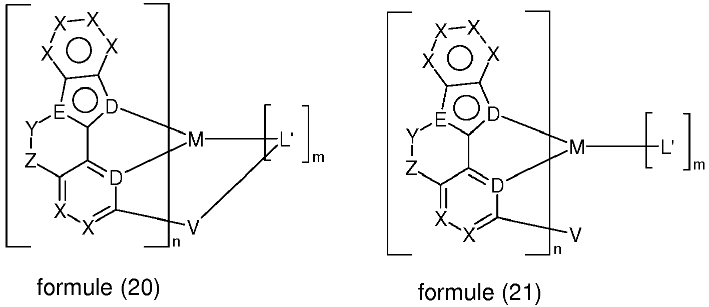 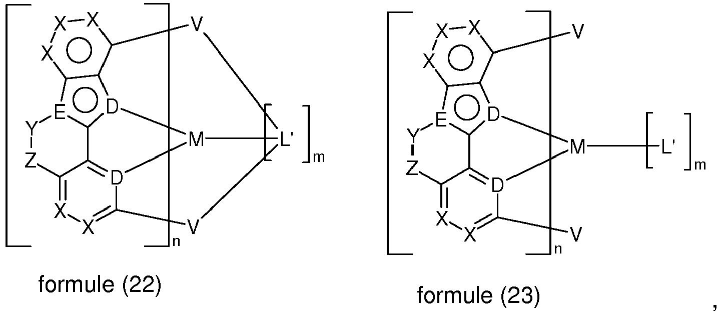 formules dans lesquelles les symboles et indices qui sont utilisés présentent les significations qui ont été données selon la revendication 1 et V représente une liaison simple ou une unité de pontage qui contient 1 à 80 atome(s), à partir des troisième, quatrième, cinquième et/ou sixième groupes principaux (le groupe 13, 14, 15 ou 16 conformément à IUPAC) ou un homocycle ou hétérocycle de 3 à 6 éléments, lequel lie de manière covalente les ligands de partie L l'un à l'autre ou les uns aux autres ou le ligand L au ligand L'.

12. Composé selon une ou plusieurs des revendications 1 à 11, **caractérisé en ce que** les ligands L' sont sélectionnés parmi le groupe qui est constitué par le monoxyde de carbone, le monoxyde d'azote, les cyanures d'alkyle, les cyanures d'aryle, les isocyanures d'alkyle, les isocyanures d'aryle, les amines, les phosphines, les phosphites, les arsines, les stibines, les hétérocycles qui contiennent de l'azote, les carbènes, un hydrure, un deutérure, les halogénures F⁻, Cl⁻, Br⁻ et I⁻, les alkylacétylures, les arylacétylures, le cyanure, le cyanate, l'isocyanate, le thiocyanate, l'isothiocyanate, les alcoolates aliphatiques ou aromatiques, les thioalcoolates aliphatiques ou aromatiques, les amides, les carboxylates, les groupes aryle, O²⁻, S²⁻, les carbures, les nitrènes, N³⁻, les diamines, les imines, les diimines, les hétérocycles qui contiennent deux atomes d'azote, les diphosphines, les 1,3-dicétonates qui sont dérivés à partir des 1,3-dicétones, les 3-cétonates qui sont dérivés à partir des 3-cétoesters, les carboxylates qui sont dérivés à partir d'acides aminocarboxyliques, les salicyliminates qui sont dérivés à partir des salicylimines, les dialcoolates qui sont dérivés à partir des dialcools, les dithiolates qui sont dérivés à partir des dithiols, les bis(pyrazolylborates), les bis(imidazolyl)borates, les 3-(2-pyridyl)diazoles, les 3-(2-pyridyl)triazoles, les ligands monoanioniques, neutres ou dianioniques bidentés, en particulier les ligands monoanioniques, lesquels ligands qui, avec le métal, forment un cycle à cinq éléments ou à six éléments cyclométallaté qui comporte au moins une liaison métal-carbone.

13. Procédé pour la préparation d'un composé selon une ou plusieurs des revendications 1 à 12 au moyen de la réaction des ligands libres L et en option L' avec des alcoxydes de métaux de la formule (75), avec des cétocétonates de métaux de la formule (76), avec des halogénures de métaux de la formule (77), avec des complexes de métaux dimériques de la formule (78) ou avec des complexes de métaux de la formule (79) : 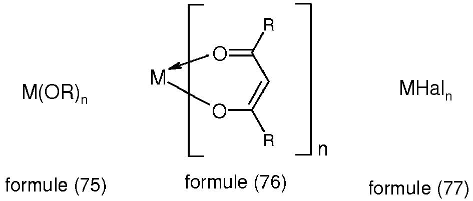 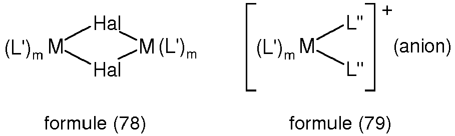 formules dans lesquelles les symboles M, m, n et R présentent les significations qui ont été indiquées selon la revendication 1, Hal = F, CI, Br ou I, L" représente un alcool ou un nitrile et (anion) est un anion de non coordination.

14. Oligomère, polymère ou dendrimère contenant un ou plusieurs composé(s) selon une ou plusieurs des revendications 1 à 12, où une ou plusieurs liaison(s) est/sont présente(s) depuis le composé sur le polymère, l'oligomère ou le dendrimère.

15. Formulation comprenant un composé selon une ou plusieurs des revendications 1 à 12 ou un oligomère, un polymère ou un dendrimère selon la revendication 14 et au moins un autre composé, en particulier un solvant et/ou un autre composé organique ou inorganique, lequel est de manière analogue utilisé dans le dispositif électronique.

16. Utilisation d'un composé selon une ou plusieurs des revendications 1 à 12 ou d'un oligomère, d'un polymère ou d'un dendrimère selon la revendication 14 dans un dispositif électronique.

17. Dispositif électronique, en particulier sélectionné parmi le groupe qui est constitué par les dispositifs électroluminescents organiques, les circuits intégrés organiques, les transistors à effet de champ organiques, les transistors à film mince organiques, les transistors à émission de lumière/électroluminescents organiques, les cellules solaires organiques, les détecteurs optiques organiques, les photorécepteurs organiques, les dispositifs à extinction de champ organiques, les cellules électrochimiques à émission de lumière ou électroluminescentes ou les diodes laser organiques, comprenant, dans au moins une couche, au moins un composé selon une ou plusieurs des revendications 1 à 12 ou un oligomère, un polymère ou un dendrimère selon la revendication 14.
